# EUROPÄISCHE PATENTSCHRIFT

(11) **EP 3 496 698 B1**
(45) Veröffentlichungstag und Bekanntmachung des Hinweises auf die Patenterteilung: **20.11.2024**
(21) Anmeldenummer: 16750164.2
(22) Anmeldetag: 09.08.2016
(51) Int. Cl.: A61K 8/34, A61K 8/37, A61K 8/49, A61K 8/92, A61Q 19/00, A23G 1/32, A23G 4/06, A61Q 5/06, A61Q 5/02, A61Q 5/12, A61Q 11/00, A61Q 13/00, A61Q 15/00, A61Q 17/04, A61Q 19/10

(54) **MISCHUNGEN ENTHALTEND (E)-3-BENZO[1,3]DIOXOL-5-YL-N,N-DIPHENYL-2-PROPENAMID**
MIXTURES COMPRISING (3)-BENZO(1,3)DIOXOL-5-YL-N,N-DIPHENYLE-2-PROPENAMIDE
COMPOSITIONS COMPRENANT (3)-BENZO(1,3)DIOXOL-5-YL-N,N-DIPHENYLE-2-PROPENAMIDE

(43) Veröffentlichungstag der Anmeldung: 19.06.2019
(73) Patentinhaber: Symrise AG, 37603 Holzminden (DE)
(72) Erfinder: JOIN, Benoit, 37603 Holzminden (DE); BACKES, Michael, 37603 Holzminden (DE); MACHINEK, Arnold, 37603 Holzminden (DE); SIMCHEN, Ulrike, 37603 Holzminden (DE); WEISSBRODT, Jenny, 37603 Holzminden (DE); FAHLE, Jens, 37627 Heinade (DE)
(74) Vertreter: Eisenführ Speiser
(86) Internationale Anmeldenummer: PCT/EP2016/068978
(87) Internationale Veröffentlichungsnummer: WO 2018/028770

(56) Entgegenhaltungen:
- EP-A1- 2 423 290
- EP-A1- 2 824 157
- WO-A2-2011/061330

## Beschreibung

### GEBIET DER ERFINDUNG

Die Erfindung befindet sich auf dem Gebiet von Mischungen, umfassend einen ausgewählten Kühlwirkstoff (E)-3-Benzo[1,3]Dioxol-5-yl-N,N-Diphenyl-2-Propenamid und mindestens eine weitere Substanz, das als Lösungsmittel für(E)-3-Benzo[1,3]Dioxol-5-yl-N,N-Diphenyl-2-Propenamid fungiert, wobei die Substanz ausgewählt wird aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Propylenglykol, weitere bestimmte Kühlwirkstoffe oder bestimmte Mischungen daraus, wobei als Lösungsmittel Benzylalkohol, 2-Phenylethanol oder Benzylbenzoat gewählt wird.

### STAND DER TECHNIK

(E)-3-Benzo[1,3]Dioxol-5-yl-N, N-Diphenyl-2-Propenamid ist unter der CAS Nr. 1309389-73-8 und unter dem Markennamen iCool^{®}MC6 der Firma Symrise AG, bekannt. Die Substanz ist ein Feststoff, das als neuer Kühlwirkstoff entwickelt wurde. In der WO 20011/061330 wird der Einsatz von (E)-3-Benzo[1,3]Dioxol-5-yl-N, N-Diphenyl-2-Propenamid als Modulator des Kälte-Menthol-Rezeptors TRPM8 offenbart. Ebenfalls wird die Anwendung des Kühlwirkstoffes in verschiedenen Formulierungen, wie Mundwasser, Zahncremes, Kaugummis etc. offenbart.

Um die Kühlwirkung von (E)-3-Benzo[1,3]Dioxol-5-yl-N, N-Diphenyl-2-Propenamid jedoch ausschöpfen zu können und zu optimieren, sowie eine einfachere Verarbeitung in Aromen und Halbfertigwaren zu gewährleisten, muss dieser Stoff vor der Verarbeitung in eine Lösung überführt werden. Allerdings ist die Löslichkeit von (E)-3-Benzo[1,3]Dioxol-5-yl-N, N-Diphenyl-2-Propenamid in manchen Fällen nicht ausreichend, so dass dies bei der Lagerung, Weiterverarbeitung und Handhabung Probleme bereitet.

Das Dokument WO 2011/061330 A2 offenbart verschiedene Mischungen von (E)-3-Benzo[1,3]Dioxol-5-yl-N, N-Diphenyl-2-Propenamid mit verschiedenen weiteren Inhaltsstoffen wie Menthol, Menthylmethylether oder Menthone Glycerin Acetal. Das Dokument offenbart nicht Benzylalkohol, 2-Phenylethanol oder Benzylbenzoat als Lösungsmittel.

Die Aufgabe der vorliegenden hat daher darin bestanden, geeignete Lösungsmittel bzw. Lösungsmittelsysteme für (E)-3-Benzo[1,3]Dioxol-5-yl-N, N-Diphenyl-2-Propenamid (hier abgekürzt mit BDDPA) zu finden. Insbesondere war es Aufgabe der Erfindung Lösungsmittel und Lösungsmittelsysteme für (E)-3-Benzo[1,3]Dioxol-5-yl-N, N-Diphenyl-2-Propenamid bereitzustellen, in der (E)-3-Benzo[1,3]Dioxol-5-yl-N, N-Diphenyl-2-Propenamid stabil gelöst gelagert werden kann. Eine Teilaufgabe bestand darin, geeignete Lösungsmittel und Kombinationen daraus bereitzustellen, in der insbesondere mehr als 2 Gew.%, bevorzugt mehr als 5 Gew.% (E)-3-Benzo[1,3]Dioxol-5-Yl-N, N-Diphenyl-2-Propenamid in Lösung gehalten wird, insbesondere bevorzugt bei 23°C. Eine weitere Teilaufgabe bestand darin, insbesondere für die Herstellung von Halbfertigwaren, Lösungsmittel bzw. Lösungsmittelsysteme zu entwickeln, in der (E)-3-Benzo[1,3]Dioxol-5-yl-N, N-Diphenyl-2-Propenamid gelöst vorliegt, die bei höheren Temperaturen, bevorzugt im Bereich von 60°C bis 80°C, bis zur Weiterverarbeitungsprozess stabil bleiben.

### BESCHREIBUNG DER ERFINDUNG

Ein erster Gegenstand der Erfindung betrifft eine Mischung, umfassend oder bestehend aus oder bestehend essentiell aus
(a) (E)-3-Benzo[1,3]Dioxol-5-yl-N,N-Diphenyl-2-Propenamid, und
(b) mindestens eine Substanz ausgewählt aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849) oder Mischungen daraus, wobei das Lösungsmittel b) Benzylalkohol, 2-Phenylethanol oder Benzylbenzoat ist.

Überraschenderweise wurde gefunden, dass die vorliegende Mischung stabil ist und der Kühlwirkstoff (E)-3-Benzo[1,3]Dioxol-5-yl-N,N-Diphenyl-2-Propenamid in einem breiten Bereich variabel, je nach Lösungsmittel bzw. Kombination der genannten Lösungsmitteln, von 2 Gew.% bis 20 Gew.% bzw. von 5 Gew.% bis 15 Gew.% stabil in Lösung gehalten werden kann.

### KÜHLWIRKSTOFFE

Kühlwirkstoffe im Sinne der vorliegenden Erfindung, welche als Lösungmittel für BDDPA fungieren können sind vorzugsweise ausgewählt aus der Gruppe bestehend aus Menthol, Menthyl Methyl Ether, Menthone Glyceryl Acetal (Frescolat^{®} MGA, FEMA GRAS 3807), Menthone Glyceryl Ketal (FEMA GRAS 3808), Menthyl Lactate (Frescolat^{®} ML, FEMA GRAS 3748), Menthol Ethylene Glycol Carbonate (Fresolcat^{®} MGC, FEMA GRAS 3805), Menthol Propylene Glycol Carbonate (Frescolat^{®} MPC, FEMA GRAS 3806), Menthyl-N-ethyloxamat (Frescolat^{®} , Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849) sowie Menthancarbonsäureestern und -amiden wie z.B. WS-3 (FEMA GRAS 3455), WS-5 (FEMA GRAS 4309), WS-12 (Frescolat^{®} SC-1, FEMA GRAS 4681) und WS-23 (FEMA GRAS 3804) sowie deren Gemische.

### OPTAMINT

Optamint ist eine Mischung aus mehr als 50 verschiedenen natürlichen ätherischen Ölen und natürlichen oder natur-identischen Aromastoffen. Optaminte weisen variable Zusammensetzungen von verschiedenen (teilweise fraktionierten) Ölen auf, welche vorzugsweise eine Mischung aus beispielsweise unterschiedlichen Pfefferminzöle und Krauseminzöle, sowie Eucalyptus Globulus Öl, Sternanisöl, Menthol, Menthon, Isomenthon, Menthylacetat, Anethol, Eucalyptol usw. darstellt. Eine exakte Wiedergabe der Zusammensetzung der Optaminte ist daher nicht möglich. Die Produktserie Optamint^{®} ist kommerziell erhältlich bei der Firma Symrise AG.

In einer erfindungsgemäßen Ausführungsform wird Benzylalkohol oder 2-Phenylethanol oder Benzylbenzoat als Lösungsmittel für BDDPA eingesetzt, besonders bevorzugt ist Benzylalkohol.

Die Verwendung von Benzylalkohol oder 2-Phenyethanol oder Benzylbenzoat hat sich als vorteilhaft erwiesen, um (E)-3-Benzo[1,3]Dioxol-5-yl-N, N-Diphenyl-2-Propenamid (BDDPA) in Lösung zu bringen und dabei auch eine stabile Lösung, für eine Lagerung, zu erhalten. Vorzugsweise kann 2 Gew.% bis 20 Gew.%, bevorzugt 5 Gew.% bis 15 Gew.% BDDPA so stabil in Lösung gehalten werden. Zudem zeigte es sich, dass eine solche Mischung bis 100°C, bevorzugt bis 90°C stabil ist. Insbesondere bevorzugt ist Benzylalkohol.

Es hat sich weiterhin als vorteilhaft erwiesen Lösungmittelkombinationen zur Lösung von BDDPA einzusetzen. Vor allem in Hinblick auf den späteren Anwendungsbereich kann durch den Einsatz von Lösungsmitteln, die ebenfalls eine Wirkung zeigen können, ein weiterer Schritt im (End-)Produktionsschritt eingespart werden.

In einer bevorzugten aber nicht durchgängig beanspruchten Ausführungsform ist das Lösungsmittel daher ein binäres System aus zwei Substanzen als Lösungsmittel für BDDPA, die ausgewählt sind aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Optamint, Propylenglykol und weiteren Kühlwirkstoff wie vorstehend beschrieben.

Insbesondere bevorzugt , aber nicht durchgängig beansprucht sind in der vorliegenden Erfindung binäre Lösungsmittelkombinationen von Benzylalkohol und einer weiteren Substanz ausgewählt aus der Gruppe bestehend aus 2-Phenylethanol, Benzylbenzoat, Diethylsuccinat, Triethylcitrat, Triacetin, Ethanol, Pfefferminzöl, Anethol, Optamint, Propylenglykol, oder einem weiteren Kühlwirkstoff wie vorstehend beschrieben.

Besonders bevorzugt sind binäre Lösungsmittelkombinationen bzw.- mischungen, die essentiell Benzylalkohol mit einem weiteren Lösungsmittel enthalten oder daraus bestehen. Besonders bevorzugt , aber nicht durchgängig beansprucht sind die binären Lösungsmittelkombinationen bzw.- -mischungen ausgewählt aus:
- Benzylalkohol und 2-Phenylethanol,
- Benzylalkohol und Benzylbenzoat,
- Benzylalkohol und Diethylsuccinat
- Benzylalkohol und Triethylcitrat,
- Benzylalkohol und Triacetin,
- Benzylalkohol und Ethanol,
- Benzylalkohol und Pfefferminzöl,
- Benzylalkohol und Anethol,
- Benzylalkohol und Optamint,
- Benzylalkohol und Propylenglykol,
- Benzylalkohol und Menthol,
- Benzylalkohol und Menthyl Lactate (Frescolat^{®} ML),
- Benzylalkohol und Menthol Propylene Glycol Carbonate (Frescolat^{®} MPC),
- Benzylalkohol und Menthol Ethylene Glycol Carbonate (Frescolat^{®} MGC),
- Benzylalkohol und Menthone Glyceryl Acetal (Frescolat^{®} MGA),
- Benzylalkohol und Menthancarbonsäureestern und -amiden.

Weiterhin sind die folgenden binären Lösungsmittelkombinationen bzw. Lösungsmittelmischungen besonders bevorzugt, aber nicht durchgangig beansprucht:
- 2-Phenylethanol und Menthol Propylene Glycol Carbonate (Frescolat^{®} MPC),
- Diethylsuccinat und 2-Phenylethanol,
- Triacetin und Benzylbenzoat,
- Triethylcitrat und Triacetin,
- 2-Phenylethanol und Pfefferminzöl,
- 2-Phenylethanol und Optamint,
- Anethol und Triacetin,
- Pfefferminzöl und Menthyl Lactate (Frescolat^{®} ML),
- Triacetin und Menthone Glyceryl Acetal (Frescolat^{®} MGA
- Optamint und Menthyl Lactate (Frescolat^{®} ML),
- Triethylcitrat und Menthol Ethylene Glycol Carbonate (Frescolat^{®} MGC).

Bevorzugte Mischungen im Sinne der vorliegenden Erfindung, enthalten als Lösungsmittel b) daher essentiell eine binäre Lösungsmittelkombination bzw. Lösungsmittelmischungen, wie oben beschrieben.

Binäre Lösungsmittelmischungen im Sinne der vorliegenden Erfindung weisen beispielsweise bevorzugt die folgenden Verhältnisse auf:
i) 2-Phenylethanol und Benzylalkohol, bevorzugt in einem Verhältnis von 10.25: 1 bis 5:1,
ii) Diethylsuccinat und 2-Phenylethanol, bevorzugt in einem Verhältnis von 1:8 bis 8:1,
iii) Triacetin und Benzylbenzoat, bevorzugt in einem Verhältnis von 4:5 bis 5:4,
iv) Benzylbenzoat und Benzylalkohol, bevorzugt in einem Verhältnis von 1:8 bis 8:1,
v) Phenylethanol und Benzylbenzoat von 1:8 bis 8:1.

Die vorgenannten bevorzugten binären Lösungsmittelmischungen zeigten sich insbesondere gut in der Eigenschaft BDDPA zu lösen und in einem breiten Bereich variabel, je nach Lösungsmittel bzw. Kombination der genannten Lösungsmittel, BDDPA in einer Menge von 2 Gew.% bis 20 Gew.%, bevorzugt 5 Gew.% bis 10 Gew.% stabil in Lösung zu halten. Dies hat den Vorteil, dass dadurch BDDPA in einer variablen, für die Endformulierung geeigneten Menge dargestellt werden kann, so dass das Angebot von Mischungen, in denen BDDPA gelöst vorliegt, breit aufgestellt ist. Zudem sind solche Mischungen bis 100°C, bevorzugt bis 90°C stabil.

Insbesondere hat sich die Lösungsmittelkombination von Benzylalkohol und einer weiteren Substanz aus der Gruppe b) als besonders vorteilhaft erwiesen, um die vorgenannten Effekte zu erhalten.

In einer weiteren bevorzugten , aber nicht durchgängig beanspruchten Ausführungsform ist das Lösungsmittel bzw. Lösungsmittelsystem für BDDPA ein ternäres System aus drei Lösungsmitteln, die ausgewählt sind aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Optamint, Propylenglykol, weiteren Kühlwirkstoffen.

Insbesondere bevorzugt sind hierbei ternäre Lösungsmittelkombinationen von Benzylalkohol und zwei weiteren Substanzen ausgewählt aus der Gruppe bestehend aus 2-Phenylethanol, Benzylbenzoat, Diethylsuccinat, Triethylcitrat, Triacetin, Ethanol, Pfefferminzöl, Anethol, Optamint, Propylenglykol, weiteren Kühlwirkstoffen wie vorstehend beschrieben.

Besonders bevorzugt , aber nicht durchgängig beansprucht sind ternäre Lösungsmittelkombinationen bzw.- mischungen, die essentiell Benzylalkohol mit zwei weiteren Lösungsmitteln enthalten oder daraus bestehen, wobei die beiden weiteren Lösungsmittel ausgewählt sind aus der Gruppe bestehend aus:
- 2-Phenylethanol und Benzylbenzoat,
- 2-Phenylethanol und Diethylsuccinat,
- Triethylcitrat und Triacetin,
- Triacetin und Ethanol,
- Triacetin und Pfefferminzöl,
- Menthol Ethylene Glycol Carbonate (Frescolat^{®} MGC) und Anethol,
- 2-Phenylethanol und Optamint,
- Optamint und Propylenglykol,
- Diethylsuccinat und Menthol,
- Triacetin und Menthyl Lactate (Frescolat^{®} ML),
- Anethol und Menthol Propylene Glycol Carbonate (Frescolat^{®} MPC),
- Triacetin und Menthol Ethylene Glycol Carbonate (Frescolat^{®} MGC),
- 2-Phenylethanol und Menthone Glyceryl Acetal (Frescolat^{®} MGA),
- 2-Phenylethanol und Menthancarbonsäureestern und -amiden,
- 2-Phenylethanol und Menthol Propylene Glycol Carbonate (Frescolat^{®} MPC),
- Triacetin und Benzylbenzoat,
- 2-Phenylethanol und Pfefferminzöl,
- Anethol und Triacetin,
- Pfefferminzöl und Menthyl Lactate (Frescolat^{®} ML),
- Triacetin und Menthone Glyceryl Acetal (Frescolat^{®} MGA),
- Optamint und Menthyl Lactate (Frescolat^{®} ML),
- Triethylcitrat und Menthol Ethylene Glycol Carbonate (Frescolat^{®} MGC).
- Benzylbenzoat und Menthol Ethylene Glycol Carbonate (Frescolat^{®} MGC),
- 2-Phenylethanol und Triethylcitrat,
- Triethylcitrat und Diethylsuccinat,
- Pfefferminzöl und Menthyl Lactate (Frescolat^{®} ML),
- Ethanol und Menthyl Lactate (Frescolat^{®} ML).

Weiterhin bevorzugt, aber nicht durchgängig beansprucht, sind folgenden ternären Lösungsmittelkombinationen bzw. Lösungsmittelmischungen:
- Triethylcitrat und Triacetin Menthyl Lactate (Frescolat^{®} ML),
- Triacetin, 2-Phenylethanol und Pfefferminzöl,
- 2-Phenylethanol, Optamint und Pfefferminzöl,
- 2-Phenylethanol, Triacetin und Optamint,
- Anethol, Benzylalkohol und Triacetin,
- 2-Phenylethanol und Benzylbenzoat,
- 2-Phenylethanol und Diethylsuccinat,
- Triethylcitrat, Triacetin und Pfefferminzöl
- Optamint, Triacetin und Ethanol,
- Triacetin, Menthol Ethylene Glycol Carbonate (Frescolat^{®} MGC) und Anethol
- 2-Phenylethanol, Optamint und Propylenglykol,
- Diethylsuccinat, Triacetin und Menthol,
- Triacetin, Benzylbenzoat und Menthyl Lactate (Frescolat^{®} ML),
- Anethol, Menthol Propylene Glycol Carbonate (Frescolat^{®} MPC) und Menthol Ethylene Glycol Carbonate (Frescolat^{®} MGC),
- Triacetin, 2-Phenylethanol und Menthone Glyceryl Acetal (Frescolat^{®} MGA),
- Pfefferminzöl, 2-Phenylethanol und Menthancarbonsäureestern und -amiden,
- Triacetin, 2-Phenylethanol und Menthol Propylene Glycol Carbonate (Frescolat^{®} MPC),
- Menthyl Lactate (Frescolat^{®} ML), 2-Phenylethanol und Pfefferminzöl,
- Anethol, Triacetin und Menthone Glyceryl Acetal (Frescolat^{®} MGA),
- Optamint, Benzylbenzoat und Menthyl Lactate (Frescolat^{®} ML),
- Benzylbenzoat, Triethylcitrat und Menthol Ethylene Glycol Carbonate (Frescolat^{®} MGC).

Ternäre Lösungsmittelmischungen im Sinne der vorliegenden Erfindung weisen beispielsweise bevorzugt die folgenden Verhältnisse auf:
i) 2-Phenylethanol, Benzylalkohol und Triethylcitrat, bevorzugt in einem Verhältnis von 10:1:15 bis 5:1:3, oder
ii) Triethylcitrat, Benzylalkohol und Diethylsuccinat, bevorzugt in einem Verhältnis von 4:1:7 bis 7: 1:4, oder
iii) Triacetin, 2-Phenylethanol und Pfefferminzöl, bevorzugt in einem Verhältnis von 2:2:4 bis 4:4:2.

Die vorgenannten ternären Lösungsmittelkombinationen und -mischungen zeigten sich insbesondere gut in der Eigenschaft BDDPA zu lösen und in einem breiten Bereich variabel, je nach Lösungsmittel bzw. Kombination der genannten Lösungsmittel, BDDPA in einer Menge von 2 Gew.% bis 20 Gew.%, bevorzugt 5 Gew.% bis 10 Gew.% stabil in Lösung zu halten. Dies hat den Vorteil, dass dadurch BDDPA in einer variablen, für die Endformulierung geeigneten Menge dargestellt werden kann, so dass das Angebot von Mischungen, in denen BDDPA gelöst vorliegt, breit aufgestellt ist. Zudem sind solche Mischungen bis 100°C, bevorzugt bis 90°C stabil.

In einer weiteren bevorzugten, aber nicht durchgängig beanspruchten Ausführungsform ist das Lösungsmittel bzw. Lösungsmittelsystem für BDDPA ein quartäres System aus vier Lösungsmitteln, die ausgewählt sind aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Optamint, weiteren Kühlwirkstoffen.

Insbesondere bevorzugt, aber nicht durchgängig beansprucht sind hierbei quartäre Lösungsmittelkombinationen von Benzylalkohol und drei weiteren Substanzen ausgewählt aus der Gruppe bestehend aus 2-Phenylethanol, Benzylbenzoat, Diethylsuccinat, Triethylcitrat, Triacetin, Ethanol, Pfefferminzöl, Anethol, Optamint, Propylenglykol, weiteren Kühlwirkstoffen wie vorstehend beschrieben.

Besonders bevorzugt, aber nicht durchgängig beansprucht sind quartäre Lösungsmittelkombinationen bzw.- mischungen, die essentiell Benzylalkohol mit drei weiteren Lösungsmitteln enthalten oder daraus bestehen, wobei die beiden weiteren Lösungsmittel ausgewählt sind aus der Gruppe bestehend aus:
- 2-Phenylethanol,Triethylcitrat und Triacetin,
- Pfefferminzöl, 2-Phenylethanol und Triethylcitrat,
- Triethylcitrat, Menthyl Lactate (Frescolat^{®} ML) und Diethylsuccinat
- Triethylcitrat, Triacetin und Anethol,
- 2-Phenylethanol, Triacetin, und Optamint,
- Pfefferminzöl, Benzylalkohol und Menthyl Lactate (Frescolat^{®} ML),
- Optamint, Ethanol und Menthyl Lactate (Frescolat^{®} ML),
- 2-Phenylethanol, Benzylbenzoat und Diethylsuccinat,
- Triethylcitrat, Triacetin und Ethanol,
- Pfefferminzöl, Anethol und Optamint,
- 2-Phenylethanol, Benzylbenzoat und Propylenglykol,
- 2-Phenylethanol, Benzylbenzoat und Menthol Propylene Glycol Carbonate (Frescolat^{®} MPC),
- Triethylcitrat, Optamint und Ethanol,
- Triacetin, Benzylbenzoat und Menthoxy-2-methyl-1,2-propandiol,
- Menthone Glyceryl Acetal (Frescolat^{®} MGA), Triacetin und Anethol.

Weiterhin bevorzugt, aber nicht durchgängig beansprucht sind die folgenden quartären Lösungsmittelkombinationen und Lösungsmittel mischungen:
- Anethol, Triacetin, Pfefferminzöl und Menthol Ethylene Glycol Carbonate (Frescolat^{®} MGC)
- Triacetin, Ethanol, 2-Phenylethanol und Pfefferminzöl,
- 2-Phenylethanol, Optamint, Diethylsuccinat und Pfefferminzöl,
- Anethol, 2-Phenylethanol, Benzylalkohol und Triacetin,

Die vorgenannten quartären Lösungsmittelmischungen zeigten sich insbesondere gut in der Eigenschaft BDDPA zu lösen und in einem breiten Bereich variabel, je nach Lösungsmittel bzw. Kombination der genannten Lösungsmittel, BDDPA in einer Menge von 2 Gew.% bis 20 Gew.%, bevorzugt 5 Gew.% bis 10 Gew.% stabil in Lösung zu halten. Dies hat den Vorteil, dass dadurch BDDPA in einer variablen, für die Endformulierung geeigneten Menge dargestellt werden kann, so dass das Angebot von Mischungen, in denen BDDPA gelöst vorliegt, breit aufgestellt ist. Zudem sind solche Mischungen bis 100°C, bevorzugt bis 90°C stabil.

Insbesondere enthalten oder bestehen die vorliegenden erfindungsgemäßen Mischungen vorzugsweise aus Komponente a) in einer Menge von 2 Gew.% bis 20 Gew.%, bevorzugt von 2 Gew.% bis 10 Gew.%, ganz besonders bevorzugt von 5 Gew.% bis 10 Gew.%, insbesondere bevorzugt von 5 Gew% bis 8 Gew.%, und Komponente b) in einer Menge von 98 Gew.% bis 80 Gew,%, bezogen auf die Gesamtmischung, mit der Maßgabe, dass beide Komponenten a) und b) zusammen 100 Gew.% ergeben.

Diese Zusammensetzung der erfindungsgemäßen Mischung ist besonders vorteilhaft, da dadurch die Menge an BDDPA in der Endformulierung gesteuert werden kann, so dass das Endprodukt BDDPA in einer Menge von in etwa 5 ppm bis 50 ppm, vorzugsweise 10 ppm bis 30 ppm, besonders bevorzugt 10 bis 20 ppm, enthält.

Bevorzugte, aber nicht durchgängig beanspruchte Mischungen weisen bevorzugt die folgende Zusammensetzung auf oder bestehen aus:
- 5-10 Gew.% BDDPA in 95-90 Gew.% Benzylalkohol, besonders bevorzugt 8-10 Gew.% BDDPA in 92-90 Gew.% Benzylalkohol, oder
- 1-4 Gew.% BDDPA in 99-96 Gew.% Triethylcitrat, oder
- 1-3 Gew.% BDDPA in 99-97 Gew.% Triacetin, oder
- 3-6 Gew.% BDDPA in 97-94 Gew.% Diethylsuccinat, oder
- 5-15 Gew.% BDDPA in 95-85 Gew.% 2-Phenylethanol, oder
- 5-10 Gew.% BDDPA in 95-90 Gew.% Benzylbenzoat, oder
- 1-3 Gew.% BDDPA in 99-97 Gew.% Optamint, oder
- 1-4 Gew.% BDDPA in 99-96 Gew.% weitere Kühlwirkstoffe wie vorstehend beschrieben, oder
- 2-4 Gew.% BDDPA in 98-96 Gew.% Propylenglykol, oder
- 0.5-2 Gew.% BDDPA in 95.5-98 Gew.% Ethanol, oder
- 0.5-2 Gew.% BDDPA in 95.5-98 Gew.% Menthylacetat, oder
- 1-4 Gew.% BDDPA in 99-96 Gew.% Pfefferminzöl, oder
- 2-5 Gew.% BDDPA in 98-95 Gew.% Anethol,
wobei sich jeweils beide Komponenten (BDDPA und Lösungsmittel) in der Mischung immer zu 100 Gew.% addieren. Besonders bevorzugt besteht eine erfindungsgemäße Mischung aus 5-10 Gew.% BDDPA in 95-90 Gew.% Benzylalkohol, besonders bevorzugt 8-10 Gew.% BDDPA in 92-90 Gew.% Benzylalkohol.

Bevorzugte, aber nicht durchgängig beanspruchte erfindungsgemäße Mischungen, in denen das Lösungsmittel b) ein binäres System ist, weisen bevorzugt die folgende Zusammensetzung auf oder bestehen aus:
- 5-15 Gew.% BDDPA in 5-10 Gew.% Benzylalkohol und 80-90 Gew.% 2-Phenylethanol, oder
- 5-14 Gew.% BDDPA in 8-12 Gew.% Diethylsuccinat und 78-87 Gew.% 2-Phenylethanol, oder
- 3-14 Gew.% BDDPA in 38-42 Gew.% Triacetin und 48-59 Gew.% Benzylbenzoat, oder
- 3-5 Gew.% BDDPA in 47-55 Gew.% Triethylcitrat und 50-60 Gew.% Triacetin, oder
- 5-14 Gew.% BDDPA in 80-85 Gew.% 2-Phenylethanol und 15-20 Gew.% Pfefferminzöl, oder
- 5-14 Gew.% BDDPA in 80-85 Gew.% 2-Phenylethanol und 15-20 Gew.% Optamint, oder
- 5-7 Gew.% BDDPA in 55-65 Gew.% Anethol und 40-45 Gew.% Triacetin, oder
- 5-8 Gew.% BDDPA in 85-95 Gew.% Pfefferminzöl und 10 -12 Gew.% weitere Kühlwirkstoffe wie vorstehend beschrieben, oder
- 5-14 Gew.% BDDPA in 90-95 Gew.% Benzylalkohol und 5-7 Gew.% einem Kühlwirkstoff wie vorstehend beschrieben.
wobei sich jeweils beide Komponenten (BDDPA und Lösungsmitteln) in der Mischung immer zu 100 Gew.% addieren.

In einer weiteren bevorzugten, aber nicht durchgängig beanspruchten Ausführungsform besteht das Lösungsmittel aus einer Kombination aus mehr als 4 Lösungsmitteln, die ausgewählt sind aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Optamint, Propylenglykol, weiteren Kühlwirkstoff wie vorstehend beschrieben. Vorzugsweise enthält oder besteht ein solches Lösungsmittelsystem aus fünf, sechs, sieben, acht, neun, zehn oder elf der vorgenannten Substanzen als Lösungsmittel bzw. Lösungsmittelsystem für BDDPA.

Die Mischungen, umfassend oder bestehend aus a) (E)-3-Benzo[1,3]Dioxol-5-yl-N,N-Diphenyl-2-Propenamid und b) mindestens ein Lösungsmittel ausgewählt aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Optamint, Propylenglykol, weiteren Kühlwirkstoff wie vorstehend beschrieben, sind vorzugsweise bis 100°C stabil, wobei BDDPA in der Mischung gelöst vorliegt.

Es hat sich als vorteilhaft erwiesen Lösungsmittel bzw. Lösungsmittelsysteme bereitzustellen, in denen BDDPA gelöst vorliegt und die bis zu 100°C, bevorzugt bis 90°C stabil sind, da die erfindungsgemäßen Mischungen vorzugsweise als Halbfertigwaren hergestellt werden und noch weiterverarbeitet werden müssen. Da im späteren Verarbeitungsprozess zum Endprodukt, die Mischungen (prozesstechnisch bedingt) teilweise bis zu 100°C, bevorzugt bis 90°C erhitzt werden müssen, ist es von Vorteil, erfindungsgemäße Mischungen bereitzustellen, in denen BDDPA bis zu einer Temperatur von bis etwa 100°C, bevorzugt bis etwa 90°C stabil gelöst vorliegt.

Insbesondere liegen in den erfindungsgemäßen Mischungen (E)-3-Benzo[1,3]Dioxol-5-Yl-N,N-Diphenyl-2-Propenamid vorzugsweise schon bei Raumtemperatur von 23°C im Lösungsmittel bzw. in den vorgenannten Lösungsmittelsysteme gelöst vor. BDDPA ist ein Feststoff. Für eine Weiterverarbeitung wird BDDPA jedoch in gelöster Form gebraucht. Daher ist es von (wirtschaftlichen) Vorteil, dass zum Lösen bzw. in Lösung bringen von BDDPA möglichst wenig Energie verbraucht wird. Ferner erleichtert es die Lagerung von flüssigen BDDPA, wenn die Mischung bei Raumtemperatur von 23°C, flüssig bleibt und nicht re-kristallisiert oder dergleichen instabil wird, so dass zum Lösen von BDDPA die Mischung erst einmal wieder erwärmt werden müsste.

Ein weiterer wichtiger Aspekt der vorliegenden Erfindungen sind Partikel, erhältlich dadurch, dass die erfindungsgemäße Mischung einem Sprühtrocknungsverfahren oder Sprühgranulationsverfahren unterzogen wird.

Da die vorliegende erfindungsgemäße Mischung eine Halbfertigware ist, die noch weiterverarbeitet wird, in dem sie in Endprodukten einformuliert wird, hat es sich als vorteilhaft erwiesen, die erfindungsgemäße Mischung weiterzuverarbeiten, in dem diese sprühgetrocknet oder sprühgranuliert wird, insbesondere im Hinblick auf die leichtere Handhabung, Lagerung und Kompaktheit der Mischung.

Ein weiterer Aspekt der vorliegenden Erfindung sind daher ebenfalls Halbfertigprodukte, umfassend oder bestehend im Wesentlichen aus erfindungsgemäßen Mischungen oder (sprühgetrocknete bzw. sprühgranulierten) Partikeln daraus.

Ein weiterer Aspekt der vorliegenden Offenbarung betrifft kosmetische und/oder dermatologische Zubereitungen, die die oben genannte Mischung enthalten oder und zwar vorzugsweise in Mengen von etwa 0,1 bis etwa 10 Gew.-%, insbesondere etwa 0,5 bis etwa 8 Gew.-% und insbesondere etwa 1 bis etwa 5 Gew.-%. Bei den Mitteln kann es sich sowohl um Haut-, Körperpflege- oder Haarbehandlungsmittel, einschließlich Sonnenschutzmitteln und Mund- und Zahnpflegemitteln sowie (medizinischen) Kaugummis handeln. Besonders bevorzugte Anwendungen im Bereich der kosmetischen und dermatologischen Zubereitungen sind Duschbäder, Shampoos, Seifen, Lufterfrischer und dergleichen.

Zu den besonders bevorzugten pharmazeutischen Zubereitungen zählen Stoffe zur Linderung von Schmerzen der Schleimhäute, speziell Erkältungssäfte, -sprays, -dragees und - bonbons.

Bevorzugt umfasst oder besteht die vorliegende Beschreibung Produkte, enthaltend die erfindungsgemäßen Mischungen oder Partikeln, wobei die Produkte ausgewählt sind aus der Gruppe bestehend aus Nahrungsmitteln, Mundpflegemitteln, Körperpflegemitteln oder pharmazeutischen Mitteln.

Die kosmetischen, dermatologischen und/oder pharmazeutischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Silikonverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### GEWERBLICHE ANWENDBARKEIT

Ein weiterer Gegenstand der Erfindung betrifft die Verwendung von mindestens einem erfindungsgemäßen Lösungsmittel ausgewählt gemäß Anspruch 14 zum Lösen von festen (E)-3-Benzo[1,3]Dioxol-5-yl-N,N-Diphenyl-2-Propenamid, wobei bevorzugt (E)-3-Benzo[1,3]Dioxol-5-yl-N,N-Diphenyl-2-Propenamid bei Raumtemperatur (23°C) gelöst im genannten Lösungsmittel bzw. Mischungen daraus vorliegt.

In der genannten erfindungsgemäßen Verwendung finden die vorstehenden beschriebenen bevorzugten Lösungsmitteln, insbesondere Benzylalkohol, aber auch die bevorzugten binären, ternären und quartären Lösungsmittelsysteme ihre bevorzugte Anwendung.

Ein weiterer Aspekt der vorliegenden Offenbarung betrifft zudem ein Lösungsmittelsystem zum Lösen von (E)-3-Benzo[1,3]Dioxol-5-yl-N,N-Diphenyl-2-Propenamid, ausgewählt aus oder bestehend aus im Wesentlichen mindestens einer Verbindung oder mindestens zwei, drei, vier, fünf, sechs, sieben, acht, neun, zehn oder alle Verbindungen aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Optamint, Propylenglykol, weiteren Kühlwirkstoff wie vorstehend beschrieben, wobei bevorzugt (E)-3-Benzo[1,3]Dioxol-5-yl-N,N-Diphenyl-2-Propenamid bei Raumtemperatur (23°C) gelöst im genannten Lösungsmittelsystem vorliegt.

Insbesondere bevorzugt umfassen oder bestehen die Lösungsmittelsysteme zum Lösen von (E)-3-Benzo[1,3]Dioxol-5-yl-N,N-Diphenyl-2-Propenamid aus den vorstehenden beschriebenen bevorzugten Ausführungsformen an Lösungsmitteln, insbesondere Benzylakohol, aber auch die bevorzugten binären, ternären und quartären Lösungsmittelsysteme.

Insbesondere bevorzugt, aber nicht durchgängig beansprucht ist eine Mischung, umfassend oder die im Wesentlichen besteht aus a) (E)-3-Benzo[1,3]Dioxol-5-yl-N,N-Diphenyl-2-Propenamid, und b) mindestens eine Substanz ausgewählt aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Optamint, Propylenglykol, weitere Kühlwirkstoffe oder Mischungen daraus. Wobei für die Kombination der Substanz a) mit Substanzen b) die vorstehenden beschriebenen bevorzugten Kombinationen, insbesondere mit Benzylalkohol, aber auch insbesondere die bevorzugten binären, ternären und quartären Lösungsmittelsysteme, auch hierauf Anwendung finden.

Besonders bevorzugt ist eine erfindungsgemäße Mischung, welche 8-10 Gew.% BDDPA in 92-90 Gew.% Benzylalkohol umfasst oder im Wesentlichen daraus besteht.

### BEISPIELE

### BEISPIEL 1

### Löslichkeit von (E)-3-Benzo[1,3]Dioxol-5-yl-N,N-Diphenyl-2-Propenamid

Die Löslichkeit von (E)-3-Benzo[1,3]Dioxol-5-yl-N,N-Diphenyl-2-Propenamid (BDDPA) in verschiedenen Lösungsmitteln wurde getestet. Es zeigte sich das die in **Tabelle** 1 gelisteten Formulierungen bei Raumtemperatur (23°C) stabil waren.

**Tabelle 1**

| Bei Raumtemperatur (23°C) stabil, gelöste Formulierungen von BDDPA (Mengenangaben als Gew.-%) | | |
|---|---|---|
| **Mischung** | **Lösungsmittel** | **BDDPA** |
| 1 | Ethanol, | 1 |
| 2 | Menthylacetat, | 1 |
| 3 | Benzylalkohol | 10 |
| 4 | Triethylcitrat, | 3 |
| 5 | Triethylcitrat | 2 |
| 6 | Triethylcitrat | 1 |
| 7 | Triacetin | 3 |
| 8 | Triacetin | 2 |
| 9 | Pfefferminzöl | 3 |
| 10 | Pfefferminzöl | 2 |
| 11 | Optamint | 3 |
| 12 | Optamint | 2 |
| 13 | Pfefferminzöl | 3 |
| 14 | Pfefferminzöl | 2 |
| 15 | Frescolat ^{®}MPC | 2 |
| 16 | Frescolat ^{®}MPC | 1 |
| 17 | Anethol | 5 |
| 18 | Anethol | 3 |

Die Menge des Lösungsmittels addiert mit der Menge von (E)-3-Benzo[1,3]Dioxol-5-yl-N,N-Diphenyl-2-Propenamid addiert sich in jeder Mischung (1 bis 18) zu 100 Gew.%. So lautet die Zusammensetzung von **Mischung 1:** 99 Gew.-% Ethanol, 1 Gew.-% BDDPA.

Die Tabelle zeigt, dass BDDPA sich in den vorliegenden Lösungsmitteln bereits bei Temperaturen ≤ 65°, ggf. unter Zuhilfenahme von Ultraschallbedingungen, löst und bei Raumtemperatur (23°C) BDDPA in den jeweiligen Lösungsmitteln komplett gelöst vorliegt.

### BEISPIEL 2

### Stabilität bei 5°C

Einige der Mischungen aus Beispiel 1 wurden bei Nacht gelagert bei 5°C, um die Stabilität zu beobachten. Es zeigte sich das die in **Tabelle 2** gelisteten Formulierungen stabil vorliegen.

**Tabelle 2**

| Stabilität der Formulierung nach Lagerung von 12 h bei 5 °C (Mengenangaben als Gew.-%) | | |
|---|---|---|
| **Mischung** | **Lösungsmittel** | **BDDPA** |
| 1 | Ethanol | 1 |
| 2 | Menthylacetat | 1 |
| 3 | Benzylalkohol | 10 |
| 5 | Triethylcitrat | 2 |
| 6 | Triethylcitrat | 1 |
| 8 | Triacetin | 2 |
| 10 | Pfefferminzöl | 2 |
| 12 | Optamint | 2 |
| 16 | Frescolat^{®} MPC | 1 |
| 18 | Anethol, | 3 |

Die Menge des Lösungsmittels addiert mit der Menge von (E)-3-Benzo[1,3]Dioxol-5-yl-N,N-Diphenyl-2-Propenamid addiert sich in jeder Mischung (1 bis 18) zu 100 Gew.%. So lautet die Zusammensetzung von **Mischung 1:** 99 Gew.% Ethanol, 1 Gew.% BDDPA

Die Tabelle zeigt, dass BDDPA über Nacht im Lösungsmittel stabil gelöst bleibt, auch wenn die Temperatur unterhalb der Raumtemperatur ist, erfolgt keine Rekristallisation.

### BEISPIEL 3

### Löslichkeit von BDDPA in binären und ternären Lösungsmittelsystemen

Die Löslichkeit von (E)-3-Benzo[1,3]Dioxol-5-yl-N,N-Diphenyl-2-Propenamid (BDDPA) in verschiedenen Lösungsmittelkombinationen wurde getestet. **Tabelle 3** zeigt zusammengefasst die getesteten Formulierungen.

**Tabelle 3**

| Löslichkeit von BDDPA in Lösungsmittelkombinationen bei 23°C und 90°C (Mengenangaben als Gew.-%) | | | | |
|---|---|---|---|---|
| **Mischung** | **BDDPA** | **Lösungsmittel 1** | **Lösungsmittel 2** | **Lösungsmittel 3** |
| 19 | 5 | Triacetin 47,5 | Pfefferminzöl 47,5 | - |
| 20 | 5 | Triethylcitrat 47,5 | Pfefferminzöl 47,5 | - |
| 21 | 5 | Triethylcitrat 95,0 | - | - |
| 22 | 5,2 | Triethylcitrat 31,6 | Triacetin 31,6 | Pfefferminzöl 31,6 |
| 23 | 5 | Triacetin 45,0 | Triethylcitrat 50,0 | - |
| 24 | 5 | Triacetin 25,0 | Triethylcitrat 50,0 | Pfefferminzöl 20,0 |

Die Menge der Lösungsmittel 1 bis 3 addieren sich mit der Menge von (E)-3-Benzo[1,3]Dioxol-5-yl-N,N-Diphenyl-2-Propenamid zu 100 Gew.%: So lautet die Zusammensetzung von **Mischung 19:** 5 Gew.% BDDPA, 47,5 Gew.% Triacetin und 47,5 Gew.% Pfefferminzöl

Es zeigte sich, dass BDDPA sich in binären und ternären Lösungsmittelkombinationen bei Raumtemperatur (23°C) BDDPA komplett gelöst vorliegt und bei hohen Temperaturen von 90°C ebenfalls stabil gelöst bleibt.

### BEISPIEL 4

### Löslichkeitsverhalten von BDDPA bei hohen Temperaturen

Das Löslichkeitsverhalten von (E)-3-Benzo[1,3]Dioxol-5-yl-N,N-Diphenyl-2-Propenamid (BDDPA) in verschiedenen Lösungsmittelkombinationen bei hohen Temperaturen wurde getestet. In **Tabelle 4** ist die Zusammensetzung der getesteten Mischungen. Die Ergebnisse sind in **Tabelle** 5 zusammengefasst.

**Tabelle 4**

| Zusammensetzung der getesteten Mischungen 25-31 | | | | | | | |
|---|---|---|---|---|---|---|---|
| **Mischung** | **25** | **26** | **27** | **28** | **29** | **30** | **31** |
| **Lösungmsmittel** | Gew.-% | | | | | | |
| BDDPA | 10 | 10 | 10 | 15 | 10 | 10 | 10 |
| Triacetin | | | | | | | 40 |
| Triethylcitrat | - | 32 | | | | | |
| Benzylalkohol | - | 8 | 8 | | | | |
| Diethylsuccinat | - | 50 | - | - | 10 | | |
| 2-Phenylethanol | 90 | - | 82 | 85 | 80 | | |
| Benzylbenzoat | - | | | | | 90 | 50 |

**Tabelle 5**

| Ergebnisse Löslichkeitsverhalten von BDDPA bei hohen Temperaturen | | | | |
|---|---|---|---|---|
| **Mischung** | **Löslichkeitsverhalten** | | | **Rekristallisation** |
| | **60°C** | **70 °C** | **80 °C** | |
| **25** | teilweise | teilweise | komplett | nein |
| **26** | teilweise | teilweise | komplett | < 40 °C |
| **27** | teilweise | teilweise | komplett | nein |
| **28** | teilweise | teilweise | komplett | nein |
| **29** | teilweise | teilweise | komplett | nein |
| **30** | komplett | komplett | komplett | nein (nach 2 Tagen ja) |
| **31** | komplett | komplett | komplett | nein (nach 2 Tagen ja) |

Die Tabellen 3, 4 und 5 stellen exemplarische Mischungen dar, die für eine weitere Verarbeitung (z.B. Sprühtrocknung) bevorzugt verwendet werden können, da in diesem Fall die Mischung zum Lösen für maximal 30 Minuten auf 90 °C erwärmt wird und dann zur weiteren Verarbeitung auf 50 - 60°C abgekühlt und bei dieser Temperatur stabil ohne Ausfällungen (bis zu 6 Stunden) gelagert werden kann.

### FORMULIERUNGSBEISPIELE

### a) Mundpflege

### Formulierungsbeispiel FM-1: Mundwasser

Geeignete Mundwässer können nach folgender Basisrezeptur hergestellt werden:

| **Anteil (Gew.-%)** | **Inhaltsstoff-Typ** | **Inhaltsstoff-Beispiele** |
|---|---|---|
| 0,01-0,1 | antibakterielle Mittel | beta-Naphthol, Thymol, Chlorthymol und Hexylresorcin |
| 5-25 | Feuchthaltemittel | Glycerin, Sorbit, Propylenglykol und Polyalkylenglykol |
| 0,01-0,2 | etherische Öle | Nelkenöl, Pfefferminzöl und Krauseminzöl |
| 0-30 | Ethanol | |
| 0-5 | Polymer | Polyoxyalkylenblockcopolymere Mw 5000-30000 |
| 40-80% | Wasser | |
| 0,0125-55 | erfindungsgemäße Mischung | Siehe Tabelle1 und Tabelle 3 |
| 0-10 | Weitere Zusätze | |

Ein Mundwasser folgender Zusammensetzung wird hergestellt:

| **Anteil** | **Inhaltsstoff** |
|---|---|
| 220,75 mL | Ethanol 95% |
| 250 g | Sorbit 70% |
| 6,25 mL | 8 Gew.% BDDPA in 92 Gew.% Benzylakohol |
| 0.30 g | Pfefferminzöl, |
| 0.64 g | Methylsalicylat |
| 0.922 g | Eucalyptol |
| 0.639 g | Thymol |
| 1.50 g | Benzoesäure |
| 5.00 g | Pluronic ^{®} F127 |
| | nichtionisches Tensid |
| 0.60 g | Natrium-Saccharin |
| 0.30 g | Natriumcitrat |
| 0.10 g | Zitronensäure |
| q.s. 1 Liter | Wasser |

Zur Herstellung eines Mundwassers werden die oben beschriebenen Komponenten in den angegebenen Mengen miteinander vermischt.

### Formulierungsbeispiel FM-2: Zahnpasta

Geeignete Zahnpasten können nach folgender Basisrezeptur hergestellt werden:

| **Anteil (Gew.-%)** | **Inhaltsstoff-Typ** | **Inhaltsstoff-Beispiele** |
|---|---|---|
| 0,05-0,2 | Fluoride | Natriumfluorid, Zinn(II)-fluorid, Natriummonofluorophosphat; |
| 10-55 | Feuchthaltemittel | Glycerin, Sorbit, Propylenglykol, Polyalkylenglykol |
| 0-50 | Polymer | Polyoxyalkylenblockcopoymere Mw 5000-30000 |
| 10-50 | Wasser | |
| 10-55 | Abrasiva | Calciumpyrophosphat, Dicalciumphosphat, Siliziumoxidhydrat; |
| 2-10 | Bindemittel | Karayagummi, Tragant USP, Natriumalginat, irländisches Moos, Methylcellulose |
| 2-8 | Tensid | Natriumlaurylsulphat, Natrium-N-laurylsarcosinat, Dioctlnatriumsulphosuccinat, Natriumlaurylsulphoacetat |
| 0-10 | Persauerstoffverbindung | Wasserstoffperoxid, anorganischen Peroxiden |
| 0,0125-55% | erfindungsgemäße Mischung | |
| 0-10 s.o. | Weitere Zusätze | |

### Formulierungsbeispiel FM-3: Kaugummi

Geeignete Kaugummis können nach folgender Basisrezeptur hergestellt werden:

| **Anteil (Gew.-%)** | **Inhaltsstoff** |
|---|---|
| 15 - 25 | Kaugummi-Grundmasse ("gum-base") |
| 20 - 30 | Glucosesirup |
| 50 - 60 | Puderzucker |
| 0,1 - 10 | erfindungsgemäße Mischung |
| 1 - 2 | Weichmacher (z.B. Glycerin) |
| 3 - 6 | Wasser |

Anstelle des Glucosesirups und des Puderzuckers können für "zuckerfreie" Rezepturen auch die Zuckeralkohole Mannit, Xylit und Sorbit, "Palatinit" und andere sowie künstliche Süßstoffe, wie Saccharin, Cyclamat, Acesulfam-K und Aspartame, eingesetzt werden.

### b) Körperpflege

### Formulierungsbeispiel FM-4: Haarwasser

| **Phase** | **Anteil (Gew.-%)** | **Inhaltsstoff (INCI)** |
|---|---|---|
| A | q.s. | Parfümöl |
| | 1,00 | PEG-40 Hydrogenated Castor Oil |
| B | 59,0-65,0 | Alkohol |
| | 1,0 | Panthenol |
| | 0,5 | Polyquarternium-16 |
| | 0,1 | Menthol |
| | 32 | Aqua dem. |
| | 0,0625-6,25 | 8 Gew.% BDDPA in 92 Gew.% Benzylakohol |

Herstellung: Phase A mischen. Phase B zugeben und rühren bis alles gelöst ist, pH-Wert einstellen auf pH 7,0.

### Formulierungsbeispiel FM-5: Haargel

| **Phase** | **Anteil (Gew.-%)** | **Inhaltsstoff (INCI)** |
|---|---|---|
| A | 45,00 | Carbopol 940 1% in Wasser |
| | 0,70 | Aminomethyl Propanol |
| B | 7,50 | VP/Methacrylamide/Vinyl Imidazole Copolymer |
| | 0,10 | Parfümöl |
| | 0,30 | PEG-40 Hydrogenated Castor Oil |
| | 0,30 | Konservierungsmittel |
| | 0,05 | Disodium EDTA |
| | 0,30 | Panthenol |
| | 6,75-12,94 | Alkohol |
| | 0,0625-6,25 | 8 Gew.% BDDPA in 92 Gew.% Benzylakohol |
| | 32,75 | Aqua dem. |

Herstellung: Die Komponenten der Phase A einwiegen und homogenisieren. Phase B lösen und in Phase A einrühren. pH Wert einstellen auf pH 6,9.

### Formulierungsbeispiel FM-6: Kosmetische Sonnenschutzzubereitung

In den folgenden Rezepturen wird eine kosmetische Sonnenschutzzubereitung, enthaltend eine Kombination aus mindestens anorganischem Pigment und organische m UV-Filter beschrieben.

Die Herstellung der nachfolgend genannten Formulierungen erfolgt auf übliche, dem Fachmann bekannte Art und Weise.

| **Phase** | **Anteil (Gew.-%)** | **Komponente** | **Inhaltsstoff (INCI)** |
|---|---|---|---|
| A | 7,50 | Uvinul MC80 | Ethylhexylcinnamat |
| | 2,00 | Uvinul M 40 | Benzophenon-3 |
| | 0,80 | Rylo PG 11 | Polyglyceryldimersoyat |
| | 1,00 | Span 60 | Sorbitanstearat |
| | 0,50 | Vitamin E-Acetat | Tocopherylacetat |
| | 3,00 | Dracorin 100 SE | Glycerylstearat, PEG-100 Stearat |
| | 1,00 | Cremophor CO 410 | PEG-40-hydriertes Rizinusöl |
| B | 3,00 | T-Lite SF | Titandioxid, Aluminiumoxidhydrat, Dimethicon-/Methicon Copolymer |
| | 1,00 | Cetiol SB 45 | Butyrospermum parkii (Shea Butter) |
| | 6,50 | Finsolv TN | C12-15-Alkylbenzoat |
| C | 5,00 | Butylenglykol | Butylenglycol |
| | 0,30 | Keltrol | Xanthangummi |
| | 0,10 | Edeta BD | Dinatrium-EDTA |
| | 0,10 | Allantoin | Allantoin |
| | Ad 100 | Wasser dem. | Aqua dem. |
| D | 1,00 | Sepigel 305 | Polyacrylamid, C13-14-Isoparaffin, Laureth-7 |
| | 0,0125-12,5 | 0,0125-12,5 % | erfindungsgemäße Mischung |
| | q.s. | | Konservierungsmittel |

### Formulierungsbeispiel FM-7: Feuchtigkeitsspendende Körperpflegecreme

| **Phase** | **Anteil (Gew.-%)** | **Inhaltsstoff (INCI)** |
|---|---|---|
| A | 6,0 | PEG-7-hydriertes Rizinusöl |
| | 10,0 | Cetearylethylhexanoat |
| | 5,0 | Isopropylmyristat |
| | 7,0 | Mineralöl |
| | 0,5 | Shea Butter (Butyrospermum parkii) |
| | 0,5 | Aluminumstearat |
| | 0,5 | Magnesiumstearat |
| | 0,2 | Bisabolol |
| | 0,7 | Quaternium-18-Hectorit |
| B | 5,0 | Dipropylenglykol |
| | 0,7 | Magnesiumsulfat |
| | q.s. | Konservierungsmittel |
| | 50-62,9 | Aqua dem. |
| | q.s. | Parfümöl |
| C | 0,0125-12,5 | 8 Gew.% BDDPA in 92 Gew.% Benzylakohol |

Herstellung: Die Phasen A und B getrennt auf ca. 80 °C erwärmen. Phase B in Phase A einrühren und homogenisieren. Unter Rühren auf ca. 40 °C abkühlen, Phase C zugeben und nochmals homogenisieren. Unter Rühren auf Raumtemperatur abkühlen lassen.

### Formulierungsbeispiel FM-8: Pflegeshampoo

| **Phase** | **Anteil (Gew.-%)** | **Inhaltsstoff (INCI)** |
|---|---|---|
| A | 30,0 | Natriumlaurethsulfat |
| | 6,0 | Natriumocoamphoacetat |
| | 6,0 | Cocamidopropylbetain |
| | 3,0 | Natriumlaurethsulfat, Glykoldistearat, Cocamid-MEA, Laureth-10 |
| | 0,0125-12,5 | 8 Gew.% BDDPA in 92 Gew.% Benzylakohol |
| | 7,7 | Polyquaternium-44 |
| | 2,0 | Amodimethicon |
| | q.s. | Parfümöl |
| | q.s. | Konservierungsmittel |
| | 1,0 | Natriumchlorid |
| | 30-42 | Aqua dem. |
| B | q.s. | Zitronensäure |

Herstellung: Die Komponenten der Phase A mischen und lösen. Den pH-Wert mit Zitronensäure auf 6-7 einstellen.

### Formulierungsbeispiel FM-9: Duschgel

| **Phase** | **Anteil (Gew.-%)** | **Inhaltsstoff (INCI)** |
|---|---|---|
| A | 40,0 | Natriumlaurethsulfat |
| | 5,0 | Decylglukosid |
| | 5,0 | Cocamidopropylbetain |
| | 0,0125-12,5 | 8 Gew.% BDDPA in 92 Gew.% Benzylakohol |
| | 1,0 | Panthenol |
| | q.s. | Parfümöl |
| | q.s. | Konservierungsmittel |
| | 2,0 | Natriumchlorid |
| | 32-45 | Aqua dem. |
| B | q.s. | Zitronensäure |

Herstellung: Die Komponenten der Phase A mischen und lösen. Den pH-Wert mit Zitronensäure auf 6-7 einstellen.

### Formulierungsbeispiel FM-10: Shampoo

| **Phase** | **Anteil (Gew.-%)** | **Inhaltsstoff (INCI)** |
|---|---|---|
| A | 40,0 | Natriumlaurethsulfat |
| | 5,0 | Natrium-C12-15-Pareth-15-sulfonat |
| | 5,0 | Decylglukosid |
| | q.s. | Parfümöl |
| | 0,1 | Phytantriol |
| | 31-43 | Aqua dem. |
| | 0,0125-12,5 | 8 Gew.% BDDPA in 92 Gew.% Benzylakohol |
| | 0,3 | Polyquaternium-10 |
| | 1,0 | Panthenol |
| | q.s. | Konservierungsmittel |
| | 1,0 | Laureth-3 |
| | 2,0 | Natriumchlorid |

Herstellung: Die Komponenten der Phase A mischen und lösen. Den pH-Wert mit Zitronensäure auf 6-7 einstellen.

### Formulierungsbeispiel FM-11: Fußbalsam

| **Phase** | **Anteil (Gew.-%)** | **Inhaltsstoff (INCI)** |
|---|---|---|
| A | 2,0 | Ceteareth-6, Stearylalkohol |
| | 2,0 | Ceteareth-25 |
| | 5,0 | Cetearylethylhexanoat |
| | 4,0 | Cetylalkohol |
| | 4,0 | Glycerylstearat |
| | 5,0 | Mineralöl |
| | 0,2 | Menthol |
| | 0,5 | Kampfer |
| B | 57,8-69 | Aqua dem. |
| | q.s. | Konservierungsmittel |
| C | 1,0 | Bisabolol |
| | 1,0 | Tocopherylacetat |
| D | 0,0125-12,5 | 8 Gew.% BDDPA in 92 Gew.% Benzylakohol |
| | 5,0 | Zaubernussextrakt |

Herstellung: Die Komponenten der Phasen A und B getrennt voneinander auf ca. 80 °C erwärmen. Phase B in Phase A unter Homogenisieren einrühren. Unter Rühren abkühlen auf ca. 40 °C, die Phasen C und D hinzugeben und kurz nachhomogenisieren. Unter Rühren auf Raumtemperatur abkühlen.

### Formulierungsbeispiel FM-12: Gesichtsreinigungslotion - Typ O/W

| **Phase** | **Anteil (Gew.-%)** | **Inhaltsstoff (INCI)** |
|---|---|---|
| A | 10,0 | Cetearylethylhexanoat |
| | 10,0 | Capryl-/Caprintriglycerid |
| | 1,5 | Cyclopentasiloxan, Cyclohexasilosan |
| | 2,0 | PEG-40-hydriertes Rizinusöl |
| B | 3,5 | Capryl-/Caprintriglycerid, Natriumacrylat-Copolymer |
| C | 1,0 | Tocopherylacetat |
| | 0,2 | Bisabolol |
| | q.s. | Konservierungsmittel |
| | q.s. | Parfümöl |
| D | 3,0 | Polyquaternium-44 |
| | 0,5 | Cocotrimoniummethosulfat |
| | 0,5 | Ceteareth-25 |
| | 2,0 | Panthenol, Propylenglykol |
| | 4,0 | Propylenglykol |
| | 0,1 | Dinatrium-EDTA |
| | 0,0125-12,5 | 8 Gew.% BDDPA in 92 Gew.% Benzylakohol |
| | 49-59,7 | Aqua dem. |

Herstellung: Phase A lösen. Phase B in Phase A einrühren, Phase C in die kombinierten Phasen A und B einarbeiten. Phase D lösen, in die kombinierten Phasen A, B und C einrühren und homogenisieren. 15 min nachrühren.

### Formulierungsbeispiel FM-13: Body-Spray

| **Phase** | **Anteil (Gew.-%)** | **Inhaltsstoff (INCI)** |
|---|---|---|
| A | 3,0 | Ethylhexylmethoxycinnamat |
| | 2,0 | Diethylaminohydroxybenzoylhexylbenzoat |
| | 1,0 | Polyquaternium-44 |
| | 3,0 | Propylenglykol |
| | 2,0 | Panthenol, Propylenglykol |
| | 1,0 | Cyclopentasiloxan, Cyclohexasilosan |
| | 10,0 | Octyldodecanol |
| | 0,5 | PVP |
| | 10,0 | Capryl-/Caprintriglycerid |
| | q.s. | Parfümöl |
| | 3,0 | C12-15-Alkylbenzoat |
| | 3,0 | Glycerin |
| | 1,0 | Tocopherylacetat |
| | 0,3 | Bisabolol |
| | 0,0125-12,5 | 8 Gew.% BDDPA in 92 Gew.% Benzylakohol |
| | 46,7-58 | Alkohol |

Herstellung: Die Komponenten der Phase A einwiegen und klar lösen.

### Formulierungsbeispiel FM-14: Hautpflegegel

| **Phase** | **Anteil (Gew.-%)** | **Inhaltsstoff (INCI)** |
|---|---|---|
| A | 3,6 | PEG-40-hydriertes Rizinusöl |
| | 15,0 | Alkohol |
| | 0,1 | Bisabolol |
| | 0,5 | Tocopherylacetat |
| | q.s. | Parfümöl |
| B | 3,0 | Panthenol |
| | 0,6 | Carbomer |
| | 0,0125-12,5 | 8 Gew.% BDDPA in 92 Gew.% Benzylakohol |
| | 62,9-75,4 | Aqua dem. |
| C | 0,8 | Triethanolamin |

### Formulierungsbeispiel FM-15: After-Shave-Lotion

| **Phase** | **Anteil (Gew.-%)** | **Inhaltsstoff (INCI)** |
|---|---|---|
| A | 10,0 | Cetearylethylhexanoat |
| | 5,0 | Tocopherylacetat |
| | 1,0 | Bisabolol |
| | q.s. | Parfümöl |
| | 0,3 | Acrylate/C10-30 Alkylacrylat-Crosspolymer |
| B | 15,0 | Alkohol |
| | 1,0 | Panthenol |
| | 3,0 | Glycerin |
| | 0,125-12,5 | 8 Gew.% BDDPA in 92 Gew.% Benzylakohol |
| | 0,1 | Triethanolamin |
| | 51,1-63,6 | Aqua dem. |

Herstellung: Die Komponenten der Phase A mischen. Phase B lösen, in Phase A einarbeiten und homogenisieren.

### Formulierungsbeispiel FM-16: After-Sun-Lotion

| **Phase** | **Anteil (Gew.-%)** | **Inhaltsstoff (INCI)** |
|---|---|---|
| A | 0,4 | Acrylate/C10-30-Alkylacrylat-Crosspolymer |
| | 15,0 | Cetearylethylhexanoat |
| | 0,2 | Bisabolol |
| | 1,0 | Tocopherylacetat |
| | q.s. | Parfümöl |
| B | 1,0 | Panthenol |
| | 15,0 | Alkohol |
| | 3,0 | Glycerin |
| | 0,0125-12,5 | 8 Gew.% BDDPA in 92 Gew.% Benzylakohol |
| | 50-63,2 | Aqua dem. |
| C | 0,2 | Triethanolamin |

Herstellung: Die Komponenten der Phase A mischen. Phase B unter Homogenisieren in Phase A einrühren. Mit Phase C neutralisieren und erneut homogenisieren.

### Formulierungsbeispiel FM-17: Sonnenschutzlotion

| **Phase** | **Anteil (Gew.-%)** | **Inhaltsstoff (INCI)** |
|---|---|---|
| **A** | 4,5 | Ethylhexylmethoxycinnamat |
| | 2,0 | Diethylaminohydroxybenzoylhexylbenzoat |
| | 3,0 | Octocrylen |
| | 2,5 | Di-C12-13-Alkylmalat |
| | q.s. | Parfümöl |
| | 0,5 | Tocopherylacetat |
| | 4,0 | Polyglyceryl-3-methylglucosedistearat |
| **B** | 3,5 | Cetearylisononanoat |
| | 1,0 | VP-/EicosenCopolymer |
| | 5,0 | Isohexadecan |
| | 2,5 | Di-C12-13-Alkylmalat |
| | 3,0 | Titaniumdioxid, Trimethoxycaprylylsilan |
| C | 5,0 | Glycerin |
| | 1,0 | Natriumcetearylsulfat |
| | 0,5 | Xanthangummi |
| | 47,2-59,7 | Aqua dem. |
| D | 0,0125-12,5 | 8 Gew.% BDDPA in 92 Gew.% Benzylakohol |
| | 1,0 | Phenoxyethanol, Methylparaben, Ethylparaben, Butylparaben, Propyl-paraben, Isobutylparaben |
| | 0,3 | Bisabolol |

Herstellung: Die Komponenten der Phasen A und B getrennt voneinander auf ca. 80 °C erwärmen. Phase B in Phase A einrühren und homogenisieren. Phase C auf ca. 80 °C erwärmen und unter Homogenisieren in die kombinierten Phasen A und B einrühren. Unter Rühren auf ca. 40 °C abkühlen, Phase D zugeben und nochmals homogenisieren.

### Formulierungsbeispiel FM-18: Pflaster

50 Teile Wirkstoff gemäß Herstellungsbeispiel H 3-7 wurden in 100 Teilen einer 10% igen Natriumlaurylsulfatlösung unter starkem Rühren und Erwärmen auf 50oC dispergiert. In die entstandene Emulsion wurden 880 Teile einer 50 %igen Butylacrylatdispersion eingerührt und die erhaltene wirkstoffhaltige Polymerdispersion mittels einer geeigneten Ausstreichrakel auf einer Polyesterfolie mit 15 µm Dicke (Fa. Kalle, D-Wiesbaden) ausgestrichen und bei 35 bis 40 OC unter kontrollierter Luftfeuchtigkeit getrocknet. Je nach Rakeleinstellung resultierten Flächengewichte von 5 mg/cm2, die durch Mehrfachauftrag weiter erhöht werden konnten. Der so hergestellte selbstklebende Film mit einem Wirkstoffgehalt von 5 % wurde mit einer silikonisierten Abziehfolie aus Polyester (Scotch Pak 75 mu m, 3M) versehen und in die gewünschten Abmessungen geschnitten.

Die Mengenangabe betreffen jeweils Gewichtsteile.

### c) Nahrungsmittel

### Formulierungsbeispiel FM-19: Pudding

Rezept (für 100 ml)

| **Inhaltsstoff** | **Anteil** |
|---|---|
| Fettfreie Trockenmilch | 10,715 g |
| Saccharose | 5 g |
| Novelose Stärke, National Starch | 7 g |
| Pflanzenölgemisch | 2,2 g |
| Carrageenan | 0,016 g |
| Vanillearoma | 0,5 g |
| Natriumstearoyl-2-lactylat | 0,095 g |
| Gelber Farbstoff | 0,189 g |
| Magnesiumphosphat | 0,165 g |
| Vitaminvormischung | 1,84 g |
| Spurenelementvormischung | 0,015 g |
| 8 Gew.% BDDPA in 92 Gew.% Benzylakohol | 30 mg |
| Wasser | 76,19 g |

Herstellung: Neun Zehntel des Wassers auf 43,3 °C erhitzen. Magermilchpulver im Wasser auflösen. Öl auf 60 °C erhitzen und Carrageenan und öllösliche Vitamine zum Öl geben. Öl in das Erzeugnis einmischen. Die übrigen Bestandteile außer der modifizierten Stärke, Vanillearoma und Vitaminvormischung hinzufügen. Das Gemisch homogenisieren. Stärke langsam hinzufügen. Wirkstoff, Vitamine und Aroma hinzufügen. Feststoffgehalt standardisieren. In sterilen Einheiten erhitzen und in Dosen verpacken.

### d) Formulierungsbeispiel FM-20: Textilausrüstung mit erfindungemäßer Mischung

Zunächst stellt man eine wässrige Aufschlämmung amylosehaltiger Stärke her, indem man 570 g entionisiertes Wasser wurden mit 10 g eines handelsüblichen Konservierungsmittels versetzt. Hierin löste man 20 g Carboxymethylcellulose, gab anschließend 400 g einer amylosehaltigen Stärke mit einem Amylosegehalt von 50 Gew.-% zu und stellte unter Rühren eine Aufschlämmung her.

Anschließend erfolgt die Herstellung wässriger Flotten mit amylosehaltiger Stärke nach einer der beiden folgenden Methoden:
Methode 1: Die jeweilige Aufschlämmung wird durch Verdünnen mit Wasser auf einen Stärkegehalt von 5 oder 15 Gew.-% eingestellt.
Methode 2: Die jeweilige Aufschlämmung werden zunächst mit Wasser auf einen Stärke-Gehalt von 5 oder 15 Gew.-% verdünnt und anschließend mit 30 g/l einer 30 Gew.-%igen, wässrigen Polyurethandispersion (nicht-ionogen) versetzt.

Anschließend erfolgt die Ausrüstung eines Gewebes mit amylosehaltiger Stärke und erfindungsgemäßer Mischung:
Baumwollgewebemuster mit einem Flächengewicht von 124 g/m² wird mit einer der obe hergestellten Flotten mittels eines Foulards bis zu einer Flottenaufnahme von 80 Gew.-%, bezogen auf das Gewicht des Gewebes behandelt. Anschließend trocknet man 2 min bei 120°C.

Anschließend werden die so ausgerüsteten Gewebemuster mit einer wässrigen erfindungsgemäßen Formulierungsmischung behandelt, indem man eine wässrige Emulsion/Suspension einer erfindungsgemäßen Mischung mit einem Gehalt an BDDPA von 1 bis 7 Gew.-% bis zu einer Flottenaufnahme von 79 - 80 Gew.-% auf das Gewebemuster auffoulardiert. Anschließend werden die so behandelten Gewebemuster in einem Haushaltstrockner bis zu einer Restfeuchte von 15 % getrocknet.

Die so hergestellten wirkstoffbeladenen Gewebe können dann weiter untersucht werden, wie z.B. auf deren Kühlende Wirkung bei Hautkontakt oder deren Repellent-Wirkung auf Insekten.

### e) Aromakompositionen

Alle Angaben, soweit nicht anders vermerkt, in Gew-%.

### Aromakomposition FM-21:

Herstellung von Aromen mit Kühlwirkung vom Eukalyptus-Menthol-Typ unter Verwendung der erfindungsgemäßen Mischung:
Es wurde eine Lösung A mit der nachstehenden Zusammensetzung mit unterschiedlichen Mischungen vermischt so dass Aromakompositionen a-h erhalten wurden. Die Aromakompositionen b-f sind dabei nicht erfindungsgemäß.

### Lösung A:

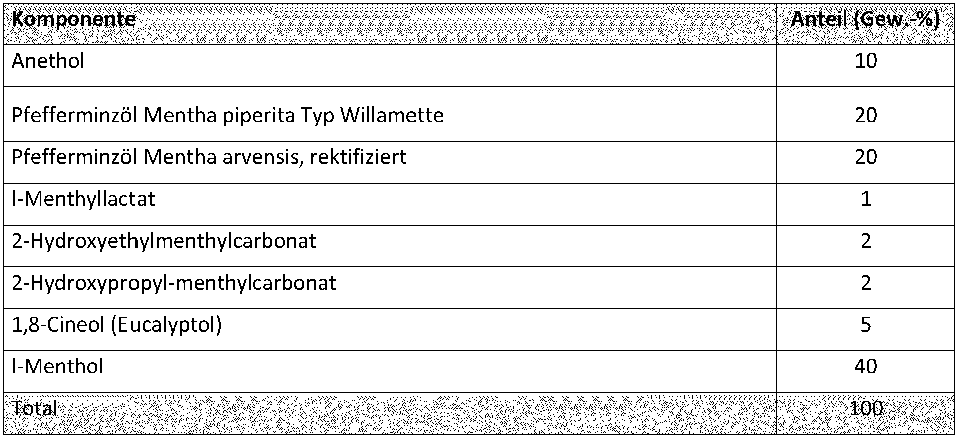

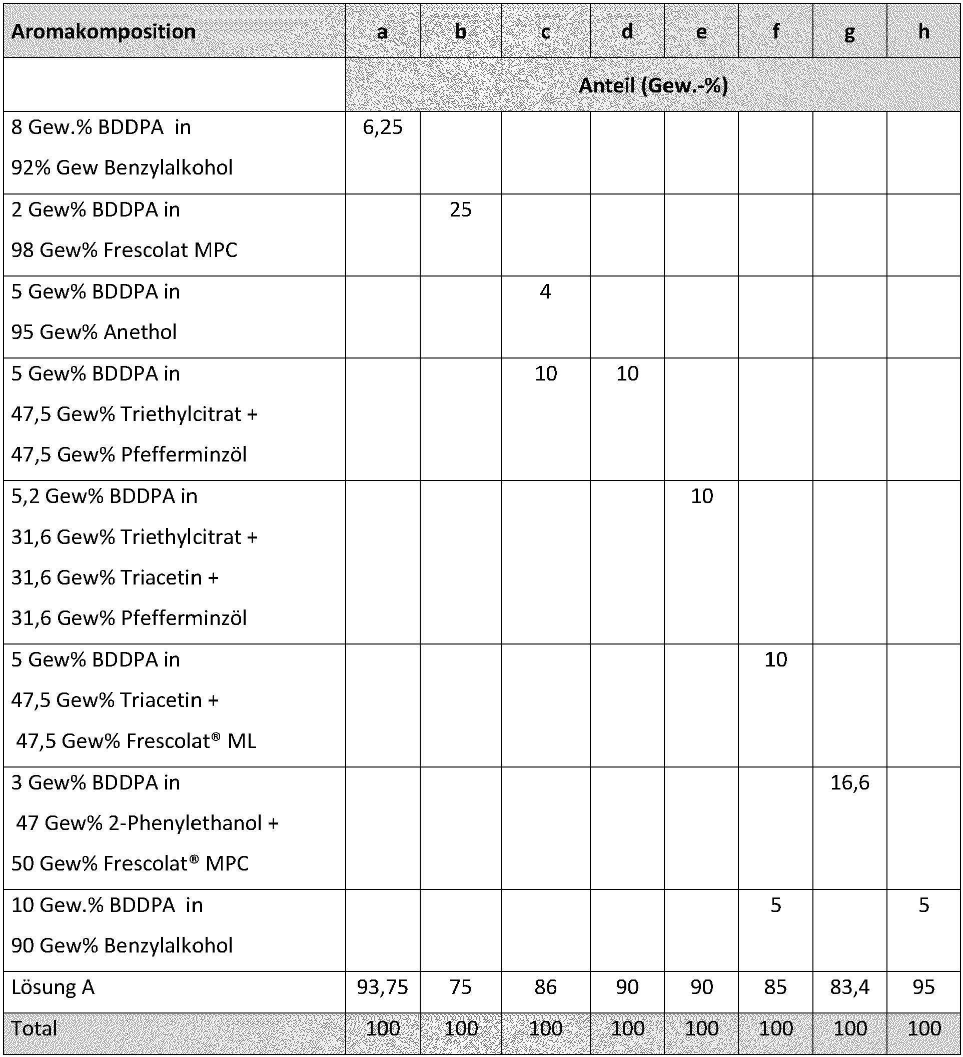

Die so erhaltenen Aromen wurden in eine Standard-Zahnpasta-Masse auf Kieselsäure-Basis in einer Konzentration von 1,2 Gew.-% eingearbeitet.

### Aromakomposition FM-22:

Herstellung von Aromen mit Kühlwirkung vom Krauseminz-Typ unter Verwendung der erfindungsgemäßen Kühlsubstanzen.

Es wurde eine Lösung A mit der nachstehenden Zusammensetzung mit unterschiedlichen Mischungen vermischt, so dass Aromakompositionen a-h erhalten wurden. Die Aromakompositionen b-f sind dabei nicht erfindungsgemäß.

### Lösung A:

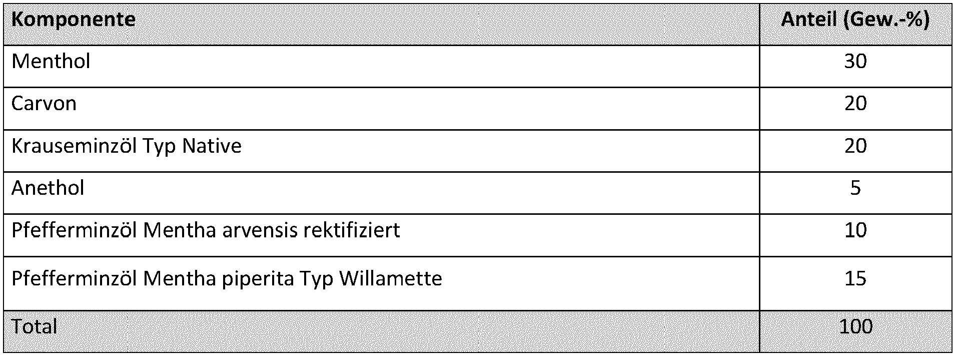

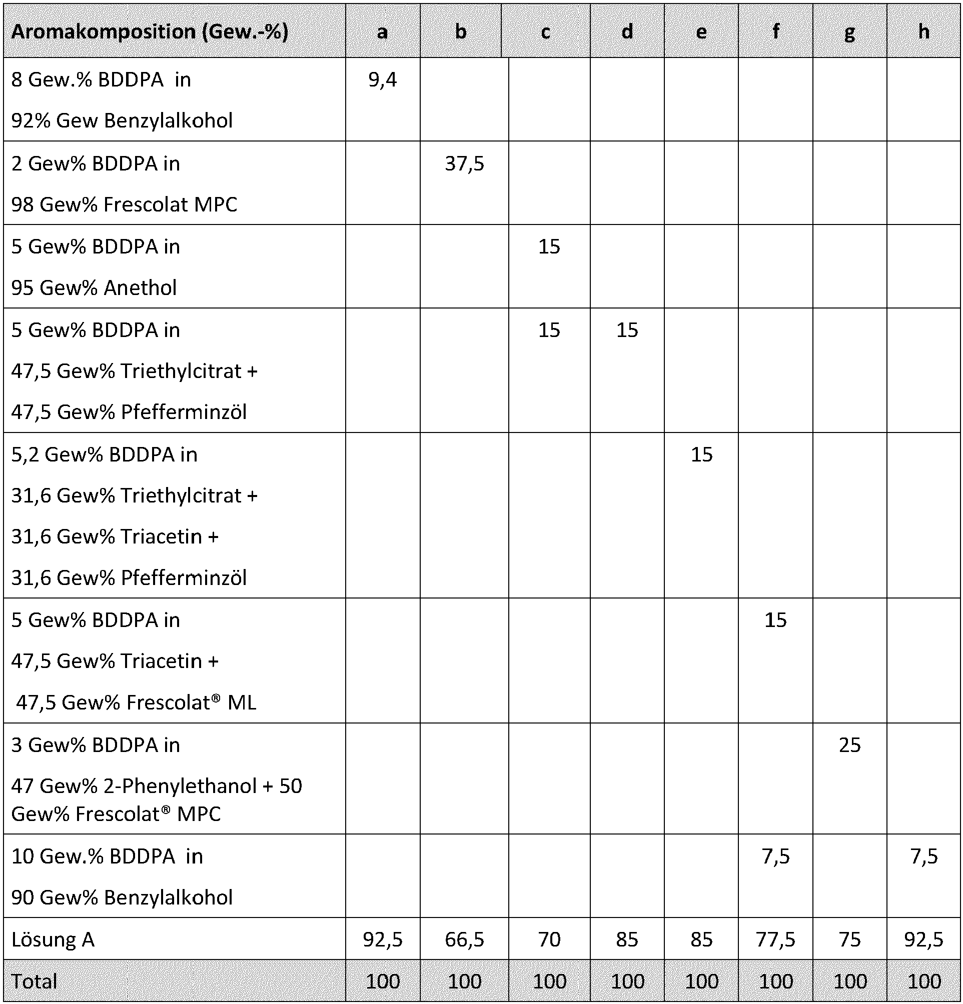

Die erhaltenen Aromen wurden mit einer Konzentration von 1,2 % in eine Zahnpasta-Masse eingearbeitet, die zu einem Anteil von 65% aus Natriumbicarbonat besteht.

**Aromakomposition FM-23:** Herstellung von Aromen mit Kühlwirkung und einem würzig-aromatischen Geschmackseindruck unter Verwendung der erfindungsgemäßen Kühlsubstanzen.

Es wurde eine Lösung A mit der nachstehenden Zusammensetzung mit unterschiedlichen Mischungen vermischt, so dass Aromakompositionen a-h erhalten wurden. Die Aromakompositionen b-f und h sind dabei nicht erfindungsgemäß.

### Lösung A:

| **Komponente** | **Anteil (Gew.-%)** |
|---|---|
| I-Menthol | 30 |
| Pfefferminzöl Mentha arvensis, rektifiziert | 25 |
| Pfefferminzöl Mentha piperita Typ Willamette | 15 |
| Anethol | 10 |
| Krauseminzöl Typ Native | 10 |
| Zimtaldehyd | 5 |
| Eugenol | 5 |
| Total | 100 |

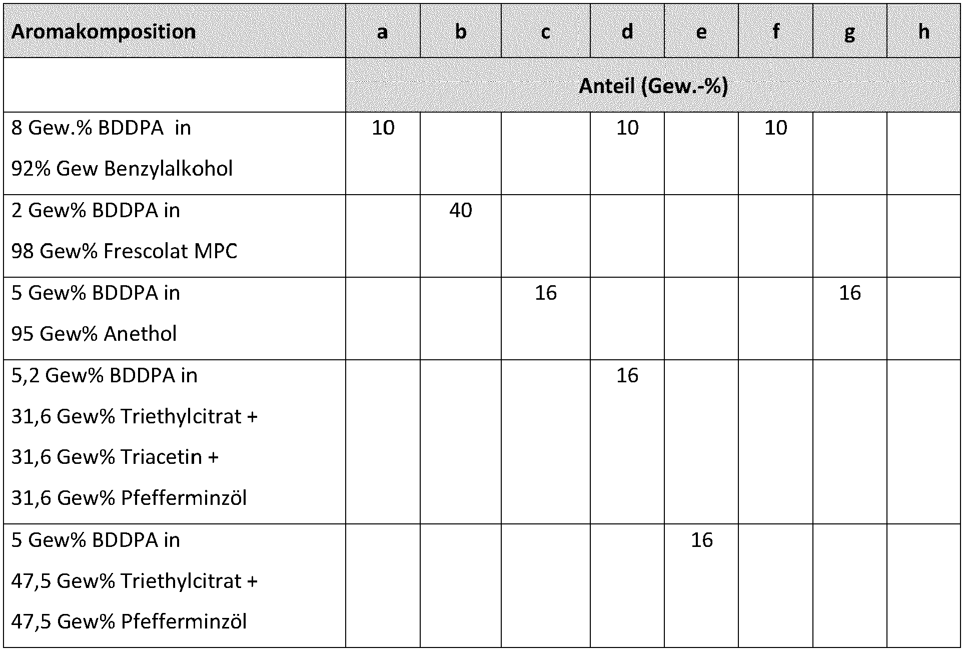

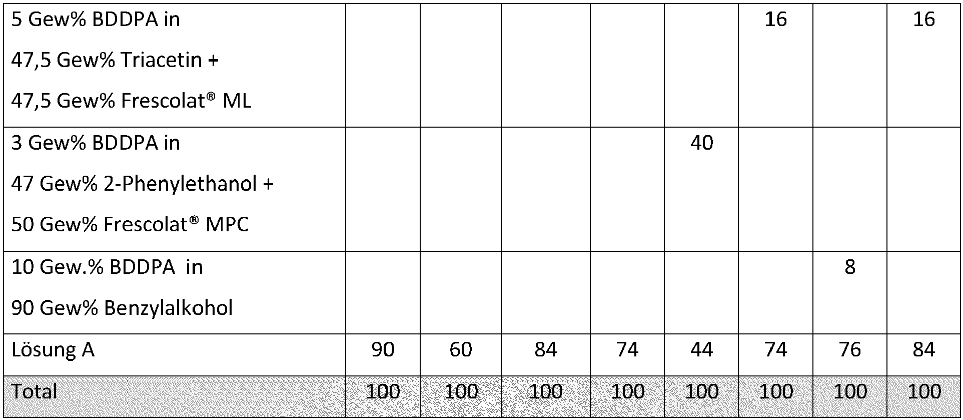

Die so erhaltenen Aromen wurden jeweils in eine Standard-Zahnpasta-Masse auf Kieselsäure-Basis in einer Konzentration von 1,2 Gew.-% eingearbeitet.

### Aromakomposition FM-24:

Herstellung von Aromen mit Kühlwirkung und Wintergrün-Geschmack unter Verwendung der erfindungsgemäßen Kühlsubstanzen:
Es wurde eine Lösung A mit der nachstehenden Zusammensetzung mit unterschiedlichen Mischungen vermischt, so dass Aromakompositionen a-h erhalten wurden. Die Aromakompositionen b-f sind dabei nicht erfindungsgemäß.

### Lösung A:

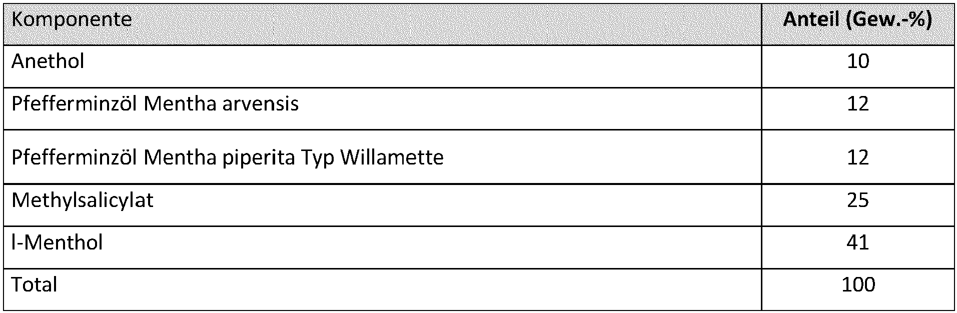

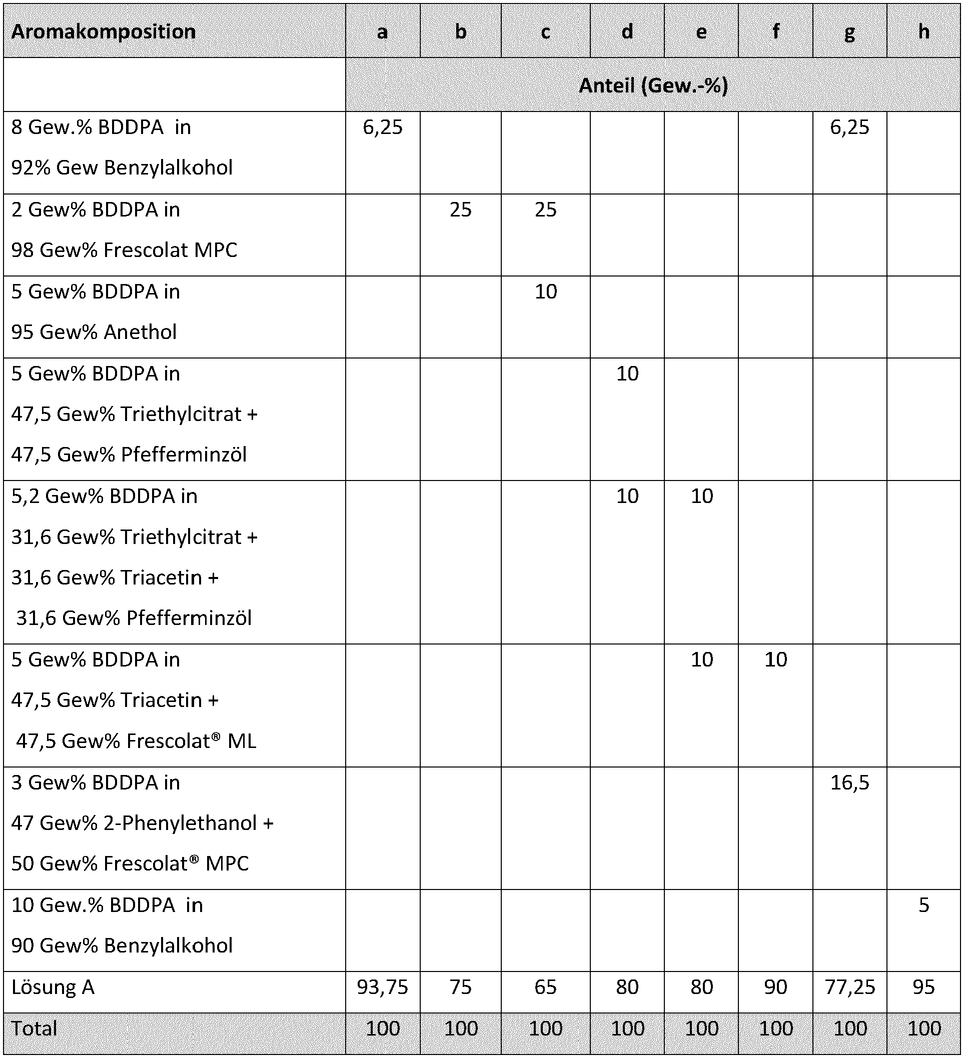

Die so erhaltenen Aromen wurden in eine Standard-Zahnpasta-Masse auf Kieselsäure-Basis in einer Konzentration von 1,2 Gew.-% eingearbeitet.

### Aromakomposition FM-25:

Herstellung von Aromen mit Kühlwirkung und Pfefferminzgeschmack unter Verwendung von den erfindungsgemäßen Kühlsubstanzen:
Es wurde eine Lösung A mit der nachstehenden Zusammensetzung mit unterschiedlichen Mischungen vermischt, so dass Aromakompositionen a-h erhalten wurden. Die Aromakompositionen b-f sind dabei nicht erfindungsgemäß.

### Lösung A:

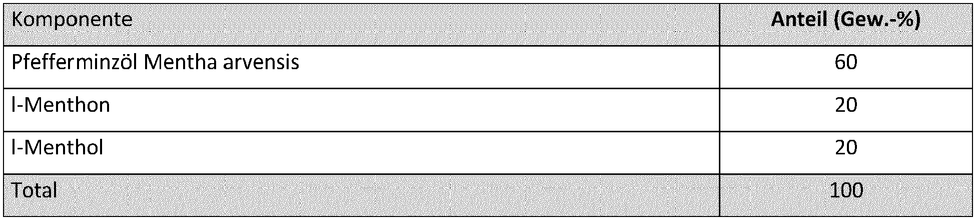

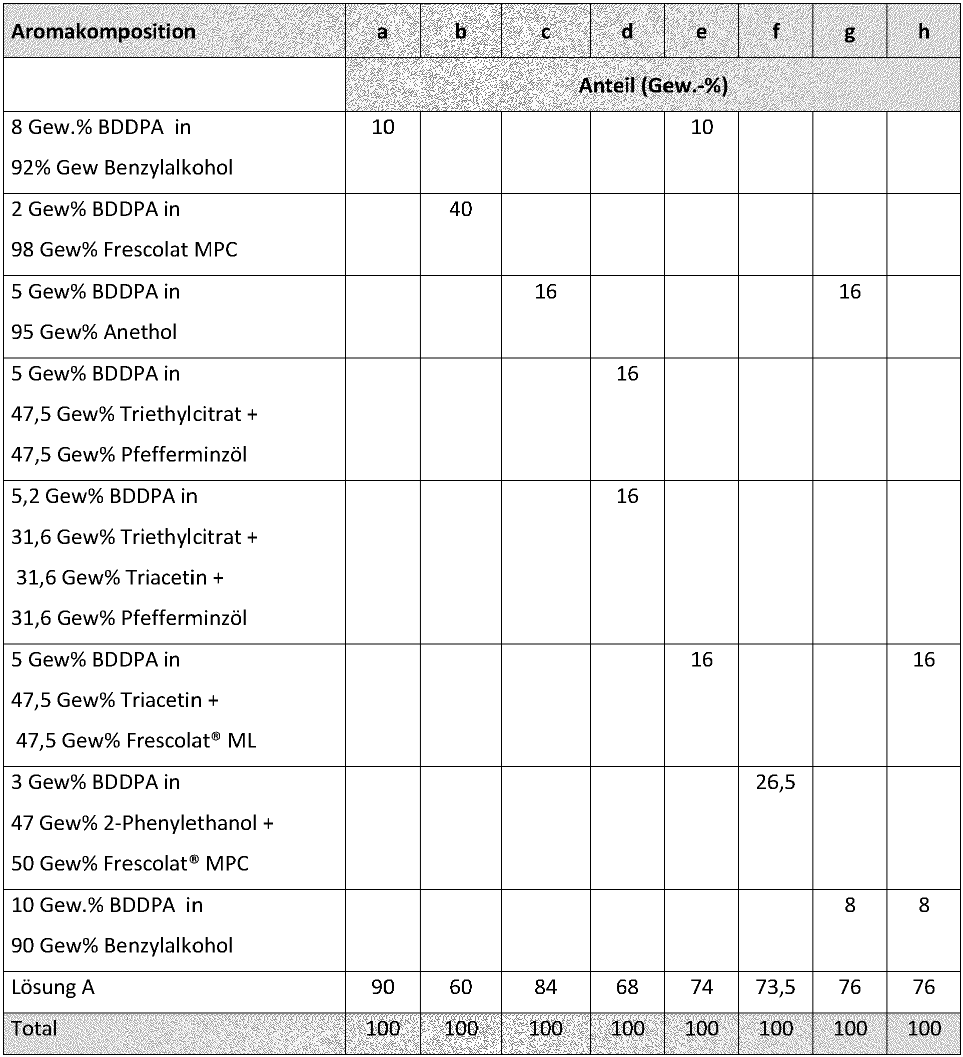

Die so erhaltenen Aromen wurden jeweils in eine zuckerfreie Standard-KaugummiMasse in einer Konzentration von 1,5 Gew.-% eingearbeitet.

### Aromakomposition FM-26:

Herstellung von Aromen mit Kühlwirkung und Krauseminzgeschmack unter Verwendung der erfindungsgemäßen Kühlsubstanzen:
Es wurde eine Lösung A mit der nachstehenden Zusammensetzung mit unterschiedlichen Mischungen vermischt, so dass Aromakompositionen a-h erhalten wurde. Die Aromakompositionen b-f sind dabei nicht erfindungsgemäß.

### Lösung A:

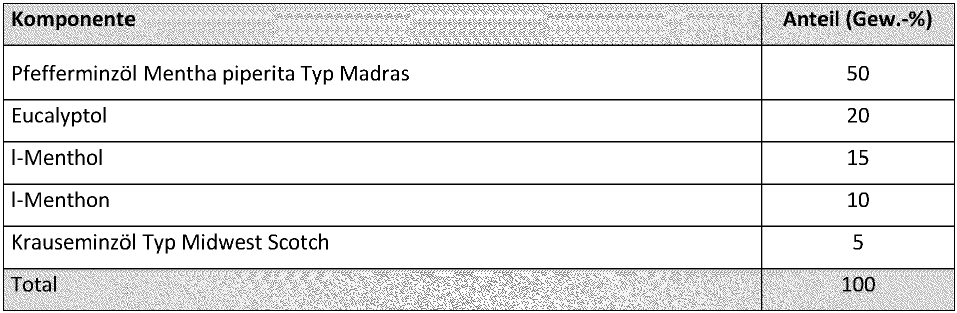

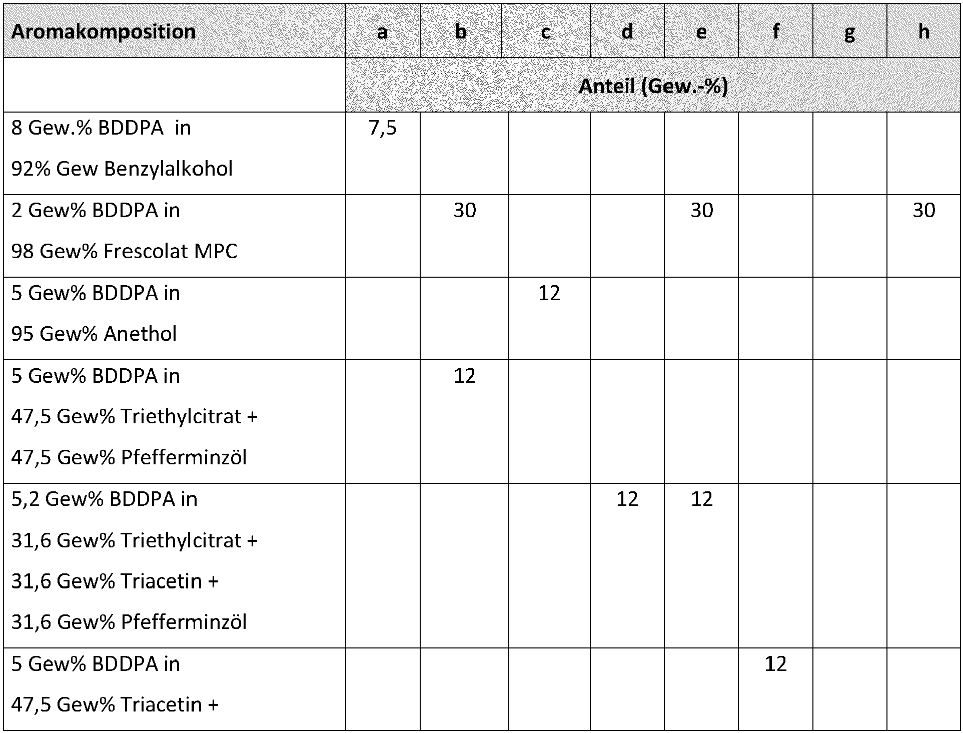

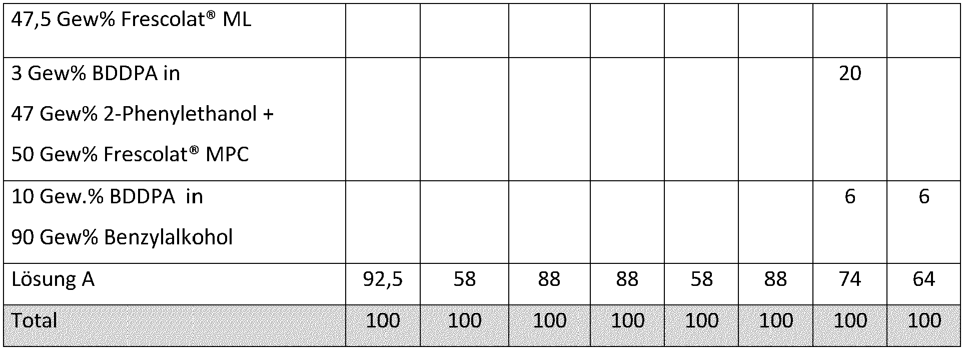

Die so erhaltenen Aromen wurden jeweils in eine zuckerfreie Standard-KaugummiMasse in einer Konzentration von 1,5 Gew.-% eingearbeitet.

### Aromakomposition FM-27:

Herstellung von Aromen mit Kühlwirkung und einem aromatisch-würzigen Zimtgeschmack unter Verwendung der erfindungsgemäßen Kühlsubstanzen:
Es wurde eine Lösung A mit der nachstehenden Zusammensetzung mit unterschiedlichen Mischungen vermischt, so dass Aromakompositionen a-h erhalten wurde. Die Aromakompositionen b und d sind dabei nicht erfindungsgemäß.

### Lösung A:

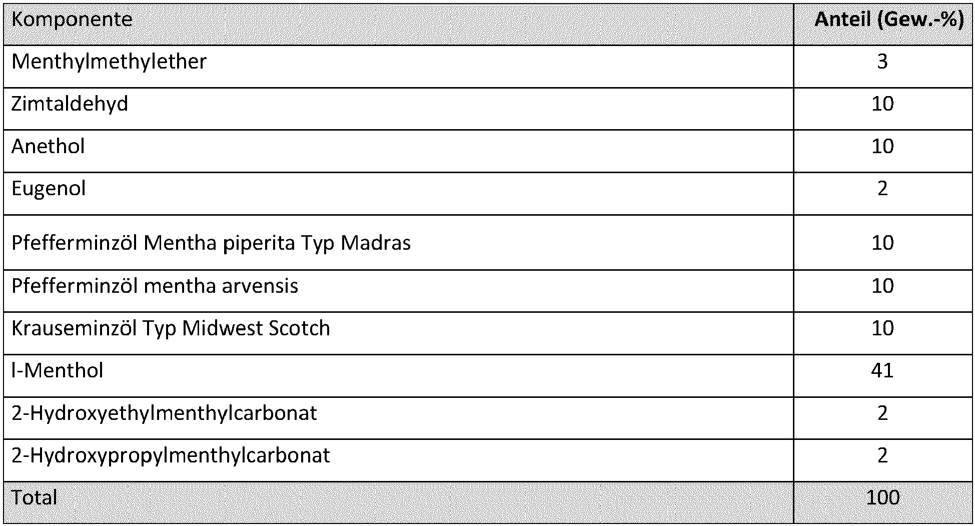

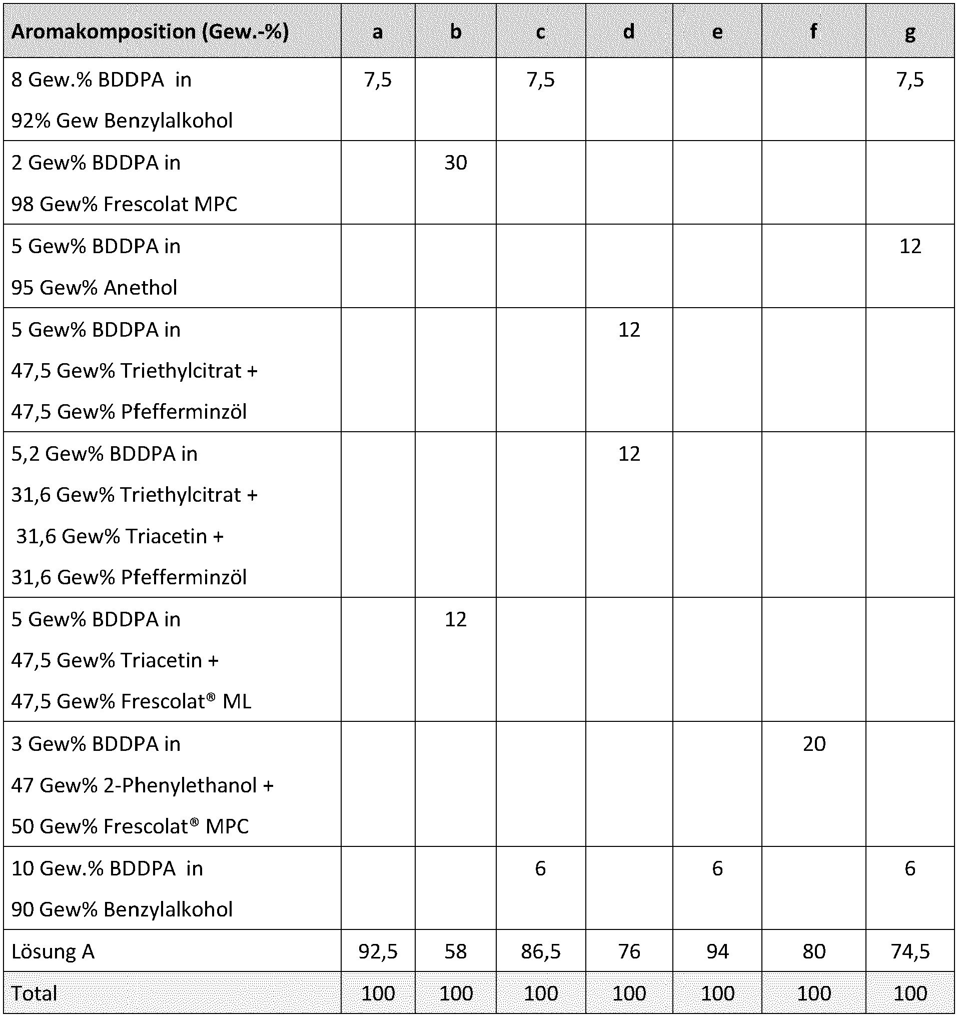

Die so erhaltenen Aromen wurden jeweils in eine zuckerfreie Standard-KaugummiMasse in einer Konzentration von 1,5 Gew.-% eingearbeitet.

### Aromakomposition FM-28:

Herstellung von Mundwasser-Aromen mit Kühlwirkung unter Verwendung der erfindungsgemäßen Kühlsubstanzen.

Es wurde eine Lösung A mit der nachstehenden Zusammensetzung mit unterschiedlichen Mischungen vermischt, so dass Aromakompositionen a-h erhalten wurde. Die Aromakompositionen b-f und h sind dabei nicht erfindungsgemäß.

### Lösung A:

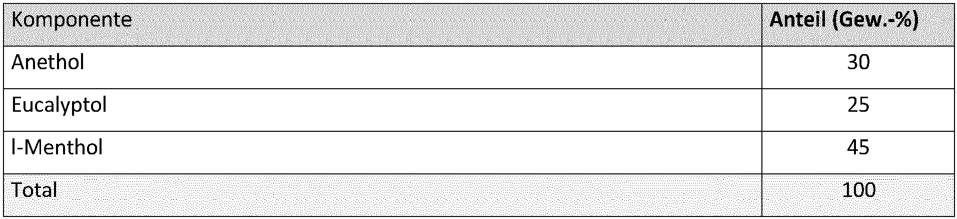

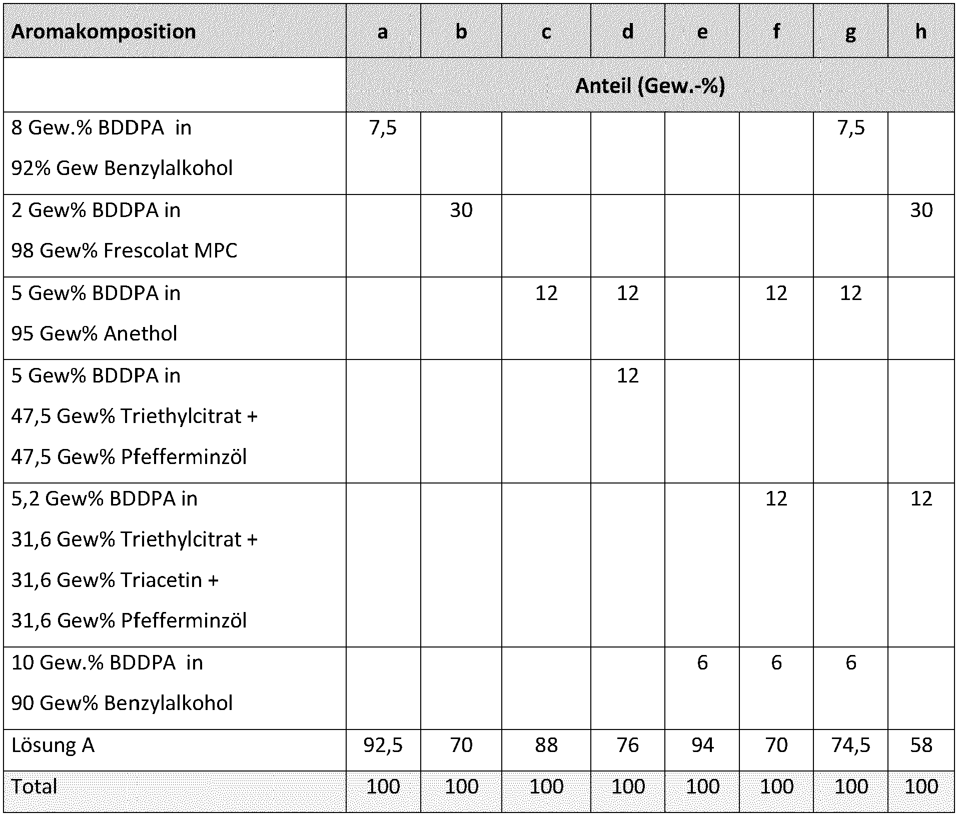

Die Aromen wurden jeweils mit einer Konzentration von 0,15 Gew.% in ein gebrauchsfertiges Mundwasser, bzw. mit einer Konzentration von 3 Gew.% in ein Mundwasserkonzentrat eingearbeitet.

Die unter den Beispielen **FM-21 bis FM-28** genannten Aromakompositionen eignen sich für einen Einsatz in einer ganzen Reihe von unterschiedlichen Fertigprodukten, wobei der Einsatz nicht nur auf Zahnpasten beschränkt ist. Bei allen nachfolgend aufgeführten Beispielen konnte ein vorteilhaftes schnell einsetzendes, aber gleichzeitig sehr lang andauerndes Frischegefühl wahrgenommen werden, ohne dass dieser Frischeeindruck durch scharfe und bittere Noten beeinträchtigt wurde.

Nachfolgend sind weitere Anwendungsbeispiele für oben genannte Aromakompositionen in weiteren Fertigprodukten aufgeführt:

### Formulierungsbeispiel FM-29: Zahnpasta (,Silica opaque')

| **Zubereitung** | **1** | **2** | **3** | **4** | **5** | **6** |
|---|---|---|---|---|---|---|
| Bestandteil | **Anteil (Gew.-%)** | | | | | |
| Entionisiertes Wasser | 26,53 | 26,53 | 26,53 | 26,53 | 26,53 | 26,53 |
| Sorbitol 70% | 45 | Ad 100 | 45 | Ad 100 | 45 | Ad 100 |
| Solbrol M Na-Salz | 0,15 | 0,15 | 0,15 | 0,15 | 0,15 | 0,15 |
| Trinatriumphosphat | 0,1 | 0,1 | 0,1 | 0,1 | 0,1 | 0,1 |
| Saccharin | 0,2 | 0,2 | 0,2 | 0,2 | 0,2 | 0,2 |
| Natriummonofluorphosphat | 1,12 | 1,12 | 1,12 | 1,12 | 1,12 | 1,12 |
| PEG 1500 | 5 | 5 | 5 | 5 | 5 | 5 |
| Sident 9 (Abrasive Silica) | 10 | 10 | 10 | 10 | 10 | 10 |
| Sident 22 S (Dickende Silica) | 8 | 8 | 8 | 8 | 8 | 8 |
| Natriumcarboxymethylcellulose | 0,9 | 0,9 | 0,9 | 0,9 | 0,9 | 0,9 |
| Titan (IV) oxid | 0,5 | 0,5 | 0,5 | 0,5 | 0,5 | 0,5 |
| Natriumlaurylsulfat (SLS) | 1,5 | 1,5 | 1,5 | 1,5 | 1,5 | 1,5 |
| Pellitorin-Lösung PLM (enthaltend 10% Pellitorin) | - | 0,025 | - | 0,025 | - | 0,025 |
| Aromakomposition FM-21 (b) | 1 | 1 | | | | |
| Aromakomposition FM-21 (c) | | | 1 | 1 | | |
| Aromakomposition FM-21 (f) | | | | | 1 | 1 |

### Formulierungsbeispiel FM-30: Zahnpasta (Calciumcarbonat-Base)

| **Zubereitung** | **1** | **2** | **3** | **4** | **5** | **6** |
|---|---|---|---|---|---|---|
| **Bestandteil** | **Anteil (Gew.-%)** | | | | | |
| Saccharin | 0,2 | 0,2 | 0,2 | 0,2 | 0,2 | 0,2 |
| Solbrol M Natriumsalz | 0,2 | 0,2 | 0,2 | 0,2 | 0,2 | 0,2 |
| Natriummonofluorphosphat | 0,8 | 0,8 | 0,8 | 0,8 | 0,8 | 0,8 |
| Sorbitol 70% | 29 | 29 | 29 | 29 | 29 | 29 |
| Calciumcarbonat | 35 | 35 | 35 | 35 | 35 | 35 |
| Sident 22 S (Verdickende Silica) | 2,5 | 2,5 | 2,5 | 2,5 | 2,5 | 2,5 |
| Natriumcarboxymethylcellulose | 1,3 | 1,3 | 1,3 | 1,3 | 1,3 | 1,3 |
| Titandioxid | 0,5 | 0,5 | 0,5 | 0,5 | 0,5 | 0,5 |
| Natriumlaurylsulfat | 2 | 2 | 2 | 2 | 2 | 2 |
| Pellitorin-Lösung PLM (enthaltend 10% Pellitorin) | - | 0,02 | - | 0,02 | - | 0,02 |
| Aromakomposition FM-21 (a) | 1 | 1 | | | | |
| Aromakomposition FM-21 (d) | | | 1 | 1 | | |
| Aromakomposition FM-21 (h) | | | | | 1 | 1 |
| Entionisiertes Wasser | 27,5 | Ad 100 | 27,5 | Ad 100 | 27,5 | Ad 100 |

### Formulierungsbeispiel FM-31: Zahnpasta mit Bleichwirkung

| **Zubereitung** | **1** | **2** | **3** | **4** | **5** | **6** |
|---|---|---|---|---|---|---|
| **Bestandteil** | **Anteil (Gew.-%)** | | | | | |
| Polyphosphat (Glass H, (n ≈ 21), Astaris) | 7 | 7 | 7 | 7 | 7 | 7 |
| Calciumperoxid | 1 | - | 2,5 | 1 | - | 2,5 |
| Na-percarbonat | - | 11 | - | - | 11 | - |
| Poloxamer 407 | 5 | 2 | 5 | 5 | 2 | 5 |
| Polyethylenglycol | 3 | - | 3 | 3 | - | 3 |
| Sorbitol, 70 %ig in Wasser | - | 22 | - | - | 22 | - |
| Glycerin | 43,8 | 12,5 | 28,6 | 43,8 | 12,5 | 28,6 |
| 1,2-Propylenglycol | 4 | - | 2,5 | 4 | - | 2,5 |
| Na-Saccharin | 0,4 | 0,2 | 0,5 | 0,4 | 0,2 | 0,5 |
| Natriumbicarbonat | - | 5 | 15 | - | 5 | 15 |
| Natriumcarbonat | 2 | 2 | 2 | 2 | 2 | 2 |
| Silica | 20 | 22 | 20 | 20 | 22 | 20 |
| Na-Carboxymethylcellulose | 0,6 | 0,55 | 0,3 | 0,6 | 0,55 | 0,3 |
| Natriumlaurylsulfat | 1 | 4 | 2 | 1 | 4 | 2 |
| Xanthan Gum | 0,2 | 0,2 | 0,2 | 0,2 | 0,2 | 0,2 |
| Titandioxid (Anatas) | 0,5 | 0,5 | 0,5 | 0,5 | 0,5 | 0,5 |
| Aromakomposition FM-21 (d) | 1 | | | | | |
| Aromakomposition FM-21 (g) | | | | 1 | | |
| Aromakomposition FM-23 (a) | | 1,25 | | | | |
| Aromakomposition FM-23 (d) | | | | | 1,25 | |
| Aromakomposition FM-23 (e) | | | 1,5 | | | |
| Aromakomposition FM-23 (g) | | | | | | 1,5 |
| Wasser dest. | Ad 100 | Ad 100 | Ad 100 | Ad 100 | Ad 100 | Ad 100 |

### Formulierungsbeispiel FM-32: Zahnpasten mit Zinn- und Zinksalzen

| **Zubereitung** | **1** | **2** | **3** | **4** | **5** | **6** |
|---|---|---|---|---|---|---|
| **Bestandteil** | **Anteil (Gew.-%)** | | | | | |
| Natriumfluorid NaF | 0,42 | 0,5 | - | 0,42 | 0,5 | - |
| Zinnfluorid SnF2 | - | 0,9 | 0,95 | - | 0,9 | 0,95 |
| Zinnchlorid SnCl2 | 1,5 | - | 2 | 1,5 | - | 2 |
| Zinklactat | 2 | 2 | - | 2 | 2 | - |
| Zinkcarbonat ZnCO3 | - | 1 | 1,5 | - | 1 | 1,5 |
| Na-gluconat | - | 0,67 | 1,5 | - | 0,67 | 1,5 |
| Poloxamer 407 | 14,5 | - | - | 14,5 | - | - |
| Polyethylenglycol | 1 | 3 | - | 1 | 3 | - |
| Sorbitol, 70 %ig in Wasser | - | 38 | 37,5 | - | 38 | 37,5 |
| Glycerin | 37,5 | 5 | 14,4 | 37,5 | 5 | 14,4 |
| 1,2-Propylenglycol | 7 | 5 | - | 7 | 5 | - |
| Na-Saccharin | 0,3 | 0,5 | 0,5 | 0,3 | 0,5 | 0,5 |
| Abrasiv-Silica | 20 | 22,5 | 25 | 20 | 22,5 | 25 |
| Natriumhydroxid | - | 0,1 | 0,2 | - | 0,1 | 0,2 |
| Natriumlaurylsulfat | - | 2 | 1,5 | - | 2 | 1,5 |
| Na-polyphosphat | - | - | 4 | - | - | 4 |
| Tetranatriumpyrophosphat | 1 | 2,5 | - | 1 | 2,5 | - |
| Farbstoff (1%ig in Wasser) | 0,4 | 0,5 | 0,5 | 0,4 | 0,5 | 0,5 |
| Aromakomposition FM-21 (b) | 0,95 | - | - | | - | - |
| Aromakomposition FM-21 (c) | | | | 0,95 | | |
| Aromakomposition FM-23 (c) | - | 1,2 | - | - | | - |
| Aromakomposition FM-23 (f) | | | | | 1,2 | |
| Aromakomposition FM-24 (a) | - | - | 1,15 | - | - | |
| Aromakomposition FM-24 (e) | | | | | | 1,15 |
| Wasser dest. | Ad 100 | Ad 100 | Ad 100 | Ad 100 | Ad 100 | Ad 100 |

### Formulierungsbeispiel FM-33: Zahnpasta auf Phosphatbasis

| **Zubereitung** | **1** | **2** | **3** | **4** | **5** | **6** |
|---|---|---|---|---|---|---|
| **Bestandteil** | **Anteil (Gew.-%)** | | | | | |
| Glycerin | 20 | 20 | 20 | 20 | 20 | 20 |
| Solbrol M (Natriumsalz) | 0,15 | 0,15 | 0,15 | 0,15 | 0,15 | 0,15 |
| Natriummonofluorphosphat | 0,76 | 0,76 | 0,76 | 0,76 | 0,76 | 0,76 |
| Saccharin | 0,2 | 0,2 | 0,2 | 0,2 | 0,2 | 0,2 |
| Dicalciumphosphat-Dihydrat | 36 | 36 | 36 | 36 | 36 | 36 |
| Aerosil^{®} 200 (Silica) | 3 | 3 | 3 | 3 | 3 | 3 |
| Natriumcarboxymethylcellulose | 1,2 | 1,2 | 1,2 | 1,2 | 1,2 | 1,2 |
| Natriumlaurylsulfat (Texapon) | 1,3 | 1,3 | 1,3 | 1,3 | 1,3 | 1,3 |
| Aromakomposition FM-22 (a) | 1 | | | | | |
| Aromakomposition FM-22 (b) | | 1 | | | | |
| Aromakomposition FM-22 (d) | | | 0,8 | | | |
| Aromakomposition FM-22 (f) | | | | 0,8 | | |
| Aromakomposition FM-22 (g) | | | | | 1 | |
| Aromakomposition FM-22 (h) | | | | | | 1 |
| Entionisiertes Wasser | 36,39 | 36,39 | 36,59 | 36,59 | 36,39 | 36,39 |

### Formulierungsbeispiel FM-34: Zahnpasta (transparente Gelformulierung)

| **Zubereitung** | **1** | **2** | **3** | **4** | **5** | **6** |
|---|---|---|---|---|---|---|
| **Bestandteil** | **Anteil (Gew.-%)** | | | | | |
| Sorbitol 70% | 63 | Ad 100 | 63 | Ad 100 | 63 | Ad 100 |
| Entionisiertes Wasser | 11,31 | 11,31 | 11,31 | 11,31 | 11,31 | 11,31 |
| Saccharin | 0,2 | 0,2 | 0,2 | 0,2 | 0,2 | 0,2 |
| Natriummonofluorphosphat | 1,14 | 1,14 | 1,14 | 1,14 | 1,14 | 1,14 |
| Solbrol | 0,15 | 0,15 | 0,15 | 0,15 | 0,15 | 0,15 |
| Trinatriumphosphat | 0,1 | 0,1 | 0,1 | 0,1 | 0,1 | 0,1 |
| PEG 1500 ( PEG 32 ) | 5 | 5 | 5 | 5 | 5 | 5 |
| Sident 9 (Abrasive Silica) | 8 | 8 | 8 | 8 | 8 | 8 |
| Sident 22 S (Verdickende Silica) | 8 | 8 | 8 | 8 | 8 | 8 |
| Natriumcarboxymethylcellulose | 0,6 | 0,6 | 0,6 | 0,6 | 0,6 | 0,6 |
| Natriumlaurylsulfat | 1,5 | 1,5 | 1,5 | 1,5 | 1,5 | 1,5 |
| Pellitorin-Lösung PLM (enthaltend 10% Pellitorin) | - | 0,025 | - | 0,025 | - | 0,025 |
| Aromakomposition FM-23 (c) | 1 | - | | | | |
| Aromakomposition FM-23 (e) | | 1 | | | | |
| Aromakomposition FM-23 (h) | | | 1 | | | |
| Aromakomposition FM-24 (b) | - | | | 1 | | |
| Aromakomposition FM-24 (d) | | | | | 1 | |
| Aromakomposition FM-24 (g) | | | | | | 1 |

### Formulierungsbeispiel FM-35: Mundwasserkonzentrat mit Aroma vom Wintergrüntyp

| **Zubereitung** | **1** | **2** | **3** | **4** | **5** | **6** |
|---|---|---|---|---|---|---|
| **Bestandteil** | **Anteil (Gew.-%)** | | | | | |
| Ethylalkohol 96% | 42 | 42 | 42 | 42 | 42 | 42 |
| Cremophor RH 455 | 5 | 5 | 5 | 5 | 5 | 5 |
| Entionisiertes Wasser | 48,67 | 48,67 | 50,67 | 49,67 | 48,67 | 48,67 |
| Allantoin | 0,2 | 0,2 | 0,2 | 0,2 | 0,2 | 0,2 |
| Natriumsaccharin 450 | 0,1 | 0,1 | 0,1 | 0,1 | 0,1 | 0,1 |
| Colour L-Blue 5000 (1% in Wasser) | 0,03 | 0,03 | 0,03 | 0,03 | 0,03 | 0,03 |
| Aromakomposition FM-24 (b) | 4 | | | | | |
| Aromakomposition FM-24 (c) | | 4 | | | | |
| Aromakomposition FM-24 (d) | | | 2 | | | |
| Aromakomposition FM-24 (e) | | | | 3 | | |
| Aromakomposition FM-24 (f) | | | | | 4 | |
| Aromakomposition FM-24 (h) | | | | | | 4 |

### Formulierungsbeispiel FM-36: Mundwasser (,ready to use' ohne Alkohol)

| **Zubereitung** | **1** | **2** | **3** | **4** | **5** | **6** |
|---|---|---|---|---|---|---|
| **Bestandteil** | **Anteil (Gew.-%)** | | | | | |
| Cremophor RH 455 | 1,8 | 1,8 | 1,8 | 1,8 | 1,8 | 1,8 |
| Sorbitol 70% | 10 | 10 | 10 | 10 | 10 | 10 |
| Natriumfluorid | 0,18 | 0,18 | 0,18 | 0,18 | 0,18 | 0,18 |
| Natriumsaccharin 450 | 0,1 | 0,1 | 0,1 | 0,1 | 0,1 | 0,1 |
| Solbrol M Natriumsalz | 0,15 | 0,15 | 0,15 | 0,15 | 0,15 | 0,15 |
| Pellitorin-Lösung PLM (enthaltend 10% Pellitorin) | - | 0,0125 | - | 0,0125 | - | 0,0125 |
| Aromakomposition FM-28 (a) | 0,2 | 0,2 | | | | |
| Aromakomposition FM-28 (d) | | | 0,2 | 0,2 | | |
| Aromakomposition FM-28 (g) | | | | | 0,2 | 0,2 |
| Entionisiertes Wasser | 87,57 | Ad 100 | 87,57 | Ad 100 | 87,57 | Ad 100 |

### Formulierungsbeispiel FM-37: Mundwasser (,ready to use' mit Alkohol)

| **Zubereitung** | **1** | **2** | **3** | **4** | **5** | **6** |
|---|---|---|---|---|---|---|
| **Bestandteil** | **Anteil (Gew.-%)** | | | | | |
| Ethylalkohol 96% | 10 | 5 | 7 | 10 | 5 | 7 |
| Cremophor CO 40 | 1 | 1 | 1 | 1 | 1 | 1 |
| Benzoesäure | 0,1 | 0,12 | 0,1 | 0,1 | 0,12 | 0,1 |
| Sorbitol 70% | 5 | 1 | 5 | 5 | 1 | 5 |
| Natriumsaccharin 450 | 0,07 | 0,05 | 0,05 | 0,07 | 0,05 | 0,05 |
| L-Blue 5000 (1% in Wasser) | 0,1 | 0,1 | 0,1 | 0,1 | 0,1 | 0,1 |
| Glycerin | - | 8 | - | - | 8 | - |
| 1,2-Propylenglycol | - | 2 | 3 | - | 2 | 3 |
| Cetylpyridiniumchlorid | - | - | 0,07 | - | - | 0,07 |
| Wasserstoffperoxid (35% H2O2 in Wasser) | - | 3 | 4 | - | 3 | 4 |
| Aromakomposition FM-24 (d) | 0,25 | - | - | | - | - |
| Aromakomposition FM-24 (h) | | | | 0,25 | | |
| Aromakomposition FM-28 (b) | - | 0,25 | 0,25 | | | |
| Aromakomposition FM-28 (c) | | | | | 0,25 | 0,25 |
| Entionisiertes Wasser | 83,8 | Ad 100 | Ad 100 | 83,8 | Ad 100 | Ad 100 |

### Formulierungsbeispiel FM-38: Zahncreme und Mundwasser als 2-in-1 Produkt

| **Zubereitung** | **1** | **2** | **3** | **4** | **5** | **6** |
|---|---|---|---|---|---|---|
| **Bestandteil** | **Anteil (Gew.-%)** | | | | | |
| Ethanol, 96%ig | 5 | 5 | 5 | 5 | 5 | 5 |
| Sorbitol, 70 %ig in Wasser | 40 | 40 | 40 | 40 | 40 | 40 |
| Glycerin | 20 | 20 | 20 | 20 | 20 | 20 |
| Saccharin | 0,2 | 0,2 | 0,2 | 0,2 | 0,2 | 0,2 |
| Na-Monofluorphosphat | 0,76 | 0,76 | 0,76 | 0,76 | 0,76 | 0,76 |
| Solbrol M, Na-Salz | 0,15 | 0,15 | 0,15 | 0,15 | 0,15 | 0,15 |
| Abrasivkieselsäure (Sident 9) | 20 | 20 | 20 | 20 | 20 | 20 |
| Verdickungskieselsäure (Sident 22S) | 2 | 2 | 2 | 2 | 2 | 2 |
| Na-Carboxymethylcellulose | 0,3 | 0,3 | 0,3 | 0,3 | 0,3 | 0,3 |
| Natriumlaurylsulfat | 1,2 | 1,2 | 1,2 | 1,2 | 1,2 | 1,2 |
| Grüner Farbstoff (1%ig in Wasser) | 0,5 | 0,5 | 0,5 | 0,5 | 0,5 | 0,5 |
| Aromakomposition FM-21 (a) | 1 | | | | | |
| Aromakomposition FM-21 (b) | | 1 | | | | |
| Aromakomposition FM-21 (c) | | | 1 | | | |
| Aromakomposition FM-21 (e) | | | | 1 | | |
| Aromakomposition FM-21 (f) | | | | | 1 | |
| Aromakomposition FM-21 (h) | | | | | | 1 |
| Wasser dest. | Ad 100 | Ad 100 | Ad 100 | Ad 100 | Ad 100 | Ad 100 |

### Formulierungsbeispiel FM-39: Standard Kaugummi

| **Zubereitung** | **1** | **2** | **3** | **4** | **5** | **6** |
|---|---|---|---|---|---|---|
| **Bestandteil** | **Anteil (Gew.-%)** | | | | | |
| Gum Base (Kaugummibase) | 21 | 21 | 21 | 21 | 21 | 21 |
| Glucosesirup | 16,5 | 17 | 16,5 | 16,5 | 17 | 16,5 |
| Glycerin | 0,5 | 0,5 | 0,5 | 0,5 | 0,5 | 0,5 |
| Zucker gepulvert | 60 | 60 | 60 | 60 | 60 | 60 |
| Aromakomposition FM-25 (b) | 2 | - | | | | |
| Aromakomposition FM-25 (c) | | 1,5 | | | | |
| Aromakomposition FM-25 (h) | | | 2 | | | |
| Aromakomposition FM-26 (c) | - | | | 2 | | |
| Aromakomposition FM-26 (d) | | | | | 1,5 | |
| Aromakomposition FM-26 (g) | | | | | | 2 |

### Formulierungsbeispiel FM-40: Zuckerfreier Kaugummi

| **Zubereitung** | **1** | **2** | **3** | **4** | **5** | **6** |
|---|---|---|---|---|---|---|
| **Bestandteil** | **Anteil (Gew.-%)** | | | | | |
| Gum Base (Kaugummibase) | 30 | 30 | 30 | 30 | 30 | 30 |
| Isomalt gepulvert | 9,5 | 9,5 | 9,5 | 9,5 | 9,5 | 9,5 |
| Xylitol | 2 | 2 | 2 | 2 | 2 | 2 |
| Mannit D | 3 | 3 | 3 | 3 | 3 | 3 |
| Aspartam | 0,1 | 0,1 | 0,1 | 0,1 | 0,1 | 0,1 |
| Acesulfam K | 0,1 | 0,1 | 0,1 | 0,1 | 0,1 | 0,1 |
| EmulgumTM (Soja-Lecithine mit hohem Gehalt an Phospholipiden) | 0,3 | 0,3 | 0,3 | 0,3 | 0,3 | 0,3 |
| Sorbitol (70% in Wasser) | 13 | 13 | 13 | 13 | 13 | 13 |
| 1,2-Propylenglyol | - | 1 | - | 1 | - | 1 |
| Glycerin | 1 | - | 1 | - | 1 | - |
| Pellitorin-Lösung PLM (enthaltend 10% Pellitorin) | - | 0,035 | - | 0,035 | - | 0,035 |
| Aromakomposition FM-25 (a) | 1 | 1 | | | | |
| Aromakomposition FM-25 (d) | | | 0,8 | 0,8 | | |
| Aromakomposition FM-25 (f) | | | | | 1 | 1 |
| Sorbit gepulvert | 40 | Ad 100 | 40,2 | Ad 100 | 40 | Ad 100 |

### Formulierungsbeispiel FM-41: Kaugummis (mit Zucker und zuckerfrei)

| **Zubereitung** | **1** | **2** | **3** | **4** | **5** | **6** |
|---|---|---|---|---|---|---|
| **Bestandteil** | **Anteil (Gew.-%)** | | | | | |
| Gum Base (Kaugummibase) | 21 | 30 | 21 | 30 | 21 | 30 |
| Glycerin | 0,5 | 1 | 0,5 | 1 | 0,5 | 1 |
| Glucosesirup | 16,5 | - | 16,5 | - | 16,5 | - |
| Palatinit | - | 9,5 | - | 9,5 | - | 9,5 |
| Xylitol | - | 2 | - | 2 | - | 2 |
| Mannitol | - | 3 | - | 3 | - | 3 |
| Aspartam | - | 0,1 | - | 0,1 | - | 0,1 |
| Acesulfam K | - | 0,1 | - | 0,1 | - | 0,1 |
| EmulgumTM (Emulgator) | - | 0,3 | - | 0,3 | - | 0,3 |
| Sorbitol 70%, in Wasser | - | 14 | - | 14 | - | 14 |
| Aromakomposition FM-26 (a) | 1 | 1,4 | | | | |
| Aromakomposition FM-26 (f) | | | 0,8 | 1,2 | | |
| Aromakomposition FM-26 (h) | | | | | 1 | 1,4 |
| Puderzucker | Ad 100 | - | Ad 100 | - | Ad 100 | - |
| Sorbitol (in Pulverform) | - | Ad 100 | - | Ad 100 | - | Ad 100 |

### Formulierungsbeispiel FM-42: Zuckerfreie Kaugummis

Die Kaugummibase K1 bestand aus 2,0% Butyl Rubber (Isobuten-Isopren-Copolymer, MW = 400000, 6,0% Polyisobuten (MW = 43.800), 43,5% Polyvinylacetat (MW = 12.000), 31,5% Polyvinylacetat (MW = 47.000), 6,75% Triacetin und 10,25% Calciumcarbonat. Die Herstellung der Kaugummibase K1 und der Kaugummis kann analog zu US 5,601,858 erfolgen.

| **Zubereitung** | **1** | **2** | **3** | **4** | **5** | **6** |
|---|---|---|---|---|---|---|
| **Bestandteil** | **Anteil (Gew.-%)** | | | | | |
| Kaugummibase K1 | 26 | 27 | 26 | 26 | 27 | 26 |
| Triacetin | 0,25 | 0,25 | 0,25 | 0,25 | 0,25 | 0,25 |
| Lecithin | 0,5 | 0,5 | 0,5 | 0,5 | 0,5 | 0,5 |
| Mannitol | 15,3 | 15,2 | 15,1 | 15,3 | 15,2 | 15,1 |
| Glycerin | 12,1 | 12 | 11,8 | 12,1 | 12 | 11,8 |
| Saccharin-Na | 0,17 | - | 0,1 | 0,17 | - | 0,1 |
| Verkapseltes Aspartam | 1,08 | 1,18 | 1,08 | 1,08 | 1,18 | 1,08 |
| Amorphes Silica | 1 | 1 | 1 | 1 | 1 | 1 |
| Baumwollsamenöl | 0,5 | 0,5 | 0,5 | 0,5 | 0,5 | 0,5 |
| Polyoxyethylen-sorbitanmonolaurat (E-432) | 1 | 1 | 1 | 1 | 1 | 1 |
| Verkapseltes I-Carvon (Beladung: 30%) | - | 0,2 | - | - | 0,2 | - |
| I-Menthyl-I-lactat | - | - | 0,2 | - | - | 0,2 |
| Aromakomposition FM-26 (c) | 1 | - | 1,7 | | | |
| Aromakomposition FM-26 (d) | | | | 0,8 | - | 1,4 |
| Aromakomposition FM-25 (b) | 0,5 | 1,4 | - | | | |
| Aromakomposition FM-25 (e) | | | | 0,5 | 1,4 | - |
| Sorbitol, kristallin | Ad 100 | Ad 100 | Ad 100 | Ad 100 | Ad 100 | Ad 100 |

### Formulierungsbeispiel FM-43: Zuckerfreie Kaugummis

Die Kaugummibase K2 bestand aus 28,5% Terpenharz, 33,9% Polyvinylacetat (MW = 14.000), 16,25% hydriertes Pflanzenöl, 5,5% Mono- und Diglyceride, 0,5% Polyisobuten (MW 75.000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis kann analog zu US 6,986,907 erfolgen.

| **Zubereitung** | **1** | **2** | **3** | **4** | **5** | **6** |
|---|---|---|---|---|---|---|
| **Bestandteil** | **Anteil (Gew.-%)** | | | | | |
| Kaugummibase K2 | 25,3 | 27,3 | 26,3 | 25,3 | 27,3 | 26,3 |
| Glycerin | 2,4 | 2,4 | 2,4 | 2,4 | 2,4 | 2,4 |
| Lecithin | 7 | 7 | 7 | 7 | 7 | 7 |
| Aspartam | 0,14 | 0,14 | 0,14 | 0,14 | 0,14 | 0,14 |
| Verkapseltes Aspartam | 0,68 | 0,68 | 0,68 | 0,68 | 0,68 | 0,68 |
| Menthol, sprühgetrocknet (Beladung: 25%) | 0,5 | - | 0,5 | 0,5 | - | 0,5 |
| Kirscharoma, sprühgetrocknet (enthält Benzaldehyd) | - | 1 | - | - | 1 | - |
| Aromakomposition FM-25 (b), sprühgetrocknet | 1,5 | 1,7 | - | | | |
| Aromakomposition FM-25 (c), sprühgetrocknet | | | | 1,5 | 1,7 | - |
| Aromakomposition FM-27 (c) | 1 | - | 1,5 | | | |
| Aromakomposition FM-27 (h) | | | | 1 | - | 1,5 |
| Sorbitol | Ad 100 | Ad 100 | Ad 100 | Ad 100 | Ad 100 | Ad 100 |

Die Kaugummis der Rezeptur (1) und (2) wurden als Streifen, die der Rezeptur (3) als Kompaktate in Kissenform hergestellt und anschließend mit Xylit dragiert.

**Formulierungsbeispiel FM-44:** Herstellung von Aromen mit Kühlwirkung vom Geschmackstyp "Eisbonbon" unter Verwendung der Kühlsubstanzer Die Formulierungsbeispiele c-f sind dabei nicht erfindungsgemäß.

| **Zubereitung** | **a** | **b** | **c** | **d** | **e** | **f** | **g** | **h** |
|---|---|---|---|---|---|---|---|---|
| **Komponente** | **Anteil (Gew.-%)** | | | | | | | |
| Isoamylacetat | 2 | 2 | 2 | 2 | 2 | 2 | 2 | 2 |
| Ethylbutyrat | 0,5 | - | 0,5 | - | 0,5 | - | 0,5 | - |
| Butylbutyrat | - | 0,5 | - | 0,5 | - | 0,5 | - | 0,5 |
| Ethylvanillin | 2 | - | 2 | - | 2 | - | 2 | - |
| Vanillin | - | 1 | - | 1 | - | 1 | - | 1 |
| FrambinonTM [4-(4-Hydroxyphenyl)-2-butanon] | 0,5 | 0,5 | 0,5 | 0,5 | 0,5 | 0,5 | 0,5 | 0,5 |
| I-Menthol | 5 | 5 | 5 | 5 | 5 | 5 | 5 | 5 |
| Pfefferminzöl Typ piperita | 6 | 6 | 6 | 6 | 6 | 6 | 6 | 6 |
| Triacetin | - | 84 | - | 84,5 | - | 84,5 | - | 84 |
| 1,2-Propylenglycol | 83 | - | 83,5 | - | 83,5 | - | 83 | - |
| 8 Gew.% BDDPA in 92 Gew% Benzylalkohol | 0,5 | 0,5 | | | | | | |
| 2 Gew% BDDPA in 98 Gew% Frescolat MPC | 0,5 | 0,5 | | | | | | |
| 5 Gew% BDDPA in 95Gew% Anethol | | | 0,5 | 0,5 | 0,2 | 0,2 | | |
| 5 Gew% BDDPA in 47,5 Gew% Triethylcitrat + 47,5 Gew% Pfefferminzöl | | | | | 0,3 | 0,3 | | |
| 5 Gew% BDDPA in 31,6 Gew% Triethylcitrat + 31,6 Gew% Triacetin + 31,6 Gew% Pfefferminzöl | | | | | | | 0,5 | 0,5 |
| 3 Gew% BDDPA in 47 Gew% 2-Phenylethanol + 50 Gew% Frescolat^{®} MPC | | | | | | | 0,5 | 0,5 |

Die Aromen wurden mit Konzentrationen von 0,15 bis 0,2 Gew-% in verschiedene Bonbonmassen eingearbeitet.

### Formulierungsbeispiel FM-45: Bonbon ('Hardboiled candy'), zuckerfrei

| **Zubereitung** | **1** | **2** | **3** | **4** | **5** | **6** |
|---|---|---|---|---|---|---|
| **Bestandteil** | **Anteil (Gew.-%)** | | | | | |
| Wasser | 2,24 | 2,24 | 2,24 | 2,24 | 2,24 | 2,24 |
| Isomalt | 94,98 | Ad 100 | Ad 100 | 94,98 | Ad 100 | Ad 100 |
| Xylitol | 2,4 | 2,4 | 2,4 | 2,4 | 2,4 | 2,4 |
| Sucralose | 0,03 | 0,03 | 0,03 | 0,03 | 0,03 | 0,03 |
| Acesulfam K | 0,05 | 0,05 | 0,05 | 0,05 | 0,05 | 0,05 |
| Citronensäure | 0,05 | 0,05 | 0,05 | 0,05 | 0,05 | 0,05 |
| Pellitorin-Lösung PLM (enthaltend 10% Pellitorin) | - | 0,0075 | 0,01 | - | 0,0075 | 0,01 |
| Aromakomposition FM-21 (c) | 0,25 | 0,2 | | | | |
| Aromakomposition FM-21 (d) | | | | 0,25 | 0,2 | |
| Aroma Typ Eisbonbon (Beispiel FM-44 (a)) | | | 0,25 | | | |
| Aroma Typ Eisbonbon (Beispiel FM-44 (e)) | | | | | | 0,2 |

### Formulierungsbeispiel FM-46: Bonbons ('Hardboiled candy')

| **Zubereitung** | **1** | **2** | **3** | **4** | **5** | **6** |
|---|---|---|---|---|---|---|
| **Bestandteil** | **Anteil (Gew.-%)** | | | | | |
| Wasser | 2,75 | 2,5 | 2,5 | 2,75 | 2,5 | 2,5 |
| Zucker | 60,1 | Ad 100 | Ad 100 | 60,1 | Ad 100 | Ad 100 |
| Glucosesirup | 36,9 | 36 | 36 | 36,9 | 36 | 36 |
| Maltose | - | 2 | 2 | - | 2 | 2 |
| Palmkernöl | - | 0,8 | 0,8 | - | 0,8 | 0,8 |
| Citronensäure | - | 0,25 | 0,25 | - | 0,25 | 0,25 |
| Ginseng Extrakt | - | 0,4 | 0,4 | - | 0,4 | 0,4 |
| Blauer Farbstoff | - | 0,01 | 0,01 | - | 0,01 | 0,01 |
| Aromakomposition FM-22 (a) | 0,25 | 0,35 | | | | |
| Aromakomposition FM-22 (d) | | | | 0,25 | 0,35 | - |
| Aroma Typ Eisbonbon (Beispiel FM-44 (b)) | | | 0,175 | | | |
| Aroma Typ Eisbonbon (Beispiel FM-44 (d)) | | | | | | 0,175 |

### Formulierungsbeispiel FM-47: Instant-Getränkepulver

| **Zubereitung** | **1** | **2** | **3** | **4** | **5** | **6** |
|---|---|---|---|---|---|---|
| **Bestandteil** | **Anteil (Gew.-%)** | | | | | |
| Citronensäure | 11,58 | 11,58 | 11,58 | 11,58 | 11,58 | 11,58 |
| Trinatriumcitrat | 0,7 | 0,7 | 0,7 | 0,7 | 0,7 | 0,7 |
| Tricalciumphosphat | 0,6 | 0,6 | 0,6 | 0,6 | 0,6 | 0,6 |
| Vitamin C | 0,66 | 0,66 | 0,66 | 0,66 | 0,66 | 0,66 |
| Grindsted^{®} JU 543 Stabilizer System (Danisco) | 0,9 | 0,9 | 0,9 | 0,9 | 0,9 | 0,9 |
| Saccharin | 0,561 | 0,561 | 0,561 | 0,561 | 0,561 | 0,561 |
| Citronenaroma, sprühgetrocknet | 1,75 | - | 1,75 | - | 1,75 | - |
| Orangenaroma, sprühgetrocknet | | 1,85 | | 1,85 | | 1,85 |
| Aromakomposition FM-21 (a), sprühgetrocknet auf Maltodextrin (DE 15-19) und Gummi Arabicum, Aromabeladung 40% | 1,75 | | | | | |
| Aromakomposition FM-21 (c), sprühgetrocknet auf Maltodextrin (DE 15-19) und Gummi Arabicum, Aromabeladung 40% | | | 1,75 | | | |
| Aromakomposition FM-21 (h), sprühgetrocknet auf Maltodextrin (DE 15-19) und Gummi Arabicum, Aromabeladung 40% | | | | | 1,75 | |
| Aromakomposition FM-23 (b); sprühgetrocknet auf Maltodextrin (DE 15-19) und Gummi Arabicum, Aromabeladung 40% | | 1,2 | | | | |
| Aromakomposition FM-23 (d); sprühgetrocknet auf Maltodextrin (DE 15-19) und Gummi Arabicum, Aromabeladung 40% | | | | 1,2 | | |
| Aromakomposition FM-23 (e); sprühgetrocknet auf Maltodextrin (DE 15-19) und Gummi Arabicum, Aromabeladung 40% | | | | | | 1,2 |
| Zucker (Saccharose) | Ad 100 | Ad 100 | Ad 100 | Ad 100 | Ad 100 | Ad 100 |

45 g dieser Instant-Getränkepulver wurden jeweils in 1000 mL unter Rühren gelöst.

### Formulierungsbeispiel FM-48: Halsbonbons mit flüssig-viskoser Kernfüllung (centerfilled hard candy)

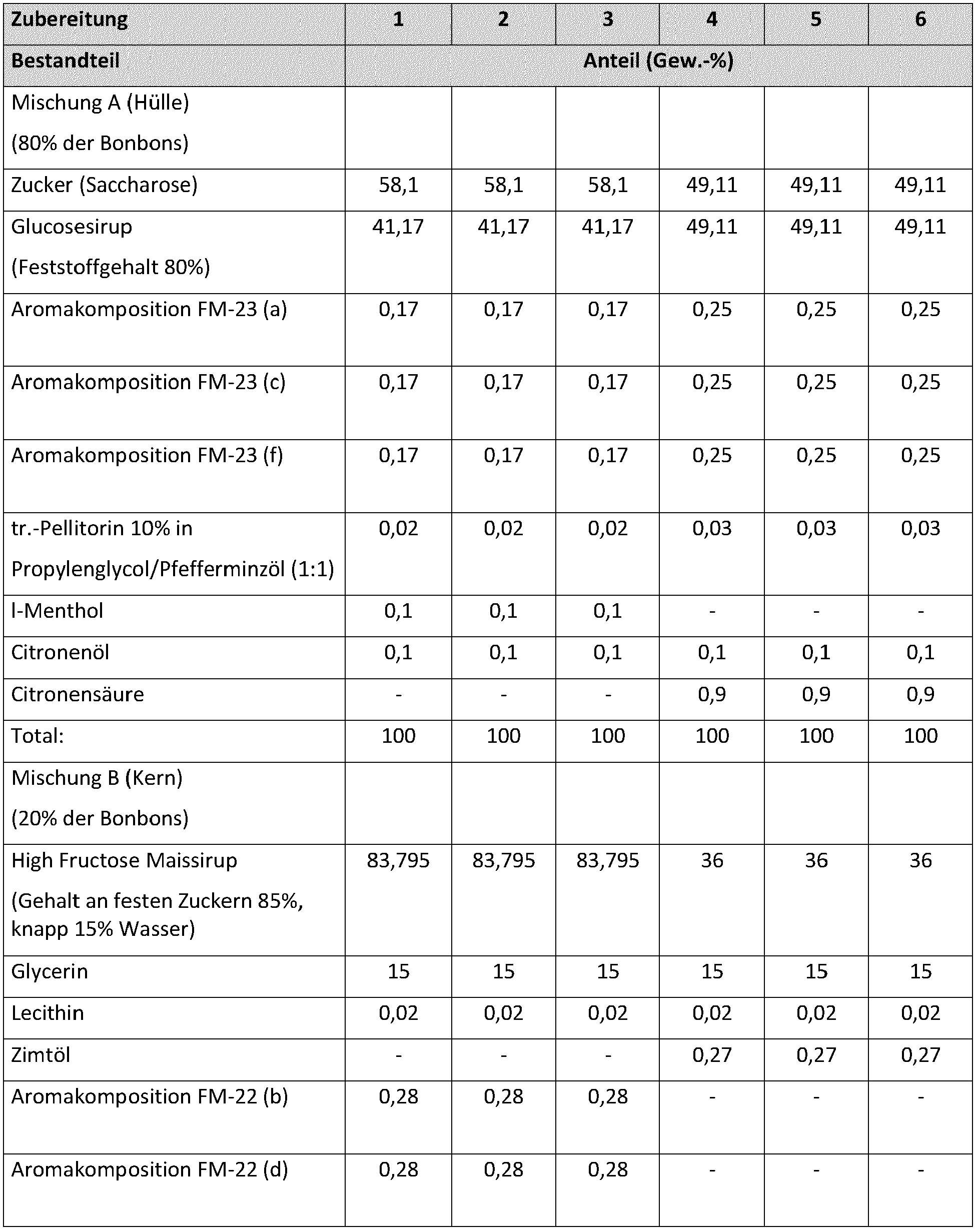

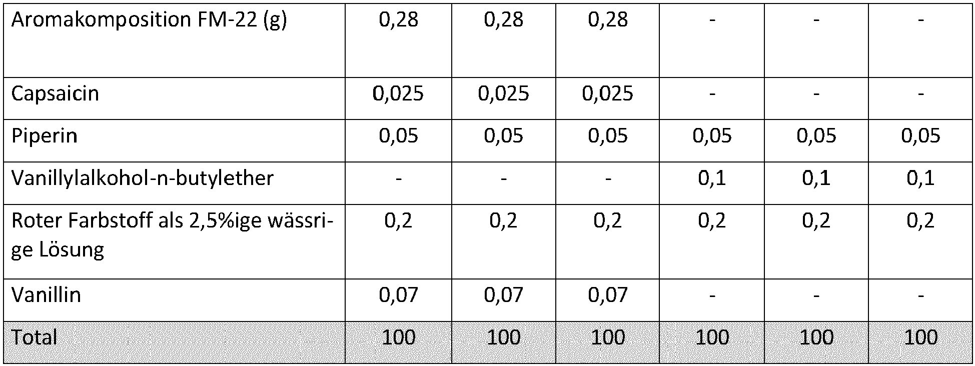

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Formulierungsbeispiel FM-49: Zum Direktverzehr geeignete Gelatinekapseln

| **Zubereitung** | **1** | **2** |
|---|---|---|
| **Bestandteile** | **Anteil (Gew.-%)** | **Anteil (Gew.-%)** |
| Gelatinehülle: | | |
| Glycerin | 2,014 | 2,014 |
| Gelatine 240 Bloom | 7,91 | 7,91 |
| Sucralose | 0,070 | 0,070 |
| Allura Red (roter Farbstoff) | 0,006 | 0,006 |
| Brillant Blue (blauer Farbstoff) | 0,005 | 0,005 |
| Kernzusammensetzung: | | |
| Aromakomposition FM-21 (a) | 15 | |
| Aromakomposition FM-21 (b) | | 15 |
| Pflanzenöltriglyceride (Kokonussölfraktion; coconut oil fraction) | Ad 100 | Ad 100 |

Die zum Direktverzehr geeigneten Gelatinekapseln wurden gemäß WO 2004/050069 hergestellt und hatten einen Durchmesser von 5 mm; das Gewichtsverhältnis von Kernmaterial zu Hüllmaterial lag bei 90 : 10.

### Formulierungsbeispiel FM-50:

Herstellung eines Kaubonbons mit kühlendem Himbeergeschmack unter Verwendung der Kühlsubstanzei. Die Zubereitungen 2-4 sind dabei nicht erfindungsgemäß.

| **Zubereitung** | 1 | **2** | **3** | **4** | **5** | **6** |
|---|---|---|---|---|---|---|
| **Bestandteile** | **Anteil (Gew.-%)** | | | | | |
| Wasser | 7,8 | 7,79 | 7,805 | 7,8 | 7,815 | 7,81 |
| Zucker Raffinade C4 | 42,1 | 42,1 | 42,1 | 42,1 | 42,1 | 42,1 |
| Glucose Sirup Dextrose 40 | 37,3 | 37,3 | 37,3 | 37,3 | 37,3 | 37,3 |
| gehärtetes Pflanzenfett Schmelzpunkt 32-36°C | 6,6 | 6,6 | 6,6 | 6,6 | 6,6 | 6,6 |
| Lecithin Emulgator (Sojalecithin) | 0,3 | 0,3 | 0,3 | 0,3 | 0,3 | 0,3 |
| Gelatine (Schweinegelatine) | 0,8 | 0,8 | 0,8 | 0,8 | 0,8 | 0,8 |
| Fondant Typ - S30 | 4,8 | 4,8 | 4,8 | 4,8 | 4,8 | 4,8 |
| Himbeeraroma | 0,22 | 0,22 | 0,22 | 0,22 | 0,22 | 0,22 |
| Menthyllactat | 0,06 | 0,06 | 0,06 | 0,06 | 0,06 | 0,06 |
| 8 Gew.% BDDPA in 92 Gew% Benzylalkohol | 0,02 | | | | | |
| 2 Gew% BDDPA in 98 Gew% Frescolat MPC | | 0,03 | | | | |
| 5,2 Gew% BDDPA in 31,6 Gew% Triethylcitrat + 31,6 Gew% Triacetin + 31,6 Gew% Pfefferminzöl | | | 0,015 | | | |
| 5 Gew% BDDPA in 95Gew% Anethol | | | | 0,02 | | |
| 3 Gew% BDDPA in 47 Gew% 2-Phenylethanol + 50 Gew% Frescolat^{®} MPC | | | | | 0,005 | |
| 5 Gew% BDDPA in 47,5 Gew% Triacetin + 47,5 Gew% Frescolat^{®} ML | | | | | | 0,01 |

Herstellungshinweise:
a) Gelatine mit Wasser (1,8 fache Menge der Gelatine) bei 70°C 2 Stunden quellen lassen;
b) Zucker, Sirup, Wasser, Fett und Lecithin auf 123°C kochen;
c) Gelatinelösung langsam mit dem Kochansatz vermischen;
d) Himbeeraroma, Menthyllactat und die erfindungsgemäßen Kühlsubstanzen vermischen und optional Farbe unterrühren;
e) resultierende Masse auf einem Kühltisch auf ca. 70°C temperieren, danach Fondant zugeben und auf einer Ziehmaschine ca. 3 Minuten belüften;
f) Kaubonbonmasse anschließend schneiden und verpacken.

### Formulierungsbeispiel FM-51:

Herstellung eines Extrudates für die Bereitung von Getränkemischungen mit Kühlwirkung
Alle Angaben, soweit nicht anders vermerkt, in Gew-%.

| **Zubereitung** | **1** | **2** | **3** |
|---|---|---|---|
| **Bestandteil** | **Anteil (Gew.-%)** | **Anteil (Gew.-%)** | **Anteil (Gew.-%)** |
| Glukosesirup, sprühgetrocknet (DE-Wert: 31-34) [Glucidex IT33W (Firma Roquette)] | 62,0 | 62,0 | 62,0 |
| Maltodextrin (DE-Wert: 17-20), Firma Cerestar | 28,4 | 28,4 | 28,4 |
| Emulgator Monomuls, Emulgator auf der Basis von gehärtetem Palmöl; Schmelzpunkt: 64°C, (Firma Grünau) | 1,8 | 1,8 | 1,8 |
| Dextrosemonohydrat (DE-Wert: 99,5), Firma Cerestar | 1,8 | 1,8 | 1,8 |
| Wasser | 2,0 | 2,0 | 2,0 |
| Orangen-Vanillearoma | 3,2 | 3,2 | 3,2 |
| Aromakomposition FM-21 (a) | 0,8 | | |
| Aromakomposition FM-21 (b) | | 0,8 | |
| Aromakomposition FM-21 (f) | | | 0,8 |

Herstellungshinweis (siehe auch WO 03/092412):
Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten.

### Formulierungsbeispiel FM-52:

Herstellung von Wirbelschichtgranulaten für die Bereitung von Getränkemischungen mit Kühlwirkung
In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 8 Gew.% Citronenaroma, 3 Gew.-% Aromakomposition FM-21 (a) bis (h), 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Formulierungsbeispiel FM-53:

Herstellung von Teebeuteln mit Rooibos- bzw. schwarzem Tee und Extrudaten aus Beispiel S-31 bzw. Granulaten aus Beispiel S-32 für die Bereitung von Teegetränken mit kühlernder Wirkung.

Jeweils 800 g Rotbuschtee (Rooibos-Tee) wurden einmal mit 33 g der Extrudate aus Beispiel FM-51 und einmal mit 30 g Granulaten aus Anwendungsbeispiel FM-52 gemischt, portioniert und anschließend in Teebeutel abgefüllt.

Jeweils 800 g schwarzer Tee (Blattgrad Fannings) wurden einmal mit 33 g der Extrudate aus Beispiel S-51 und einmal mit 30 g Granulaten aus Beispiel FM-52 gemischt, portioniert und anschließend in Teebeutel abgefüllt.

### Formulierungsbeispiel FM-54:

Herstellung einer zuckerhaltigen, bzw. zuckerreduzierten Eiscreme mit lang anhaltender Kühlwirkung unter Verwendung der erfindugsgemäßen Kühlsubstanzen

| **Zubereitung** | **1** | **2** | **3** | **4** | **5** | **6** |
|---|---|---|---|---|---|---|
| **Bestandteil** | **Anteil (Gew.-%)** | | | | | |
| Magermilch | 56,75 | 60,55 | 56,75 | 60,55 | 56,75 | 60,55 |
| Pflanzenfett, Schmelzbereich 35 - 40°C | 20 | 20 | 20 | 20 | 20 | 20 |
| Zucker (Saccharose) | 12 | 8 | 12 | 8 | 12 | 8 |
| Magermilchpulver | 5 | 5 | 5 | 5 | 5 | 5 |
| Glucosesirup 72 % Trockensubstanz | 5 | 5 | 5 | 5 | 5 | 5 |
| Emulgator SE 30 (Grindstedt Products, Dänemark) | 0,65 | 0,65 | 0,65 | 0,65 | 0,65 | 0,65 |
| Vanillearoma, enthaltend 1 % Vanillin und 2,5% einer Zusammensetzung aus BDDPA (8 Gew.%) + Benzylalkohol (92% Gew) | 0,2 | 0,2 | 0,2 | 0,2 | 0,2 | 0,2 |
| Vanillearoma, enthaltend 1 % Vanillin und 1% einer Zusammensetzung aus BDDPA (3 Gew%) + 2-Phenylethanol (47 Gew%) + Frescolat^{®} MPC (50 Gew%) | 0,2 | 0,2 | 0,2 | 0,2 | 0,2 | 0,2 |
| Vanillearoma, enthaltend 1 % Vanillin und 2% einer Zusammensetzung aus BDDPA (2 Gew%) + Frescolat MPC (98 Gew%) | 0,2 | 0,2 | 0,2 | 0,2 | 0,2 | 0,2 |
| Hesperitin, 2,5% in 1,2-Propylenglycol | 0 | 0,2 | 0 | 0,2 | 0 | 0,2 |

Magermilch und Glucosesirup wurden auf 55° erhitzt und Zucker, Magermilchpulver sowie Emulgator hinzugefügt. Das Pflanzenfett wurde vorerhitzt und die gesamte Masse auf 58°C erwärmt. Nach Zugabe des Aromas wurde mit Hilfe eines Durchflusshochdruckhomogenisators homogenisiert (180 / 50 bar). Die erhaltene Masse wurde 1 min lang bei 78°C temperiert, anschließend auf 2 - 4 °C abgekühlt und 10 h zur Reifung bei dieser Temperatur inkubiert. Danach wurde die gereifte Masse abgefüllt und bei -18°C eingefroren gelagert.

### Formulierungsbeispiel FM-55:

Herstellung von zuckerhaltigen und zuckerreduzierten Erfrischungsgetränken verschiedener Geschmacksrichtungen und einem lang anhaltenden erfrischenden Kühlempfinden unter Verwendung der erfindungsgemäßen Mischungen.

| **Komponente** | **Anteil** | **Zubereitung** | | | | | | | |
|---|---|---|---|---|---|---|---|---|---|
| | | **1** | **2** | **3** | **4** | **5** | **6** | **7** | **8** |
| Saccharose | % | 10,5 | 8 | 8 | 8 | 8 | 8 | 8 | 8 |
| Citronensäure | % | 0,2 | 0,2 | 0,2 | 0,2 | 0,2 | 0,2 | 0,2 | 0,2 |
| Hesperetin 1 % in 1,2-Propylenglycol | % | | 0,05 | 0,05 | 0,05 | 0,05 | 0,05 | 0,05 | 0,05 |
| Phloretin 1 % in 1,2-Propylenglycol | % | | 0,05 | 0,05 | 0,05 | 0,05 | 0,1 | 0,1 | 0,1 |
| Ethylhydroxymethylfuranon | ppb | 0,01 | 0,01 | | | | | | |
| Vanillin | ppb | 15 | 15 | | | | | | |
| Diethylmalonat | ppb | | | 70 | | | | | |
| Phenylethylacetat | ppb | | | 1 | | | | | |
| 2-Methylbutanal | ppb | | | | 0,3 | | 0,3 | | |
| Isovaleraldehyd | ppb | | | | 0,2 | | 0,2 | | |
| Furfurylacetat | ppb | | | | 0,3 | | | | |
| Massoilacton | ppb | | | | | 5 | 5 | | 5 |
| γ-Octalacton | ppb | | | | | 5 | 5 | | 5 |
| Ethylbutyrat | ppb | | | | 0,5 | | 0,5 | | 0,5 |
| Maltol | ppb | 350 | 350 | | | | 350 | | 350 |
| 2,5-Dimethyl-4-hydroxy-2H-furan-3-on | ppb | 3 | 3 | | | | 3 | | 3 |
| Ethylisobutyrat | ppb | | | 0,1 | | | 0,1 | | 0,1 |
| Ethyl-2-methylbutyrat | ppb | | | 0,1 | | | 0,1 | | 0,1 |
| 8 Gew.% BDDPA in 92 Gew% Benzylalkohol | ppm | 60 | 60 | 120 | 60 | 60 | 120 | 60 | 120 |
| Butylphenyacetat | ppb | | | | | | 10 | | |
| Acetanisol | ppb | | | | | | 20 | | |
| Methylsorbat | ppb | | | | | | 100 | | |
| L-Lysin | ppm | | | | | | | 100 | 30 |
| Äpfelsäure | ppm | | | | | | | 80 | |
| L-Arginin | ppm | | | | | | | 5 | 20 |
| L-Asparaginsäure | ppm | | | | | | | 0,5 | |
| Calciumchlorid | ppm | | | | | | | 20 | |
| Glutamin | ppm | | | | | | | 2 | |
| Kaliumhydrogenphosphat | ppm | | | | | | | 6 | |
| Magnesiumchlorid | ppm | | | | | | | 20 | |
| L-Valin | ppm | | | | | | | 0,5 | |
| Glycin | ppm | | | | | | | | 40 |
| L-Alanin | ppm | | | | | | | | 20 |
| L-Serin | ppm | | | | | | | | 50 |
| Wasser | Ad 100 | | | | | | | | |

Die Stoffe wurden vorgelegt und mit Wasser auf 100 % aufgefüllt und gelöst. Das Produkt wurde im Bedarfsfall in Flaschen abgefüllt und carbonisiert.

### Formulierungsbeispiel FM-56:

Herstellung eines Fruchtgummis mit einem lang anhaltenden frischen kühlenden Geschmack unter Verwendung der Kühlsubstanzer Die Zubereitungen 2-5 sind dabei nicht erfindungsgemäß.

| **Zubereitung** | **1** | **2** | **3** | **4** | **5** | **6** |
|---|---|---|---|---|---|---|
| **Komponente** | **Anteil (Gew.-%)** | | | | | |
| | | | | | | |
| Wasser | 23,6 | 23,6 | 23,6 | 23,6 | 23,6 | 23,6 |
| Saccharose | 34,5 | 34,5 | 34,5 | 34,5 | 34,5 | 34,5 |
| Glucosesirup, DE 40 | 31,89 | 31,89 | 31,89 | 31,89 | 31,89 | 31,89 |
| Iso Syrup C* Tru Sweet 01750 (Cerestar GmbH) | 1,5 | 1,5 | 1,5 | 1,5 | 1,5 | 1,5 |
| Gelatine 240 Bloom | 8,2 | 8,2 | 8,2 | 8,2 | 8,2 | 8,2 |
| Gelber und roter Farbstoff | 0,01 | 0,01 | 0,01 | 0,01 | 0,01 | 0,01 |
| Citronensäure | 0,2 | 0,2 | 0,2 | 0,2 | 0,2 | 0,2 |
| Kirscharoma, enthaltend 10 Gew.-% einer Zusammensetzung aus BDDPA (8 Gew.%) + Benzylalkohol (92Gew%), bezogen auf das Aroma | 0,1 | | | | | |
| Kirscharoma, enthaltend 10 Gew.-% einer Zusammensetzung aus BDDPA (2 Gew%) + Frescolat MPC (98 Gew%), bezogen auf das Aroma | | 0,1 | | | | |
| Kirscharoma, enthaltend 5 Gew.-% an einer Zusammenstzung aus BDDPA (5 Gew%) + Triethylcitrat (47,5 Gew%) + Pfefferminzöl (47,5 Gew%), bezogen auf das Aroma | | | 0,1 | | | |
| Kirscharoma, enthaltend 5 Gew.-% an einer Zusammensetzung aus BDDPA (5,2 Gew%) + Triethylcitrat (31,6 Gew%) + Triacetin (31,6 Gew% )+ Pfefferminzöl (31,6 Gew% ), bezogen auf das Aroma | | | | 0,1 | | |
| Kirscharoma, enthaltend 10 Gew.-% an einer Zusammensetzung ausBDDPA (5 Gew%) + Triacetin (47,5 Gew%) + Frescolat^{®} ML (47,5 Gew%), bezogen auf das Aroma | | | | | 0,1 | |
| Kirscharoma, enthaltend 10 Gew.-% an einer Zusammensetzung aus BDDPA (3 Gew%) + | | | | | | 0,1 |
| 2-Phenylethanol (47 Gew%) + Frescolat^{®} MPC (50 Gew%), bezogen auf das Aroma | | | | | | |

### Formulierungsbeispiel FM-57:

Herstellung von zuckerhaltigen und zuckerreduzierten kohlensäurehaltigen Erfrischungsgetränken der Geschmacksrichtung "Cola" mit erfrischenden lang anhaltendem Kühleffekt unter Verwendung der Mischunger Die Zubereitung 3 ist dabei nicht erfindungsgemäß.

| **Zubereitung** | **1** | **2** | **3** | **4** | **5** |
|---|---|---|---|---|---|
| **Komponente** | **Anteil (Gew.-%)** | | | | |
| Phosphorsäure 85% | 0,635 | 0,635 | 0,635 | 0,635 | 0,635 |
| Zitronensäure, wasserfrei | 0,064 | 0,064 | 0,064 | 0,064 | 0,064 |
| Koffein | 0,064 | 0,064 | 0,064 | 0,064 | 0,064 |
| Succrose | 63,600 | - | - | - | 12,9 |
| Sucralose | - | 0,126 | - | - | - |
| Erythritol | - | - | 6,000 | - | - |
| Aspartam | - | - | 0,350 | - | 0,07 |
| Steviosid | - | - | - | 0,300 | - |
| Acesulfam K | - | - | - | - | 0,07 |
| Zuckercoloeur | 0,800 | 0,800 | 0,800 | 0,800 | 0,800 |
| Getränke-Emulsion Typ: Cola | 1,445 | 1,445 | 1,445 | 1,445 | 1,445 |
| Natriumbenzoat | 0,106 | 0,106 | 0,106 | 0,106 | 0,106 |
| 8 Gew.% BDDPA in 92 Gew% Benzylalkohol | 0,30 | 0,15 | | | 0,30 |
| 5 Gew% BDDPA in 47,5 Gew% Triethylcitrat + 47,5 Gew% Pfefferminzöl | | 0,15 | | | |
| 5,2 Gew% BDDPA in 31,6 Gew% Triethylcitrat + 31,6 Gew% Triacetin + 31,6 Gew% Pfefferminzöl | | | 0,30 | | |
| 3 Gew% BDDPA in 47 Gew% 2-Phenylethanol + 50 Gew% Frescolat^{®} MPC | | | | 0,15 | |
| Wasser | ad 100 | ad 100 | ad 100 | ad 100 | ad 100 |

Die festen Bestandteile bzw. Inhaltsstoffe werden einzeln mit Wasser vermischt, zusammengegeben und mit Wasser auf 100 g aufgefüllt. Anschließend lässt man das erhaltene Konzentrat über Nacht bei Raumtemperatur altern. Schließlich wird 1 Teil Konzentrat mit 5 Teilen kohlensäurehaltigem Wasser gemischt, in Flaschen gefüllt und verschlossen.

### Formulierungsbeispiel FM-58:

Herstellung von Schokoladen mit einem lang anhaltenden kühlenden Geschmack unter Verwendung der Mischunger. Die Zubereitung 3 ist dabei nicht erfindungsgemäß.
1 = dunkle Schokolade
2 = kalorienreduzierte dunkle Schokolade
3 = kalorienreduzierte dunkle Schokolade
4 = kalorienreduzierte dunkle Schokolade
5 = kalorienreduzierte Vollmilchschokolade

| **Zubereitung** | **1** | **2** | **3** | **4** | **5** |
|---|---|---|---|---|---|
| **Komponente** | **Anteil (Gew.-%)** | | | | |
| Kakaobutter | 13,50 | 13,00 | 13,50 | 9,48 | 14,00 |
| KaKaomasse | 42,00 | 39,00 | 42,00 | 44,00 | 23,00 |
| Erythritol | - | 47,37 | - | - | - |
| Maltitol, kristallin | - | - | - | 22,945 | |
| Inulin | - | - | - | 23,00 | |
| Sorbitol | - | - | 43,97 | - | - |
| Lactitol | - | - | - | - | 38,47 |
| Polydextrose | - | - | - | - | 9,70 |
| Vollmilchpulver | - | - | - | - | 14,0 |
| Sucrose | 43,9 | - | - | - | - |
| Lecitin | 0,48 | 0,48 | 0,40 | 0,48 | 0,50 |
| Vanillin | 0,02 | 0,02 | 0,02 | 0,02 | 0,20 |
| Aspartam | - | 0,03 | 0,06 | - | 0,03 |
| 8 Gew.% BDDPA in 92Gew% Benzylalkohol | 0,1 | 0,1 | | | |
| 2 Gew% BDDPA in 98 Gew% Frescolat MPC | | | 0,05 | | |
| 3 Gew% BDDPA in 47 Gew% 2-Phenylethanol + 50 Gew% Frescolat^{®} MPC | | | | 0,075 | 0,1 |

### Formulierungsbeispiel FM-59:

Herstellung eines Biermix-Getränkes mit einem lang anhaltenden frischen kühlenden Geschmack: unter Verwendung der Kühlsubstanzer Die Zubereitungen 2-4 und 6 sind dabei nicht erfindungsgemäß.

Es wurden vermischt:

| **Zubereitung** | **1** | **2** | **3** | **4** | **5** | **6** |
|---|---|---|---|---|---|---|
| **Komponente** | **Anteil (Gew.-%)** | | | | | |
| Zuckersirup | 4 | 4 | 4 | 4 | 4 | 4 |
| Bier | 50 | 50 | 50 | 50 | 50 | 50 |
| Ethylalkohol | 4 | 4 | 4 | 4 | 4 | 4 |
| Zitronensäure | 0,15 | 0,15 | 0,15 | 0,15 | 0,15 | 0,15 |
| Ascorbinsäure | 0,01 | 0,01 | 0,01 | 0,01 | 0,01 | 0,01 |
| Grapefruitsaft | 6 | 6 | 6 | 6 | 6 | 6 |
| Grapefruitaroma, enthaltend 5% einer Zusammensetzung aus 8 Gew.% BDDPA in 92 Gew% Benzylalkohol | 0,2 | | | | | |
| Grapefruitaroma, enthaltend 2% einer Zusammensetzung aus 2 Gew% BDDPA in 98 Gew% Frescolat MPC | | 0,2 | | | | |
| Grapefruitaroma, enthaltend 2% einer Zusammensetzung aus 5 Gew% BDDPA in 47,5 Gew% Triethylcitrat + 47,5 Gew% Pfefferminzöl | | | 0,2 | | | |
| Grapefruitaroma, enthaltend 4% einer Zusammensetzung aus | | | | 0,2 | | |
| 5 Gew% BDDPA in 47,5 Gew% Triacetin + 47,5 Gew% Frescolat^{®} ML | | | | | | |
| Grapefruitaroma, enthaltend 5% einer Zusammensetzung aus 3 Gew% BDDPA in 47 Gew% 2-Phenylethanol + 50 Gew% Frescolat^{®} MPC | | | | | 0,2 | |
| Grapefruitaroma, enthaltend 4% einer Zusammensetzung aus 5 Gew% BDDPA in 95Gew% Anethol | | | | | | 0,2 |
| Wasser | ad 100 | ad 100 | ad 100 | ad 100 | ad 100 | ad 100 |
| Kohlensäure | 0,7 | 0,7 | 0,7 | 0,7 | 0,7 | 0,7 |

Die in den vorstehenden Anwendungsbeispielen gefundenen Effekte lassen sich - gegebenenfalls durch vom Fachmann unschwer vorzunehmende Modifikationen - auf alle Produkte der jeweiligen Produktgruppe, d.h. insbesondere auf Zahnpasten, Kaugummis, Mundwässer, Halsbonbons, Gelatinekapseln, Kaubonbons und Tee in Beuteln übertragen. Dabei ist es für den Fachmann aufgrund der vorliegenden Beschreibung unschwer erkennbar, dass ohne großen Aufwand die erfindungsgemäßen Verbindungen und Mischungen - eventuell unter geringfügigen Modifikationen - untereinander austauschbar sind. Das bedeutet, dass die in den Produkten der Anwendungsbeispiele verwendete erfindungsgemäße Verbindung als Platzhalter auch für die anderen erfindungsgemäßen Verbindungen und Mischungen aufgefasst werden muss. Auch die Konzentration der verwendeten erfindungsgemäßen Verbindung oder Mischung ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischen Bestandteile sind in der oben stehenden Beschreibung offenbart.

Die folgenden Bespiele verdeutlichen die Einsatzmöglichkeiten der erfindungsgemäß zu verwendenden Kühlsubstanzen in kosmetischen Formulierungen, durch deren Verwendung auf der Haut ein als angenehm empfundenes Kälteempfinden und eine Hautberuhigung erzielt werden können.

### Formulierungsbeispiele FM-60 bis FM-66

FM-60 = Aerosol Deo-Spray (nicht erfindungsgemäß)
FM-61 = Sport Shower Gel (nicht erfindungsgemäß)
FM-62= After Shave Balm
FM-63 = Eau de Toilette
FM-64 = Fuß Spray
FM-65 = Deo Stick
FM-66 = Deo APP Roll on Emulsion (nicht erfindungsgemäß)

| **Rohstoff** | **INCI-Name** | **Beispiel** | | | | | | |
|---|---|---|---|---|---|---|---|---|
| | | **FM-60** | **FM-61** | **FM-62** | **FM-63** | **FM-64** | **FM-65** | **FM-66** |
| | | **Anteil (Gew .-%)** | | | | | | |
| 8 Gew.% BDDPA in 92Gew% Benzylalkohol | | | | 0,5 | 0,5 | | 0,5 | |
| 2 Gew% BDDPA in 98 Gew% Frescolat MPC | | | 0,5 | | | | | 0,5 |
| 5 Gew% BDDPA in 47,5 Gew% Triethylcitrat + 47,5 Gew% Pfefferminzöl | | 0,5 | | | | 0,5 | | |
| 5,2 Gew% BDDPA in 31,6 Gew% Triethylcitrat + 31,6 Gew% Triacetin + 31,6 Gew% Pfefferminzöl | | | 0,5 | 0,1 | | | 0,5 | |
| 5 Gew% BDDPA in 47,5 Gew% Triacetin + 47,5 Gew% Frescolat^{®} ML | | 0,5 | | | | | | 0,5 |
| 3 Gew% BDDPA in 47 Gew% 2-Phenylethanol + 50 Gew% Frescolat^{®} MPC | | | | | 0,5 | 0,5 | | |
| Allantoin | Allantoin | | | 0,1 | | | | |
| (-) alpha Bisabolol Natural | Bisabolol | 0,1 | | | | | | |
| Abil 350 | Dimethicone | | | 3,0 | | | | |
| Akyposoft 100 BVC | Sodium Laureth-11 Carboxylate, Laureth-10 | | 8,5 | | | | | |
| Aloe Vera Gel Konzentrat 10:1 | Aloe Barbadensis Leaf Juice | | | | | | 1,0 | |
| Arlypon F | Laureth-2 | | 2,5 | | | | | |
| Carbopol Ultrez-21 | Acrylates/C10-30 Alkyl Acrylate Crosspolymer | | | 0,4 | | | | |
| Covi-Ox T-70 | Tocopherol | | | 0,1 | | | | |
| Dehyton K | Cocoamidopropyl Betaine | | 7,0 | | | | | |
| Deolite | Dimethyl Phenylpropanol Pentylene | | | | | | 0,5 | 0,5 |
| | Glycol | | | | | | | |
| Dow Corning 246 fluid | Cyclohexasiloxane | | | | | | | 1,0 |
| D-Panthenol 75 L | Panthenol | | | 1,0 | | | | |
| Dracorin^{®} 100 S.E.P. | Glyceryl Stearate, PEG-100 Stearate | | | | | | | 0,5 |
| Dracorin^{®} GOC | Glyceryl Oleate Citrate | | | | | | | 2,0 |
| | Caprylic Capric Triglyceride | | | | | | | |
| Dragocide^{®} Liquid | Phenoxyethanol, Methylparaben, Ethylparaben, Butylparaben, Propylparaben, Isobutylparaben | | 0,5 | 0,8 | | | | 0,8 |
| Dragosantol^{®} 100 | Bisabolol | | | 0,2 | | 0,2 | | 0,2 |
| Dragoxat^{®} 89 | Ethylhexyl Isononanoate | | | | | | 1,0 | |
| EDTA BD | Disodium EDTA | | | 0,1 | | | | |
| Ethanol 96% | Ethanol | 26,7 | | | 80,15 | 44,1 | | |
| Extrapone^{®}Ginkgo Biloba | Propylene Glycol, Water (Aqua), Ginkgo Biloba Leaf Extract, Glucose, Lactic Acid | | 1,0 | | | | | |
| Farnesol | Farnesol | | | | | 0,5 | | |
| Fragrance | Perfum | 1,0 | 1,5 | 1,0 | 10,0 | 0,5 | 0,5 | 0,4 |
| Frescolat^{®} MGA | Menthone Glycerin Acetal | | | | | | 0,8 | |
| Frescolat^{®} ML | Menthyl Lactate | | 0,4 | 0,8 | | 0,2 | | 0,3 |
| Genapol LRO Liquid | Sodium Laureth Sulfate | | 39,2 | | | | | |
| Glycerin 99,5% | Glycerin | | | 2,5 | | | | 4,0 |
| Isodragol^{®} | Triisononanoin | | | | | | | 1,0 |
| Jojoba Oil | Simmondsia Chinensis (Jojoba) Seed Oil | | | 2,0 | | | | |
| Natrium Hydroxid 10% Lsg. | Sodium Hydroxide | | 0,1 | 0,8 | | | | 0,6 |
| Natrium Stearat | Sodium Stearate | | | | | | 9,0 | |
| Neutral Oil | Caprylic/Capric Triglyceride | | | | | | | 3,5 |
| PCL -Liquid100 | Cetearyl Ethylhexanoate | | | 3,0 | | 1,0 | | |
| Pemulen TR-2 | Acrylates/C10-30 Alkyl Acrylate Cross-polymer | | | | | | | 0,3 |
| Polymer JR400 | Polyquaternium-10 | | 0,3 | | | | | |
| Propane Butane 2,7 bar | Propane, Butane | 70,2 | | | | 49,5 | | |
| Propylene Glycol | Propylene Glycol | | | | | | 35,7 | |
| Rezal 36 GP | Aluminium Zirconium Tetrachlorohydrex GLY | | | | | | | 5,0 |
| Solubilizer | PEG-40Hydrogenated Castor Oil, Trideceth-9, Propylene Glycol, Water (Aqua) | | 0,5 | | 1,0 | 1,0 | | |
| SymAmide UDA | Undecylenamide DEA, Diethanolamine | | | | | 1,0 | | |
| SymCalmin^{®} | Pentylene Glycol, Butylene Glycol, Hydroxyphenyl Propamidobenzoic Acid | | | 0,5 | | | | |
| SymClariol^{®} | Decylene Glycol | 0,5 | | | | 0,5 | | |
| SymDeo^{®} MPP | Dimethyl Phenylbutanol | 0,5 | | | | | | 0,5 |
| SymMollient^{®} W/S | Trideceth-9, PEG-5 Isononanoate | | | | 1,0 | 0,5 | | |
| SymRelief^{®} | Bisabolol, Zingiber Officinale (Ginger) Root Extract | | 0,2 | 0,2 | | | | |
| SymVital^{™} | Aloe Barbadensis Leaf Juice Powder, Magnesium Ascorbyl Phosphate, Rubus Idaeus (Raspberry) Leaf Extract | | | 0,1 | | | | |
| Vitamin E acetat | Tocopherol Acetate | | | 0,5 | | | | |
| Wasser | Water (Aqua) | | ad 100 | ad 100 | ad 100 | | ad 100 | ad 100 |

### Formulierungsbeispiele FM-67 bis FM-72:

FM-67 = Tagescreme O/W, ca. SPF 15
FM-68 = Sonnenschutzemulsion ca. SPF 25 (nicht erfindungsgemäß)
FM-69 = After Sun Spray
FM-70 = After Shave
FM-71 = Creme W/O
FM-72 = Haar Conditioner

| **Rohstoff** | **INCI-Name** | **Beispiele** | | | | | |
|---|---|---|---|---|---|---|---|
| | | **FM-67** | **FM-68** | **FM-69** | **FM-70** | **FM-71** | **FM-72** |
| | | **Anteil (Gew.-%)** | | | | | |
| 8 Gew.% BDDPA in 92 Gew% Benzylalkohol | | 0,1 | | 0,1 | | | |
| 5 Gew% BDDPA in 47,5 Gew% Triethylcitrat + 47,5 Gew% Pfefferminzöl | | 0,5 | 0,5 | | | 0,5 | 0,1 |
| 5,2 Gew% BDDPA in 31,6 Gew% Triethylcitrat + 31,6 Gew% Triacetin + 31,6 Gew% Pfefferminzöl | | | 0,25 | 0,2 | | | |
| 3 Gew% BDDPA in 47 Gew% 2-Phenylethanol + 50 Gew% Frescolat^{®} MPC | | | | | 1 | 1 | 2 |
| Allantoin | Allantoin | | | 0,1 | | | |
| (-) alpha Bisabolol Natural | Bisabolol | | | 0,2 | | 0,3 | |
| Abil 350 | Dimethicone | 2,0 | | | | | |
| Aluminium Stearate | Aluminium Stearate | | | | | 1,2 | |
| Arlypon F | Laureth-2 | | | | | | |
| Biotive^{®} L-Arginine | Arginine | | 0,5 | | | | |
| Carbopol Ultrez-10 | Carbomer | 0,2 | | 0,2 | | | |
| Covi-Ox T-70 | Tocopherol | | | | 0,1 | | |
| Cutina GMS V | Glyceryl Stearate | 2,0 | | 2,0 | | | |
| Dehyquart A CA | Cetrimonium Chloride | | | | | | 4,0 |
| Dow Corning 246 fluid | Cyclohexasiloxane | | | | 2,0 | | |
| D-Panthenol 75 L | Panthenol | | | | 1,0 | | 1,0 |
| Dracorin^{®} CE | Glyceryl | | 2,0 | | | | |
| | Stearate/Citrate | | | | | | |
| Dracorin^{®} GOC | Glyceryl Oleate Citrate | | | | 2,0 | | |
| | Caprylic Capric Triglyceride | | | | | | |
| Drago-Beta-Glucan | Water (Aqua), Butylene Glycol, Glycerin, Avena Sativa (Oat) | | | 2,0 | | | |
| | Kernel Extract | | | | | | |
| DragoCalm^{®} | Water, Glycerin, | | | 1,0 | | | |
| | Avena Sativa | | | | | | |
| | (Oat Kernel Extract) | | | | | | |
| Dragocide^{®} Liquid | Phenoxyethanol, Methylparaben, Ethylparaben, Butylparaben, Propylparaben, Isobutylparaben | 0,8 | | | | 0,8 | 0,8 |
| Dragoderm^{®} | Glycerin, Triticum Vulgare (Wheat) Gluten, Water (Aqua) | | | 2,0 | 2,0 | | 2,0 |
| Dragosan W/O P | Sorbitan | | | | | 8,0 | |
| | Isostearate, | | | | | | |
| | Hydrogenated Castor Oil, | | | | | | |
| | Ceresin, Beeswax | | | | | | |
| | (Cera Alba) | | | | | | |
| Dragosine^{®} | Carnosine | | | 0,2 | | | |
| Dragoxat^{®} 89 | Ethylhexyl | | 3,0 | 4,0 | 1,0 | 5,0 | |
| | Isononanoate | | | | | | |
| EDTA BD | Disodium EDTA | | 0,1 | 0,1 | | | |
| Emulsiphos^{®} | Potassium Cetyl Phosphate, | 2,0 | | 2,0 | | | |
| | Hydrogenated Palm Glycerides | | | | | | |
| Ethanol 96% | Ethanol | | | | 65,0 | | |
| Farnesol | Farnesol | | | | | | |
| Fragrance | Perfum | 0,3 | 0,4 | 0,3 | 1,0 | 0,3 | 0,3 |
| Frescolat^{®} ML | Menthyl Lactate | 0,2 | | | 0,3 | | |
| Fruitapone^{®} Orange B | Propylene Glycol, Water (Aqua), Citric Acid, Citrus Aurantium Dulcis (Orange) Juice, Trideceth-9, Bisabolol | 1,0 | | | | | |
| Glycerin 99,5% | Glycerin | 2,0 | | 3,0 | 4,0 | 3,0 | |
| Hydrolite^{®}-5 | Pentylene Glycol | | 5,0 | | 5,0 | | |
| Hydroviton^{®}-24 | Water, Pentylene Glycol, Glycerin, Lactic Acid, | | | 1,0 | | 2,0 | |
| | Sodium Lactate, Serine, Urea, Sorbitol, Sodium Chloride, | | | | | | |
| | Allantoin | | | | | | |
| Iso Adipat | Diisopropyl Adipate | | | 1,0 | 5,0 | | |
| Jojoba Oil | Simmondsia Chinensis (Jojoba) Seed Oil | | | | | 2,0 | |
| Keltrol CG RD | Xanthan Gum | 0,1 | 0,1 | 0,2 | | | |
| Lanette O | Cetearyl Alcohol | 3,0 | 2,0 | 3,0 | | | 3,5 |
| Mineral Oil | Mineral Oil | | | | | 8,0 | |
| Natrium Chlorid | Sodium Chloride | | | | | 1,0 | 2,0 |
| Natrium Hydroxid 10% Lsg. | Sodium Hydroxide | 0,5 | | | | 0,4 | |
| Neo Heliopan^{®} 303 | Octocrylene | 5,0 | 8,0 | | | | |
| Neo Heliopan^{®} 357 | Butylmethoxydibenzoylmethane | 1,1 | 3,0 | | | | |
| Neo Heliopan^{®} HMS | Homosalate | | 5,0 | | | | |
| Neo Heliopan^{®} Hydro, 25% Lsg. neutralisiert mit Biotive L-Arginin | Phenylbenzimidazole Sulfonic | 3,0 | 8,0 | | | | |
| | Acid | | | | | | |
| Neo Heliopan^{®}AP, 10% Lsg., neutralisiert mit NAOH | Disodium Phenyl Dibenzimidazole | 3,0 | 13,3 | | | | |
| | Tetrasulfonate | | | | | | |
| Neo Heliopan^{®} OS | Ethylhexyl Salicylate | 5,0 | | | | | |
| Neutral Oil | Caprylic/Capric Triglyceride | | | | 5,0 | | |
| Ozokerite Wax 2389 | Ozokerite | | | | | 2,0 | |
| Pemulen TR-2 | Acrylates/C10-30 Alkyl Acrylate Crosspolymer | | | | 0,3 | | |
| Polyquart H81 | PEG-15 Coco Polyamine | | | | | | 3,0 |
| Propylene Glycol | Propylene Glycol | 3,0 | 4,0 | | | | |
| Softisan 100 | Hydrogenated Coco Glycerides | | 1,5 | | | | |
| Squalan, Vegetable Based | Squalane | | | 3,0 | | | |
| SymCalmin^{®} | Pentylene Glycol, Butylene Glycol, Hydroxyphenyl Propamidoben-zoic Acid | | | 1,0 | | | |
| SymDiol^{®} 68 | 1,2 Hexanediol, Caprylyl Glycol | | | 1,0 | | | |
| SymGlucan^{®} | Water (Aqua) Glycerin, Beta Glucan | | | | 1,0 | | |
| SymMollient^{®} W/S | Trideceth-9, | | | | 0,5 | | |
| | PEG-5 Isononanoate | | | | | | |
| SymRelief^{®} | Bisabolol, | | | | 0,2 | | |
| | Zingiber Officinale (Ginger) Root Extract | | | | | | |
| SymRepair^{®} | Hexyldecanol, Bisabolol, Cetylhydroxyproline Palmitamide, Stearic Acid, | | | 2,0 | 3,0 | | |
| | Brassica Campestris (Rapeseed Sterols) | | | | | | |
| SymVital^{™} | Aloe Barbadensis Leaf Juice Powder, Magnesium Ascorbyl Phosphate, Rubus Idaeus (Raspberry) Leaf Extract | 0,3 | | | | | |
| Triethanolamine 99% | Triethanolamine | | | 0,4 | 0,3 | | |
| Vitamin E acetat | Tocopherol Acetate | | 0,5 | | | 0,2 | |
| Wasser | Water (Aqua) | ad 100 | ad 100 | ad 100 | ad 100 | ad 100 | ad 100 |

## Patentansprüche

1. Mischung, umfassend oder bestehend aus
a) (E)-3-Benzo[1,3]Dioxol-5-yl-N,N-Diphenyl-2-Propenamid, und
b) mindestens eine Substanz ausgewählt aus der Gruppe bestehend aus:
Benzylalkohol, 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849) oder Mischungen daraus,
wobei das Lösungsmittel b) Benzylalkohol, 2-Phenylethanol oder Benzylbenzoat ist.

2. Mischung nach Anspruch 1, wobei das Lösungsmittel ein binäres System ist, welches aus zwei Lösungsmitteln besteht, wobei das erste Lösungsmittel ausgewählt ist aus der Gruppe wie in Anspruch 1 definiert und das zweite Lösungsmittel ausgewählt ist aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849).

3. Mischung nach Anspruch 2, wobei das Lösungsmittel eine Mischung aus Benzylalkohol und einem weiteren Lösungsmittel aus der Gruppe bestehend aus 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849), ist.

4. Mischung nach Anspruch 1, wobei das Lösungsmittel ein ternäres System ist, welches aus drei Lösungsmitteln besteht, wobei das erste Lösungsmittel ausgewählt ist aus der Gruppe wie in Anspruch 1 definiert und die zwei weiteren Lösungsmittel ausgewählt sind aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849).

5. Mischung nach Anspruch 4, wobei das Lösungsmittel ein ternäres System ist, welches aus drei Lösungsmitteln besteht, die ausgewählt sind aus Benzylalkohol und zwei weiteren Lösungsmitteln aus der Gruppe bestehend aus 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849), ist.

6. Mischung nach Anspruch 1, wobei das Lösungsmittel ein quartäres System ist, welches aus vier Lösungsmitteln besteht, die ausgewählt sind aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849).

7. Mischung nach Anspruch 2, wobei das Lösungsmittel b) ausgewählt ist aus den folgenden binären Lösungsmittelkombinationen:
• Benzylalkohol und 2-Phenylethanol,
• Benzylalkohol und Benzylbenzoat,
• Benzylalkohol und Diethylsuccinat
• Benzylalkohol und Triethylcitrat,
• Benzylalkohol und Triacetin,
• Benzylalkohol und Ethanol,
• Benzylalkohol und Pfefferminzöl,
• Benzylalkohol und Anethol,
• Benzylalkohol und Propylenglykol,
• Benzylalkohol und Menthol,
• Benzylalkohol und Menthyl Lactate,
• Benzylalkohol und Menthol Propylene Glycol Carbonate,
• Benzylalkohol und Menthol Ethylene Glycol Carbonate,
• Benzylalkohol und Menthone Glyceryl Acetal,
• 2-Phenylethanol und Menthol Propylene Glycol Carbonate,
• Diethylsuccinat und 2-Phenylethanol,
• Triacetin und Benzylbenzoat,
• 2-Phenylethanol und Pfefferminzöl.

8. Mischung nach Anspruch 4, wobei das Lösungsmittel b) ausgewählt ist aus den folgenden ternären Lösungsmittelkombinationen:
Benzylalkohol mit:
• 2-Phenylethanol und Benzylbenzoat,
• 2-Phenylethanol und Diethylsuccinat,
• Triethylcitrat und Triacetin,
• Triacetin und Ethanol,
• Triacetin und Pfefferminzöl,
• Menthol Ethylene Glycol Carbonate und Anethol,
• Diethylsuccinat und Menthol,
• Triacetin und Menthyl Lactate,
• Anethol und Menthol Propylene Glycol Carbonate,
• Triacetin und Menthol Ethylene Glycol Carbonate,
• 2-Phenylethanol und Menthone Glyceryl Acetal,
• 2-Phenylethanol und Menthol Propylene Glycol Carbonate,
• Triacetin und Benzylbenzoat,
• 2-Phenylethanol und Pfefferminzöl,
• Anethol und Triacetin,
• Pfefferminzöl und Menthyl Lactate,
• Triacetin und Menthone Glyceryl Acetal,
• Triethylcitrat und Menthol Ethylene Glycol Carbonate,
• Benzylbenzoat und Menthol Ethylene Glycol Carbonate,
• 2-Phenylethanol und Triethylcitrat,
• Triethylcitrat und Diethylsuccinat,
• Pfefferminzöl und Menthyl Lactate,
• Ethanol und Menthyl Lactate,
oder
• Triacetin, 2-Phenylethanol und Pfefferminzöl,
• Anethol, Benzylalkohol und Triacetin,
• Triacetin, Benzylbenzoat und Menthyl Lactate,
• Triacetin, 2-Phenylethanol und Menthone Glyceryl Acetal,
• Triacetin, 2-Phenylethanol und Menthol Propylene Glycol Carbonate,
• Menthyl Lactate, 2-Phenylethanol und Pfefferminzöl,
• Benzylbenzoat, Triethylcitrat und Menthol Ethylene Glycol Carbonate,

9. Mischung nach einem der vorhergehenden Ansprüche 1 bis 8, wobei Komponente a) in einer Menge von 2 Gew.% bis 20 Gew.% und Komponente b) in einer Menge von 98 Gew.% bis 80 Gew,% in der Mischung vorliegt, bezogen auf die Gesamtmischung, mit der Maßgabe dass beide Komponenten a) und b) sich zusammen zu 100 Gew.% addieren.

10. Mischung nach einem der vorhergehenden Ansprüche 1 bis 9, wobei die Mischung bis 100°C stabil ist.

11. Mischung nach einem der vorhergehenden Ansprüche 1 bis 9, wobei (E)-3-Benzo[1,3]Dioxol-5-yl-N,N-Diphenyl-2-Propenamid (Komponente a)) in der Mischung bei Raumtemperatur (23°C) stabil gelöst vorliegt.

12. Partikel erhältlich dadurch, dass eine Mischung nach einem der Ansprüche 1 bis 9 einem Sprühtrocknungsverfahren oder Sprühgranulationsverfahren unterzogen wird.

13. Halbfertigprodukt, umfassend eine Mischung nach einem der Ansprüche 1 bis 9 oder Partikel nach Anspruch 12.

14. Verwendung von mindestens einem Lösungsmittel ausgewählt aus der Gruppe bestehend aus Benzylalkohol, 2-Phenylethanol, Benzylbenzoat, Diethylsuccinat, Triethylcitrat, Triacetin, Ethanol, Pfefferminzöl, Anethol, Propylenglykol, Menthol, Menthyl Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat (Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849) oder Mischungen daraus, zum Lösen von festem (E)-3-Benzo[1,3]Dioxol-5-yl-N,N-Diphenyl-2-Propenamid bei Raumtemperatur, vorzugsweise bei etwa 23°C, wobei das Lösungsmittel ausgewählt ist wie in einem der Ansprüche 1 bis 8 definiert.

## Claims

1. A mixture comprising or consisting of
a) (E)-3-benzo[1,3]dioxol-5-yl-N,N-diphenyl-2-propenamide, and
b) at least one substance selected from the group consisting of:
benzyl alcohol, 2-phenylethanol, benzyl benzoate, diethyl succinate, triethyl citrate, triacetin, ethanol, peppermint oil, anethole, propylene glycol, menthol, menthyl methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate (monomethyl succinate (FEMA GRAS 3810), monomenthyl glutarate (FEMA GRAS 4006), menthoxy-1,2-propanediol (FEMA GRAS 3784), menthoxy-2-methyl-1,2-propanediol (FEMA GRAS 3849) or mixtures thereof,
wherein the solvent b) is benzyl alcohol, 2-phenylethanol or benzyl benzoate.

2. The mixture according to claim 1, wherein the solvent is a binary system, consisting of two solvents, wherein the first solvent is selected from the group as defined in claim 1 and the second solvent is selected from the group consisting of benzyl alcohol, 2-phenylethanol, benzyl benzoate, diethyl succinate, triethyl citrate, triacetin, ethanol, peppermint oil, anethole, propylene glycol, menthol, menthyl methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate (monomethyl succinate (FEMA GRAS 3810), monomenthyl glutarate (FEMA GRAS 4006), menthoxy-1,2-propanediol (FEMA GRAS 3784), menthoxy-2-methyl-1,2-propanediol (FEMA GRAS 3849).

3. The mixture according to claim 2, wherein the solvent is a mixture of benzyl alcohol and another solvent selected from the group consisting of 2-phenylethanol, benzyl benzoate, diethyl succinate, triethyl citrate, triacetin, ethanol, peppermint oil, anethole, propylene glycol, menthol, menthyl methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate (monomethyl succinate (FEMA GRAS 3810), Monomenthyl Glutarate (FEMA GRAS 4006), menthoxy-1,2-propanediol (FEMA GRAS 3784), menthoxy-2-methyl-1,2-propanediol (FEMA GRAS 3849).

4. The mixture according to claim 1, wherein the solvent is a ternary system consisting of three solvents, wherein the first solvent is selected from the group as defined in claim 1 and the two further solvents are solvents are selected from the group consisting of benzyl alcohol, 2-phenylethanol, benzyl benzoate, diethyl succinate, triethyl citrate, triacetin, ethanol, peppermint oil, anethole, propylene glycol, menthol, menthyl methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate (monomethyl succinate (FEMA GRAS 3810), monomenthyl glutarate (FEMA GRAS 4006), menthoxy-1,2-propanediol (FEMA GRAS 3784), menthoxy-2-methyl-1,2-propanediol (FEMA GRAS 3849).

5. The mixture according to claim 4, wherein the solvent is a ternary system consisting of three solvents selected from benzyl alcohol and two further solvents selected from the group consisting of 2-phenylethanol, benzyl benzoate, diethyl succinate, triethyl citrate, triacetin, ethanol, peppermint oil, anethole, propylene glycol, menthol, menthyl methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate (monomethyl succinate (FEMA GRAS 3810), monomenthyl glutarate (FEMA GRAS 4006), menthoxy-1,2-propanediol (FEMA GRAS 3784), menthoxy-2-methyl-1,2-propanediol (FEMA GRAS 3849).

6. The mixture according to claim 1, wherein the solvent is a quaternary system consisting of four solvents selected from the group consisting of benzyl alcohol, 2 phenyl ethanol, benzyl benzoate, diethyl succinate, triethyl citrate, triacetin, ethanol, peppermint oil, anethole, propylene glycol, menthol, menthyl methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate (monomethyl succinate (FEMA GRAS 3810), monomenthyl glutarate (FEMA GRAS 4006), menthoxy-1,2-propanediol (FEMA GRAS 3784), menthoxy-2-methyl-1,2-propanediol (FEMA GRAS 3849).

7. The mixture according to claim 2, wherein the solvent b) is selected from the following binary solvent combinations:
• benzyl alcohol and 2-phenylethanol,
• benzyl alcohol and benzyl benzoate,
• benzyl alcohol and diethyl succinate
• benzyl alcohol and triethyl citrate,
• benzyl alcohol and triacetin,
• benzyl alcohol and ethanol,
• benzyl alcohol and peppermint oil,
• benzyl alcohol and anethole,
• benzyl alcohol and propylene glycol,
• benzyl alcohol and menthol,
• benzyl alcohol and menthyl lactate,
• benzyl alcohol and menthol propylene glycol carbonate,
• benzyl alcohol and menthol ethylene glycol carbonate,
• benzyl alcohol and menthone glyceryl acetal,
• 2-phenylethanol and menthol propylene glycol carbonate,
• diethyl succinate and 2-phenylethanol,
• triacetin and benzyl benzoate,
• 2-phenylethanol and peppermint oil.

8. The mixture according to claim 4, wherein the solvent b) is selected from the following ternary solvent combinations:
benzyl alcohol with:
• 2-phenylethanol and benzyl benzoate,
• 2-phenylethanol and diethyl succinate,
• triethyl citrate and triacetin,
• triacetin and ethanol,
• triacetin and peppermint oil,
• menthol ethylene glycol carbonate and anethole,
• diethyl succinate and menthol,
• triacetin and menthyl lactate,
• anethole and menthol propylene glycol carbonate,
• triacetin and menthol ethylene glycol carbonate,
• 2-phenylethanol and menthone glyceryl acetal,
• 2-phenylethanol and menthone propylene glycol carbonate,
• triacetin and benzyl benzoate,
• 2-phenylethanol and peppermint oil,
• anethole and triacetin,
• peppermint oil and menthyl lactate,
• triacetin and menthone glyceryl acetal,
• triethyl citrate and menthol ethylene glycol carbonate,
• benzyl benzoate and menthol ethylene glycol carbonate,
• 2-phenylethanol and triethyl citrate,
• triethyl citrate and diethyl succinate,
• peppermint oil and menthyl lactate,
• ethanol and menthyl lactate,
or
• triacetin, 2-phenylethanol and peppermint oil,
• anethole, benzyl alcohol and triacetin,
• triacetin, benzyl benzoate and menthyl lactate,
• triacetin, 2-phenylethanol and menthone glyceryl acetal,
• triacetin, 2-phenylethanol and menthone propylene glycol carbonate,
• menthyl lactate, 2-phenylethanol and peppermint oil,
• benzyl benzoate, triethyl citrate and menthol ethylene glycol carbonate.

9. The mixture according to any one of the preceding claims 1 to 8, wherein component a) is present in an amount of 2 wt.-% to 20 wt.-% and component b) is present in an amount of 98 wt.-% to 80 wt.-% in the mixture, based on the total mixture, with the proviso that both components a) and b) together add up to 100 wt.-%.

10. The mixture according to any one of the preceding claims 1 to 9, wherein the mixture is stable up to 100°C,

11. The mixture according to any one of the preceding claims 1 to 9, wherein (E)-3-benzo[1,3]dioxol-5-yl-N,N-diphenyl-2-propenamide (component a)) is stably dissolved in the mixture at room temperature (23°C).

12. Particles obtainable by subjecting a mixture according to any one of claims 1 to 9 to a spray drying process or spray granulation process.

13. A semi-finished product comprising a mixture according to any one of claims 1 to 9 or particles according to claim 12.

14. Use of at least one solvent selected from the group consisting of benzyl alcohol, 2-phenylethanol, benzyl benzoate, diethyl succinate, triethyl citrate, triacetin, ethanol, peppermint oil, anethole, propylene glycol, menthol, menthyl methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), menthol propylene glycol carbonate (FEMA GRAS 3806), menthyl-N-ethyloxamate (monomethyl succinate (FEMA GRAS 3810), monomenthyl glutarate (FEMA GRAS 4006), menthoxy-1,2-propanediol (FEMA GRAS 3784), menthoxy-2-methyl-1,2-propanediol (FEMA GRAS 3849) or mixtures thereof, for dissolving solid (E)-3-benzo[1,3]dioxol-5-yl-N,N-diphenyl-2-propenamide at room temperature, preferably at about 23°C, wherein the solvent is selected as defined in any one of claims 1 to 8.

## Revendications

1. Mélange comprenant ou constitué de
a) (E)-3-benzo[1,3]dioxol-5-yl-N,N-diphényl-2-propénamide, et
b) d'au moins une substance choisie dans le groupe constitué par:
l'alcool benzylique, le 2-phényléthanol, le benzoate de benzyle, le succinate de diéthyle, le citrate de triéthyle, la triacétine, l'éthanol, l'huile de menthe poivrée, l'anéthol, le propylène glycol, le menthol, l'éther de menthylméthyle, l'acétal de menthone glycéryle (FEMA GRAS 3807), le cétal de menthone glycéryle (FEMA GRAS 3808), le lactate de menthyle (FEMA GRAS 3748), le carbonate de menthol éthylène glycol (FEMA GRAS 3805), le carbonate de menthol propylène glycol (FEMA GRAS 3806), le menthyl-N-éthyloxamate (succinate de monométhyle (FEMA GRAS 3810), le glutarate de monomenthyle (FEMA GRAS 4006), le menthoxy-1,2-propanediol (FEMA GRAS 3784), le menthoxy-2-méthyl-1,2-propanediol (FEMA GRAS 3849) ou leurs mélanges,
dans lequel le solvant b) est l'alcool benzylique, le 2-phényléthanol ou le benzoate de benzyle.

2. Mélange selon la revendication 1, dans lequel le solvant est un système binaire qui est constitué de deux solvants, dans lequel le premier solvant est choisi dans le groupe tel que défini dans la revendication 1 et le deuxième solvant est choisi dans le groupe constitué par l'alcool benzylique, le 2-phényléthanol, le benzoate de benzyle, le succinate de diéthyle, le citrate de triéthyle, la triacétine, l'éthanol, l'huile de menthe poivrée, l'anéthol, le propylène glycol, le menthol, l'éther de menthylméthyle, l'acétal de menthone glycéryle (FEMA GRAS 3807), le cétal de menthone glycéryle (FEMA GRAS 3808), le lactate de menthyle (FEMA GRAS 3748), le carbonate de menthol éthylène glycol (FEMA GRAS 3805), le carbonate de menthol propylène glycol (FEMA GRAS 3806), le menthyl-N-éthyloxamate (succinate de monométhyle (FEMA GRAS 3810), le glutarate de monomenthyle (FEMA GRAS 4006), le menthoxy-1,2-propanediol (FEMA GRAS 3784), le menthoxy-2-méthyl-1,2-propanediol (FEMA GRAS 3849).

3. Mélange selon la revendication 2, dans lequel le solvant est un mélange d'alcool benzylique et d'un autre solvant du groupe constitué par le 2-phényléthanol, le benzoate de benzyle, le succinate de diéthyle, le citrate de triéthyle, la triacétine, l'éthanol, l'huile de menthe poivrée, l'anéthol, le propylène glycol, le menthol, l'éther de menthylméthyle, l'acétal de menthone glycéryle (FEMA GRAS 3807), le cétal de menthone glycéryle (FEMA GRAS 3808), le lactate de menthyle (FEMA GRAS 3748), le carbonate de menthol éthylène glycol (FEMA GRAS 3805), le carbonate de menthol propylène glycol (FEMA GRAS 3806), le menthyl-N-éthyloxamate (succinate de monométhyle (FEMA GRAS 3810), le glutarate de monomenthyle (FEMA GRAS 4006), le menthoxy-1,2-propanediol (FEMA GRAS 3784), le menthoxy-2-méthyl-1,2-propanediol (FEMA GRAS 3849).

4. Mélange selon la revendication 1, dans lequel le solvant est un système ternaire qui est constitué de trois solvants, dans lequel le premier solvant est choisi dans le groupe tel que défini dans la revendication 1 et les deux autres solvants sont choisis dans le groupe constitué par l'alcool benzylique, le 2-phényléthanol, le benzoate de benzyle, le succinate de diéthyle, le citrate de triéthyle, la triacétine, l'éthanol, l'huile de menthe poivrée, l'anéthol, le propylène glycol, le menthol, l'éther de menthylméthyle, l'acétal de menthone glycéryle (FEMA GRAS 3807), le cétal de menthone glycéryle (FEMA GRAS 3808), le lactate de menthyle (FEMA GRAS 3748), le carbonate de menthol éthylène glycol (FEMA GRAS 3805), le carbonate de menthol propylène glycol (FEMA GRAS 3806), le menthyl-N-éthyloxamate (succinate de monométhyle (FEMA GRAS 3810), le glutarate de monomenthyle (FEMA GRAS 4006), le menthoxy-1,2-propanediol (FEMA GRAS 3784), le menthoxy-2-méthyl-1,2-propanediol (FEMA GRAS 3849).

5. Mélange selon la revendication 4, dans lequel le solvant est un système ternaire qui est constitué de trois solvants qui sont choisis parmi l'alcool benzylique et deux autres solvants du groupe constitué par le 2-phényléthanol, le benzoate de benzyle, le succinate de diéthyle, le citrate de triéthyle, la triacétine, l'éthanol, l'huile de menthe poivrée, l'anéthol, le propylène glycol, le menthol, l'éther de menthylméthyle, l'acétal de menthone glycéryle (FEMA GRAS 3807), le cétal de menthone glycéryle (FEMA GRAS 3808), le lactate de menthyle (FEMA GRAS 3748), le carbonate de menthol éthylène glycol (FEMA GRAS 3805), le carbonate de menthol propylène glycol (FEMA GRAS 3806), le menthyl-N-éthyloxamate (succinate de monométhyle (FEMA GRAS 3810), le glutarate de monomenthyle (FEMA GRAS 4006), le menthoxy-1,2-propanediol (FEMA GRAS 3784), le menthoxy-2-méthyl-1,2-propanediol (FEMA GRAS 3849).

6. Mélange selon la revendication 1, dans lequel le solvant est un système quaternaire qui est constitué de quatre solvants qui sont choisis dans le groupe constitué par l'alcool benzylique, le 2-phényléthanol, le benzoate de benzyle, le succinate de diéthyle, le citrate de triéthyle, la triacétine, l'éthanol, l'huile de menthe poivrée, l'anéthol, le propylène glycol, le menthol, l'éther de menthylméthyle, l'acétal de menthone glycéryle (FEMA GRAS 3807), le cétal de menthone glycéryle (FEMA GRAS 3808), le lactate de menthyle (FEMA GRAS 3748), le carbonate de menthol éthylène glycol (FEMA GRAS 3805), le carbonate de menthol propylène glycol (FEMA GRAS 3806), le menthyl-N-éthyloxamate (succinate de monométhyle (FEMA GRAS 3810), le glutarate de monomenthyle (FEMA GRAS 4006), le menthoxy-1,2-propanediol (FEMA GRAS 3784), le menthoxy-2-méthyl-1,2-propanediol (FEMA GRAS 3849).

7. Mélange selon la revendication 2, dans lequel le solvant b) est choisi parmi les combinaisons binaires de solvants suivantes:
• alcool benzylique et 2-phényléthanol,
• alcool benzylique et benzoate de benzyle,
• alcool benzylique et succinate de diéthyle
• alcool benzylique et citrate de triéthyle,
• alcool benzylique et triacétine,
• alcool benzylique et éthanol,
• alcool benzylique et huile de menthe poivrée,
• alcool benzylique et anéthol,
• alcool benzylique et propylène glycol,
• alcool benzylique et menthol,
• alcool benzylique et lactate de menthyle,
• alcool benzylique et carbonate de menthol propylène glycol,
• alcool benzylique et carbonate de menthol éthylène glycol,
• alcool benzylique et acétal de menthone glycéryle,
• 2-phényléthanol et carbonate de menthol propylène glycol,
• succinate de diéthyle et 2-phényléthanol,
• triacétine et benzoate de benzyle,
• 2-phényléthanol et huile de menthe poivrée,

8. Mélange selon la revendication 4, dans lequel le solvant b) est choisi parmi les combinaisons ternaires de solvants suivantes:
Alcool benzylique avec:
• 2-phényléthanol et benzoate de benzyle,
• 2-phényléthanol et succinate de diéthyle,
• citrate de triéthyle et triacétine,
• triacétine et éthanol,
• triacétine et huile de menthe poivrée,
• carbonate de menthol éthylène glycol et anéthol,
• succinate de diéthyle et menthol,
• triacétine et lactate de menthyle,
• anéthol et carbonate de menthol propylène glycol,
• triacétine et carbonate de menthol éthylène glycol,
• 2-Phényléthanol et acétal de menthone glycéryle,
• 2-Phényléthanol et carbonate de menthol propylène glycol,
• triacétine et benzoate de benzyle,
• 2-phényléthanol et huile de menthe poivrée.
• anéthol et triacétine,
• huile de menthe poivrée et lactate de menthyle,
• triacétine et acétal de menthone glycéryle,
• citrate de triéthyle et carbonate de menthol éthylène glycol,
• benzoate de benzyle et carbonate de menthol éthylène glycol,
• 2-phényléthanol et citrate de triéthyle,
• citrate de triéthyle et succinate de diéthyle,
• huile de menthe poivrée et lactate de menthyle,
• éthanol et lactate de menthyle,
ou
• triacétine, 2-phényléthanol et huile de menthe poivrée,
• anéthol, alcool benzylique et triacétine,
• triacétine, benzoate de benzyle et lactate de menthyle,
• triacétine, 2-phényléthanol et acétal de menthone glycéryle,
• triacétine, 2-Phényléthanol et carbonate de menthol propylène glycol,
• lactate de menthyle, 2-phényléthanol et huile de menthe poivrée,
• benzoate de benzyle, citrate de triéthyle et carbonate de menthol éthylène glycol.

9. Mélange selon l'une quelconque des revendications précédentes 1 à 8, dans lequel le composant a) est présent dans le mélange en une quantité comprise entre 2 % en poids et 20 % en poids et le composant b) est présent dans le mélange en une quantité comprise entre 98 % en poids et 80 % en poids, par rapport au mélange total, à condition que les deux composants a) et b) totalisent ensemble 100 % en poids,

10. Mélange selon l'une quelconque des revendications précédentes 1 à 9, dans lequel le mélange est stable jusqu'à 100 °C.

11. Mélange selon l'une quelconque des revendications précédentes 1 à 9, dans lequel (E)-3-benzo[1,3]dioxol-5-yl-N,N-diphényl-2-propénamide (composant a)) est en solution stable à température ambiante (23 °C) dans le mélange.

12. Particules aptes à être obtenues en soumettant un mélange selon l'une quelconque des revendications 1 à 9 à un procédé de séchage par pulvérisation ou à un procédé de granulation par pulvérisation.

13. Produit semi-fini, comprenant un mélange selon l'une quelconque des revendications 1 à 9 ou des particules selon la revendication 12.

14. Utilisation d'au moins un solvant choisi dans le groupe constitué par l'alcool benzylique, le 2-phényléthanol, le benzoate de benzyle, le succinate de diéthyle, le citrate de triéthyle, la triacétine, l'éthanol, l'huile de menthe poivrée, l'anéthol, le propylène glycol, le menthol, l'éther de menthylméthyle, l'acétal de menthone glycéryle (FEMA GRAS 3807), le cétal de menthone glycéryle (FEMA GRAS 3808), le lactate de menthyle (FEMA GRAS 3748), le carbonate de menthol éthylène glycol (FEMA GRAS 3805), le carbonate de menthol propylène glycol (FEMA GRAS 3806), le menthyl-N-éthyloxamate (succinate de monométhyle (FEMA GRAS 3810), le glutarate de monomenthyle (FEMA GRAS 4006), le menthoxy-1,2-propanediol (FEMA GRAS 3784), le menthoxy-2-méthyl-1,2-propanediol (FEMA GRAS 3849) ou des mélanges de ceux-ci, pour dissoudre du (E)-3-benzo[1,3]dioxol-5-yl-N,N-diphényl-2-propénamide solide à température ambiante, de préférence à environ 23 °C, le solvant étant choisi parmi ce qui est défini dans l'une quelconque des revendications 1 à 8.
